# EUROPEAN PATENT APPLICATION

(11) **EP 3 260 149 A1**
(43) Date of publication of application: **27.12.2017**
(21) Application number: 17162961.1
(22) Date of filing: 27.03.2017
(51) Int. Cl.: A61M 5/24, A61M 5/28, A61M 5/142

(54) **A METHOD FOR FIXATION OF A CARTRIDGE IN AN INJECTION DEVICE**

(30) Priority: 23.06.2016 EP 16175887; 23.06.2016 EP 16175898; 12.01.2017 EP 17151131
(71) Applicant: TecPharma Licensing AG, 3401 Burgdorf (CH)
(72) Inventor: Hirschel, Jürg, 3007 Bern (CH); Hostettler, Patrick, 3415 Hasle (CH); Scheurer, Simon, 3006 Bern (CH)

(57) **Abstract**

A method for fixating a cartridge (26) adapted for receiving a medicament (134) to a cartridge holder (4d) of an injection device, comprising the steps of:
- Providing a cartridge (26) having cylindrical glass barrel (125) and a septum (27) connected to the distal end of the glass barrel (125) using a crimp (27a),
- Providing a cartridge holder (4d),
- Inserting the cartridge (26) into the cartridge holder (4d) such that the distal end of the cartridge (26) first enters the cartridge holder (4d) and is received by a second cavity (128) which is part of the cartridge holder (4d),
Characterized by that the cartridge (26) is axially and rotationally fixed (4d) after the insertion step c) by at least one elastic attachment means (138) for attaching the cartridge (26) to the cartridge holder (4d).

## Description

### Background

Devices for delivery of medication to the patient which are adhered to the skin of the patient have been developed in the past, either for delivery of multiple adjustable doses superimposed on a basal rate (WO0240083 A2), or for multiple fixed doses including a basal rate (WO11046950 A1). Those devices have been preliminary developed for the treatment of diabetes. Alternatively, patch type of bolus injectors were developed also for the treatment of other diseases like cardiovascular diseases, auto-immune diseases or cancer for the delivery of a single dose as described in WO10029054 A1. Such a device is adhered to the patients' skin and activated automatically, or by the use of an activation button, or via a remote control system if the device is enabled to connect to other devices, and subsequently the single bolus volume will be injected.

The term "medicament" or "medication" includes any flowable medical formulation suitable for controlled administration through a means such as, for example, a cannula or a hollow needle and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from -or harvested by- biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances.

Medical delivery devices in general or patch devices have different functionalities combined in one system. For example the patient first sets a dose which is subsequently delivered by a delivery mechanism. Prior to delivery it is required to insert a needle or canula in a patient which is either done manually, or using a needle insertion and/or retraction system. After the delivery - especially for reusable devices, the drive mechanism needs to be reset. The different functionalities are required at different times during the injection procedure and therefore coupling systems are needed that couple and decouple the functionalities from another. Such a coupling can be a permanent coupling between two parts which are engaged and biased with respect to each other but transmit substantial forces or torques in one rotation direction only. An example is a one-way ratchet system which transmits rotation from one part to another in one rotation direction only. In the opposite rotation direction the two parts ratchet versus each other producing audible clicks, for example delivery clicks. A disadvantage of such a one way ratchet is that the ratchet system requires a certain torque to ratchet the system, e.g. at the cost of the efficiency of the device. As an alternative, for cases where the ratcheting and efficiency losses are not desired, axial shifts are required to open or close such a ratchet system such that the unidirectional coupling of the ratchet system is closed when the two parts are intended to rotate both in one direction and the coupling is open or decoupled so that the two parts can rotate with respect to each other. For axially shifting the two parts together, a gearing or threading engagement can be used as described in US9107999, where a piston rod is linearly advanced, and the front end of the piston rod is coupled to the stopper or plunger of a reservoir. The piston rod has an internal threading which matches an outside threading of a drive sleeve. Rotation of the drive sleeve linearly advances the piston rod which is prevented from rotation due to a form-fit engagement to the housing. With this arrangement, axial movement without rotational movements are allowed. The stopper or plunger in US9107999 is threadedly engaged with the front end of the piston rod, and since rotation of the piston rod is prevented, the reservoir with the plunger needs to be screwed manually onto the front end of the non-rotating piston rod. An aspect of the present device is therefore to provide a coupling mechanism which couples and decouples, preferably a ratchet system, but which also allows for relative rotation between the two parts in the decoupled state.

Medical delivery devices such as patch pumps, patch injectors or bolus injectors that are adhered to the skin of the patient have a needle insertion mechanism for insertion of a needle or a soft canula into the skin of the patient for subcutaneous delivery of a medicament. Additionally, such a mechanism can be equipped with a needle retraction feature, to retract the needle, the soft canula or steel canula into the device before being removed from the patient's skin. Such needle insertion mechanism either inserts first a steel needle together with a soft-canula surrounding the steel needle and the needle is retracted prior to delivery of the medication through the soft canula such as described in WO0240083 A2. Alternatively, a steel canula is inserted in the tissue which is also used for subsequent delivery as shown in WO05002649 A1.

The insertion of the needle requires a linear stroke, for example of a holder that is attached to the needle or the canula. The insertion is preferably done perpendicular to the skin of the patient and consequently the stroke length required for inserting the canula occupies space in this perpendicular direction and locally increases the dimensions, e.g. thickness of the device as presented in WO13140395 A1. The increased dimensions for the linear stroke of the canula insertion reduces the usability and wearing comfort of the device for the patient.

In order to reduce the space required for the needle insertion mechanism, several solutions have been provided. In WO2011012465, the needle holder is driven by a part that axially slides in a direction perpendicular to the surface of the patient or to the bottom surface of the injection device. The sliding part has a guiding means, for example a groove or notch oriented oblique to the bottom surface of the device and the needle holder is keyed both to the groove of the sliding part and to a linear guide present in the housing or a part attached to the housing. The linear guide is oriented perpendicular to the bottom surface and ensures vertical insertion of the needle. Horizontal movement of the sliding part thus drives the needle holder through the guiding means from a needle retracted to a needle inserted position. A further horizontal movement of the sliding part ensures that a second part of the guiding curve retracts the needle from the inserted to the retracted position. The fact that both insertion and retraction are driven by the sliding part implies that the horizontal movement occupies space which enlarges the lateral dimensions of the device.

Another space saving arrangement for a needle insertion and retraction mechanism is presented in DE102004059491 where a lancet device is presented having a rotating part with a sinusoidal guide curve applied the outside surface of the rotating part. The guide curve drives the needle from the top of the sinus to the bottom as the rotating part rotates over a first angle and therewith inserting the needle into a patient. Rotation of the part over an additional angle drives the needle from the minimum of the sinus back to the maximum and thereby accomplishes retraction of the needle. In WO15032747, a needle insertion and retraction mechanism is presented based on a rotating drum having a sinusoidal shaped guiding curve designed as groove on the outside surface of the drum. It is therefore an aspect of the device presented here to overcome the drawbacks of the prior art insertion and retraction mechanisms.

The medication delivery device or patch device or patch pump delivers medication from a reservoir that is connected via a fluid path to the canula for subcutaneous delivery to the patient. Medication is preferably delivered by advancement of a stopper or plunger present in the reservoir. The plunger is advanced by a drive mechanism advancing a piston rod. The piston rod can be made as a stiff and linear piston rod, which is described in WO10029054 for a skin attachable patch device, but the linear piston rod enlarges the lateral dimensions of the device leading to discomfort for the patient. Several solutions have been provided for a space efficient design of the piston rod using curved or bendable piston rods. Such curved piston rods are either made from a helical spring as shown in US6474219 or as a segmented piston rod, with separate elements that are connected to each other via a hinge as shown in WO04056411. Both in US6474219 and WO04056411 the piston rod is driven from the outside by a nut element which rotates to advance the segments of the piston rod. On the inside of the nut there is a threading engaging an external thread on the outside of the segments of the piston rod. Once the segments are axially aligned and abut each other, an axial force can be transmitted by the stacked segments to the plunger of the reservoir. The last segment abuts the plunger to expel medication from the device and needs to be guided into the reservoir to ensure correct and plane abutment of the plunger and to prevent blockage of the piston rod in the reservoir. Therefore, the nut element driving the segmented piston rod is located close to the opening of the reservoir as in WO07038059 which requires that the drive train is arranged close to the reservoir. As an alternative, the first segment, which is furthest away from the reservoir is driven by a nut element as presented in WO9509021, however this requires a stable configuration of the last segment(s) entering the cartridge. In WO 0183008, the Innolet device, this is solved by stiffening the hinge between the last two segments or by lengthening the length of the last segment as in WO04056411. The disadvantage of lengthening the non-flexible part of the flexible piston rod is that more space is required to bring the piston rod into the position for expelling medication from the device. A longer, rigid portion of the piston sticks out of the open end of the reservoir before the piston rod can be flexed for connection to the drive mechanism. It is therefore an aspect of the present device to provide an alternative, more space saving configuration of a piston rod that is driven by the first segment and enables a reliable entrance of the last segment into the reservoir.

In US20160262984 a method for forming an aseptic primary container piercing mechanism is presented whereby a pre-sterilized container is inserted in a fluid path and subsequently the fluid path is sterilized in a final sterilization step. A full cartridge is inserted in the fluid path, which has the disadvantage that the final sterilization step (for example gamma irradiation or ethylene oxide) affects the contents, e.g. medication present in the container. For example, protein molecules can get denaturated, particles are formed, or the shelf life is negatively affected. Additionally, the full cartridge is inserted having a certain shelf life for the medication, thus requiring full assembly and delivery of the device to the customer within a short time frame to benefit from the full shelf life of the medicament. Therefore it is beneficial to provide solutions that separate the steps of the assembly of the cartridge into a fluid path and the filling of the cartridges with the medications, both in terms of the time and geographical location to increase the flexibility of the device assembly.

Injection devices, patch devices or infusion pumps may have a separate cartridge containing medication have a cartridge holder for insertion of a cartridge containing the medication. The distal end of such a cartridge comprises a septum that can be pierced by a needle that is part of a needle hub which is attachable to the end of the cartridge holder. After the injection, the needle is removed and the septum automatically closes the cartridge again to prevent exchange of gaseous components with the ambient. Depending on the medication, the septum is pierced only once or, for example in diabetes treatment, the septum is pierced multiple times and each time a new needle hub with a needle is attached (and removed after injection) from the cartridge holder.

In patch devices or infusion devices having a prefilled cartridge, the cartridge is enclosed in a cartridge holder of the device. The cartridge is closed by a septum that will be pierced just prior to use of the device, e.g. during shelflife the septum is not pierced. Upon use, the septum is pierced, for example by a spike, and a fluid connection between the contents of the cartridge and a fluid path is established.

Preferably, there is a fixation of the cartridge to the cartridge holder that prevents excessive axial and rotational play between the holder and the cartridge. During attachment and removal of the needle hub from the cartridge holder, axial and/or rotational forces act upon the cartridge and therefore requires an adequate cartridge fixation. Also during piercing of the septum by the spike of the fluid path of the patch device, forces can act upon the cartridge. Injection devices, patch devices or infusion devices containing such a cartridge may be subjected to external forces during transportation or during use and therefore it is required that the cartridge, which is preferably mainly made of glass, is fixated to prevent damage to the cartridge or glass breakage. Furthermore, it is advantageous if the glass cartridge fixed in the cartridge holder is protected by a shock absorbing or damping layer to reduce the risk of impact damage to the cartridge, for example when a pen, patch device or infusion pump drops on a floor.

Cartridges made out of glass have the tendency of having a relative large dimensional tolerance, for example in the length of the cartridge. The cartridge receiving unit of the injection system, such as a cartridge holder, is preferably made by injection molding of plastics thus ensuring smaller dimensional tolerances. Consequently it is advantageous to a have a cartridge fixation system that is adapted to compensate for differences in tolerances by the fixation system.

In EP0000654 a holding mechanism for a suspension device is shown. An adhesive material is attached to the outside surface of the device and thereby the suspension device can be engaged with a support device such that the suspension device is in an upright position during delivery.

A different approach for holding a cartridge containing medication is presented in WO12017063. The cartridge is inserted into a cartridge holder and the fixation between cartridge and the cartridge holder is achieved by a retaining ring which can be made from an elastic material. The fixation is achieved by the presence of the retaining ring between the cartridge holder and the cartridge or it is achieved by the application of an adhesive between the cartridge, the retaining ring and the cartridge holder. The adhesive is applied as a separate step which adds a manufacturing step and requires precise control of the application of the adhesive to certain areas of the cartridge holder, the retaining ring and the cartridge. Fixation of housing parts in injection devices using adhesives is generally described in WO9627400.

Another approach for fixation of a cartridge is described in WO09095332, where the cartridge is axially fixated by advancement of a piston rod to contact the plug in the cartridge during manufacturing or assembly, e.g. prior to the actual use. This has the disadvantage that during transport and shelf-life a pressure may be build up within the cartridge since the cartridge is indirectly fixated via the moveable plug that pushes against a non-compressible fluid.

In WO11159930 a damping system is present between a medicament reservoir and a pump housing to prevent excessive agitation of the fluid in the reservoir.

It is an object of the present invention to provide an alternative fixation system for a reservoir, which is simple to use, reliable, cost effective. Furthermore, a method for fixating a cartridge to a cartridge holder is presented and the fixation system combines the features of cartridge fixation, damping the cartridge in the cartridge holder and providing a sterile barrier between a second cavity that is exposed to the ambient during use and a first cavity that needs to remain sterile during shelf-life. Therefore precise application and positioning of the sterile barrier is of importance. The cartridge fixation system fixates the cartridge and compensates for relative axial, and/or radial, and/or rotational movements of the cartridge within the holder while maintaining a sterile barrier.

These objectives are achieved by the method for cartridge fixation as described in claim 1 and the medication delivery device where the cartridge is fixated according to the method, as described in claim 12. Further embodiments are disclosed in the dependent claims.

### General description

The medication delivery device is shaped such that it can be adhered to the skin of a patient, e.g. it preferably has a planar or slightly curved or anatomically shaped bottom surface with an adhesive layer which provides the attachment to the skin of the patient. A patient removes the device from a packaging and removes a protective layer from the adhesive layer prior to attachment. The medication delivery device preferably has an optical indication means which can indicate to a user that the device is ready for injection, with or without a delay time for equilibrating the device and medication to the ambient temperature if the device is taken out of the cold-chain storage conditions. The device preferably has a separate button for starting the device which can be on a top cover of the device or a switch is present on the bottom surface and skin attachment activates the device. As an alternative, also the release of the protection foil for the adhesive can activate an electrical circuit but the start medication delivery is only enabled after attachment to the skin. The control system of the device is such that the medication cannot be delivered before attachment to the skin. Yet another alternative has the option that a sensor, preferably a capacitive sensor is part of or activates the electrical circuitry or the sensor signal is processing by a processing unit which controls the device. The capacitive sensor can be directly present on the bottom surface of the device, below the adhesive layer, for example the capacitive sensor is printed on or attached to the housing of the device. Alternatively, the capacitive sensor is embedded in the carrier foil or supporting film of the adhesive layer. In yet another alternative, the capacitive sensor is attached or glued onto the carrier film or supporting film of the adhesive layer. In yet another alternative, the capacitive sensor is embedded in the adhesive layer or glue itself. The sensor measures a difference in capacity between a non-skin adhered and skin adhered position for device activation (e.g. power the electrical circuit) and/or start of the medication delivery by the delivery mechanism. In all options presented, at least one time delay preferably exists between the removal of the device from the cold-chain, the activation of the electrical circuit, and the start of the medication delivery. The signals from the switches and /or sensors are sent to the processor and depending on the set-up of the software, different time delays can exist depending on the temperature of the device and/or medication, or time from the activation of a switch or command from a separate device. The processing unit of the device can be programmed such, as to prevent the activation of the activation button before a certain waiting time or temperature has been reached. In yet another alternative, the processing unit of the device can be activated and/or controlled via a remote control system such as a bluetooth connection or another wireless network connection (WLAN) or via an RFID or NFC chip.

The optical indicator, for example a LED light, can give different color signals and/or different lighting sequences depending on the status of the device, e.g., not ready for delivery, delivery of medication, delivery finished, needle retracted, and ready for removal from the skin. The status signals can also be sent in parallel to an external device like a cellular phone using an internal transmitter/receiver unit. The housing of the medication delivery device is preferably composed of a bottom and a top cover, the bottom at least having the skin adhesive layer and a passage for the needle or canula. The top cover preferably has at least a viewing window for viewing the reservoir containing the medication.

The device can be subdivided in several modules or sub-units:
- A drive mechanism comprising the control unit: The unit is powered, preferably by a battery and contains the processor for controlling the device. Furthermore the drive mechanism for advancing the piston rod comprises a motor, gearing and coupling mechanism.
- A top cover comprising, preferably, a button and a viewing window.
- A fluid path unit: This unit comprises the mechanical parts for the needle insertion and retraction mechanism, a canula and a spike which are connected to each other via a tubing. A housing comprising a cartridge holder and preferably a cartridge. The fluid path unit is a sterile part and the marriage of the fluid path unit with the cartridge is done under controlled sterile conditions, as will be described below.
- A bottom surface module comprising the bottom surface, preferably, a capacitive sensor an adhesive layer and a cover or peel foil for the adhesive.
The different sub-units can be assembled to form the device at different locations. For example, the fluid path can be assembled at factory A, sterilized at location B whereas the assembly with the sterile cartridge is done at location C. Finally, the sterile fluid path unit containing the medication can be married with the drive mechanism, top and bottom subunits at location D.

The different sub-units in the device provide different functionalities which are either coupled to each other electronically and/or mechanically to provide a reliable and, from a patients perspective, easy functioning of the device.

After the injection is finished, the patient removes the device from the skin, the housing of the injection device and/or the adhesive layer, for example the carrier film of the adhesive layer, may be provided with grips or additional films or lips intended for easy skin removal.

The injection device, preferably a bolus injector or patch injector comprises a housing having a first compartment with a drive mechanism comprising a motor, a gearing mechanism and a piston rod that advances a plunger in a reservoir or cartridge, and a second compartment with the fluid path which can connect the contents of the reservoir to a needle or a canula intended for delivery. The latter compartment has a needle insertion and retraction mechanism and this needle insertion and retraction mechanism is either permanently coupled to the drive mechanism or selectively coupled to the drive mechanism during the insertion and retraction procedure.

For the permanent coupling the drive mechanism is preferably coupled to the insertion and retraction mechanism using a one way ratchet whereby rotation of the drive mechanism in one rotation direction advances the piston rod. In that rotation direction, the one way ratchet system is designed such that the insertion mechanism is not coupled to the drive mechanism. The one way ratchet system, preferably shaped as asymmetric toothings formed directly or indirectly on parts functionally positioned between the drive mechanism and the insertion mechanism, slide and ratchet over each other without a transformation of a torque required to activate the insertion mechanism. The one-way ratchet can be designed as an axial ratchet or as a radial ratchet. In the axial ratchet, the ratcheting element such as the asymmetric teething, point in a direction along the rotation axis. In a radial ratchet, the ratcheting element such as toothings or flexural arms point in the radial direction. Rotation of the drive mechanism in the opposite direction ensures that the asymmetric toothings are pressed into a form fit arrangement such that rotation of the drive mechanism is transmitted to the insertion mechanism and the insertion mechanism or retraction mechanism is activated. The coupling between the drive mechanism and the insertion mechanism is permanent and preferably biased by an elastic spring means. A rotation of the drive mechanism is transmitted to the needle insertion mechanism in one direction only. In the opposite rotation direction the ratchet mechanism ratchets and produces audible clicks indicating to the patient that the medication is being expelled from the device. After the medication has been expelled, the rotation direction of the drive/gearing mechanism is reversed once again to initiate the needle retraction mechanism of the device.

In another aspect of the coupling mechanism, the drive mechanism is selectively coupled to the needle insertion and retraction mechanism during the needle insertion and retraction sequences only, and the drive mechanism is decoupled from the insertion mechanism during dose delivery.

This coupling and decoupling mechanism is embodied in a housing, preferably the first compartment, comprising a first part having a longitudinal axis, which is axially fixed with respect to the housing and rotatable around the longitudinal axis, the first part having a gearing engagement, preferably a threaded engagement, to a second part which is arranged along the longitudinal axis. The gearing engagement between the first part and the second part, preferably the threaded engagement, has a first frictional resistance or friction that needs to be overcome to move the two parts relative to another, by rotation and/or axial translation. The gearing engagement can also be a helical gearing between two toothings with an angle oblique to the rotational axis. The frictional resistance between the two parts depends on the fit between the engaging elements of the two parts, the tolerances, surface topography of the interacting surfaces, type of materials selected and the presence or absence of any lubricating agents such as a silicone oil or Teflon spray. The second part is axially moveable along the longitudinal axis of the first part, preferably the axis of the first part and second part are aligned with respect to each other. Furthermore, the second part is directly or indirectly in a friction fit engagement with respect to the housing, the friction fit engagement having a second frictional resistance. This in contrast to the form fit arrangement as described in the prior art of US9107999 for advancing a piston rod; the friction fit arrangement enables axial shifts, and relative rotation between the first part and the second part whereas a form fit would only allow axial shifts without rotation.

The second frictional resistance is defined by comparable parameters as described above for the first frictional resistance. The coupling and decoupling mechanism is further defined by that the first frictional resistance is below the second frictional resistance, such that a rotation of the first part in a first rotation direction axially moves the second part away from the first part along the longitudinal axis, this being caused by the threaded or gearing engagement between the two parts. During the axial shift, the second part is prevented or at least restricted from rotation due to the friction fit to the housing. Thus during the axial shift the second part either only shifts without rotation or performs a combination of an axial shift and rotational movement. A rotation in a second rotation direction of the first part, which is opposite to the first rotation direction, reduces the axial distance between the first and second part. Once the end of the engaging elements between the first and the second part has been reached, e.g. once the end of the threaded engagement has been reached, the first part and the second part rotate in unison without axial movement, this occurs in one position where the first and second part are moved apart, and cannot be moved further apart due to the end of the threaded or gearing engagement and when the two parts abut each other at the other end of the threaded or gearing engagement. The reduction of the axial distance between the second part and the first part can, preferably result in an axial or radial abutment of the first and second part, or the second and first part can get into a coupling engagement for rotation in unison. Thus rotation in a first rotation direction of the first part axially moves the second part away from the first part until no further axial movement is allowed by the engagement, and the second part co-rotates with the first part and the friction fit engagement does not prevent this rotation, this in contrast to a form fit engagement to the housing. Reversal of the rotation direction implies that the second part axially returns back to the first position until no further axial movement is allowed or the second part abuts the first part and the second part is forced to rotate together with the first part in the second rotation direction.

The coupling mechanism has a threaded or gearing engagement between the first part and the second part and is designed as at least one thread segment present on the first part engaging at least one thread follower present on the second part or as at least one thread segment present on the second part engaging at least one thread follower present on the first part. The thread segments and thread followers each have lengths and once the end faces of the threads or thread segments, preferably radially, abut each other, then a relative rotation and/or axial shift is prevented and the first part and second part rotate in unison. As an alternative, the gearing engagement between the first and second part is a helical gearing, whereby a change of rotation direction of the first part axially shifts the second part to couple or decouple the second part.

The second part is directly or indirectly coupled to the housing via a friction fit engagement, the second part can rotate and/or axially slide with respect to the housing if the second frictional resistance or a threshold frictional resistance has been overcome.

The friction fit engagement can exist directly between the second part and the housing and is, for example, exerted by at least one O-ring, at least one flexural element or a friction coupling. This friction fit engagement can also form a sterile barrier, preferably between a compartment containing the needle insertion mechanism and the drive mechanism. Alternatively, the friction fit engagement of the second part is realized by an indirect engagement to the housing, for example via a third part that is axially fixed with respect to the housing and rotational free arranged along the longitudinal axis, the third part being in a second friction fit engagement with the housing having a third frictional resistance. This third frictional resistance is above the second frictional resistance for a correct functioning of the opening and closure of the coupling. Thus the first part is an engagement with the second part having a first frictional resistance which is below the second frictional resistance between the second part and the third part which is again below the third frictional resistance between the third part and the housing. The cascade of friction fit engagements can be designed such that they all, or at least one of them, form a sterile barrier, preferably between the needle insertion mechanism or a compartment containing a fluid path and the drive mechanism.

In one example, the first, second and third part are designed as concentrically arranged sleeves and the third part surrounds the first and second part, thus the third part has an inner dimension, preferably a circular shape, which fits around the first and second part. The arrangement of the first and second part can be such that the second part surrounds the first part or vice versa the first part surrounds at least partially the second part.

The third part and its second friction fit engagement to the housing preferably form a sterile barrier. The sterile barrier can be designed as a form fit engagement or a press-fit engagement such that the third part is allowed to rotate with respect to the housing without any axial movement. The sterile barrier is preferably formed between the first compartment preferably containing the drive mechanism and a second compartment with the needle insertion mechanism. Alternatively, the second friction fit engagement forms a sterile barrier between the ambient surrounding a subassembly of the device containing the insertion mechanism, and the outside. The sterile barrier can be formed by the fit of the two elements directly or via a separate element, for example the housing or a part of the housing is encapsulated with an elastomer covering the border between the third part and the housing. The elastomer, preferably a TPE is preferably injection molded around the housing or a part of the housing using 2-component injection molding techniques. Alternatively an O-ring can be the separate element for providing a sterile barrier.

In another aspect of the coupling mechanism, the second part is preferably arranged along the longitudinal axis of the first part between the first part and the third part. Thus the longitudinal axis of the first, second and third part are preferably identical for all parts. The second part is moveable towards the third part once the first part is rotated in the first rotation direction, and the second part is moveable towards the first part once the first part is rotated in the second rotation direction. The movements are caused by the combined interaction of the cascading frictional resistances and the threaded engagement between the first and second part. The second part and third part form a one way clutch or coupling, preferably a one-way ratchet system which is closed once the second part abuts the third part and the first, second and third part co-rotate in the first rotation direction. Once the first part rotates in the first rotation direction, the second part axially approaches the third part until the one way coupling is closed and the first, second and third part rotate together. If the first part is subsequently rotated in the second rotation direction, the second part moves axially away from the third part towards the first part thereby opening the coupling. Once the second part abuts the first part, the first and second part both rotate in the second rotation direction. In the alternative described above, a permanent one-way clutch or ratchet exists between the second part and the third part, e.g. without the axially moving second part that is driven by the first part. In that example, the one way clutch is permanently closed or biased by a spring means and rotation in the first rotation direction ensures that the first, second and third part rotate in the first rotation direction. Rotation in the second rotation direction ensures that the second and third part ratchet over each other whereby the second part rotates and the third part is in such a frictional resistance or fit with respect to the housing that the parasitic torque of the ratchet system is below the frictional engagement of the third part to the housing.

A rotation of the third part by closing of the coupling mechanism is used in the present invention to activate a needle insertion and/or needle retraction mechanism. Rotation of the third part in the first rotation direction ensures, via a coupling, preferably designed as at least one cam interacting with another part, preferably a needle control element, that the needle control element rotates over a first angle together with the first, second and third part. Rotation of the needle control element over the first specific angle ensures that the needle insertion mechanism is activated and a needle canula holder moves from the needle retracted to the needle inserted position. During a further rotation in the first rotation direction of the third part, which can be directly after the first rotation in the first rotation direction, but alternatively also after the first part has been rotated over a certain number of revolutions in the second rotation direction, the third part is rotated over a second angle in the first rotation direction, and ensures that the needle control element is rotated and/or translated to activate the needle retraction mechanism.

In a further aspect also a method is presented having a certain sequence of rotation and coupling steps. Preferably first, the first part is rotated in the first rotation direction to close the coupling between the second and third part and rotate the needle control element over a first angle to activate the needle insertion mechanism. Subsequently, the first part is rotated in the second rotation direction whereby the coupling is opened and a piston rod is advanced to expel medication from the reservoir. Once the reservoir has been emptied, the rotation direction of the first part is reversed to the first rotation direction again and the coupling between the second and third part is closed again and the needle control element is rotated over a second angle to activate the needle retraction mechanism.

An additional step to the method of coupling a drive mechanism to a needle insertion and retraction mechanism can be added prior to the first closing of the coupling for activating the needle insertion mechanism. The medication delivery device preferably comprises a reservoir with the medication and the medication is delivered by advancing the plug or plunger present in the reservoir. The advancement of the plunger is accomplished by advancing a piston rod by the drive mechanism. If there is an axial gap between the end of the piston rod and the plunger, it is advantageous to first advance the piston rod by rotating the drive mechanism in the second rotation direction before activating the needle insertion mechanism, which requires a rotation of the drive mechanism in the first rotation direction. Thus the additional step comprises rotating the first part in the second rotation direction to activate the drive mechanism for advancing the piston rod towards the plunger present in the reservoir containing the medication, prior to rotating the first part in the first rotation direction to close the one-way clutch between the second part and the third part. The gap between the end of the piston rod and the plunger can be due to the use of a non-completely filled reservoir. The first advancement of the piston rod can be done in the factory during assembly with the reservoir or afterwards by the patient during use.

The first part is either directly coupled or coupleable to the motor of the drive mechanism or the first part is coupled to the motor of the drive mechanism via a gearing arrangement. In the first option, the motor of the drive mechanism drives the first part via a unidirectional ratchet system, e.g. rotation of the motor in one rotation direction results in a rotation of the first part whereas rotation of the motor in the other rotation direction activates the ratchet, or alternatively a ratchet and pal mechanism such that the first part does not rotate but the ratchet mechanism produces audible clicks. The motor of the drive mechanism uses a separate gearing mechanism for activating the drive mechanism for advancing the piston rod. In the second option, both the first part and drive mechanism for advancing the piston rod are permanently coupled to a single gearing mechanism and rotation of the motor in both rotation directions results in a rotation of the first part and/or rotation of the drive mechanism for the advancement and/or retraction of the piston rod. The gearing mechanism in the first and second option for rotation of the first part can be accommodated to include a no-load stroke before the first part is forced to rotate.

The medication delivery device comprising the coupling mechanism described above preferably is a bolus injector, a patch injector or a patch pump.

The coupling and decoupling mechanism of the above describes the coupling between a drive mechanism and a needle insertion and retraction mechanism. The needle insertion and retraction mechanism and a method for coupling the drive mechanism to the needle insertion mechanism is described in the following for 3 embodiments of the medication delivery device.

It is an aspect to present a needle insertion and retraction mechanism for a medication delivery device for delivering a medicament from a reservoir having a septum, the delivery device having a housing with a bottom surface attachable to the skin of a patient. The reservoir preferably being an ampoule or a cartridge that is filled with a medicament. The cartridge preferably being a cylindrically shaped tube closed on one side by a plunger and on the opposite side by a septum suitable for being punctured by a spike, whereas the medication is located between the plunger and the septum. The reservoir has a longitudinal axis which is preferably oriented parallel to the bottom surface of the device (or the skin surface of the patient) for a space saving arrangement in terms of thickness of the device. The medication delivery device having further a spike inserter carrier comprising a spike for insertion through the septum of the reservoir, the spike inserter carrier being moveable parallel to the bottom surface between a first position where the spike does not penetrate the septum to a second position where the spike penetrates the septum. The spike inserter carrier being biased by a biasing means which intends to move the spike inserter carrier from the first position towards the second position. The biasing means can be, for example, a spring means, a compression spring a coil spring, a wave spring, a leaf spring or the like. Further the needle insertion and retraction mechanism comprises a canula holder for holding a needle or steel canula, the canula holder being guided in the housing for a linear movement perpendicular to the bottom surface between a needle retracted position and a needle inserted position. As an alternative, the canula holder is guided by the housing for an inclined insertion, e.g. at an angle to the normal of the bottom surface which can further reduce the height of the device. The needle retracted position being furthest away from the patients skin and the needle inserted position is defined by the canula position for, preferably, subcutaneous delivery. The canula holder is, for example, injection molded around the canula and preferably made from a polymeric material. The spike inserter carrier has a drive means, preferably a first guiding means, for driving the canula holder from the needle retracted to the needle inserted position. The canula holder preferably has a transformation means which is engaged with the first guiding means, such that the biasing means biases the canula holder for moving from a needle retracted towards the needle inserted position. Furthermore, a needle control element is part of the insertion and retraction mechanism, whereby the needle control element has an arrester or stop which directly or indirectly abuts the canula holder, or a part attached to the canula holder, and which holds the canula holder in the needle retracted position against the force of the biasing means which biases the spike inserter carrier. The needle control element thereby preventing the movement of the spike inserter carrierfrom the first position towards the second position since the canula holder and the spike inserter carrier are engaged with each other via the first guiding means and the transformation means. The arrester of the needle control element therewith also blocks the canula holder from moving from the needle retracted to the needle inserted position.

A fluid path, preferably shaped as a flexible tubing is positioned preferably between the canula or canula holder and the spike of the spike inserter carrier such that a fluid connection exists between the spike and the needle. The spike inserter carrier is laterally guided in the housing by at least one guide slot, preferably a horizontal linear guide, a channel, a groove or protrusion present on the housing or a part of the housing, for example the bottom surface and/or top cover, or the spike inserter carrier is keyed to a subassembly or a housing part, for example to a fluid path unit. The maximum lateral position of the spike inserter carrier sliding through the housing is provided by stops and counterstops present on the carrier and/or the housing. Low friction materials and/ or lubricants are selected to reduce the frictional losses between the spike inserter carrier and the housing. The spike and spike inserter carrier are preferably designed as being made in one step from a single material, preferably via an injection molding process. The spike of the spike inserter carrier is designed as a hollow cylinder having a sharp tip or ending. The side opposite to the tip is attached or attachable to the bottom surface of the spike inserter carrier. The tip is designed for penetrating the septum of the reservoir and therefore requires that the material for the spike and/or spike inserter carrier is a stiff material, for example a high modulus polymer such as PEEK, PPSU, POM or a fibre reinforced polymeric material. As an alternative, the spike is made from a metal which is attached to the bottom surface of the spike inserter carrier in a separate production step. The spike has a longitudinal axis which is preferably arranged parallel to the longitudinal axis of the reservoir with the septum. As an alternative, the reservoir is reversed in the device such that the spike of the carrier penetrates the plunger of the reservoir instead of the septum. In either arrangement, the system is designed that after penetration of the septum or plunger, the medication can flow from the reservoir, through the spike via the tubing to the canula. The spike inserter carrier is guided, preferably by the housing to perform a linear movement which is preferably parallel to the longitudinal axis of the reservoir and preferably the longitudinal axis of both the reservoir and the spike are aligned with respect to each other such that the septum of the reservoir is punctured in the center.

The biasing means for biasing the spike inserter carrier is positioned preferably between the housing or a housing part and the spike inserter carrier such that the carrier intends to move from the position which is furthest away from the septum (or as an alternative from the plunger) towards the septum penetrated position. The biasing means is a spring, a magnetic biasing means, an electromagnetic biasing means, a pyrogenic driven biasing means or a gas driven biasing means. After release of the biasing means, a force is transmitted from the biasing means to the spike inserter carrier which subsequently ensures that the septum or plunger is penetrated. The stop position is either defined by abutment of the base of the spike inserter carrier or base of the spike with the septum, or by a separate stop present on the housing.

The spike inserter carrier comprises the driving means or first guiding means, which is preferably designed as a linear guide for guiding and/or biasing the canula holder via the transformation means. The transformation means is preferably designed as a protrusion which fits into the linear guide of the driving means. The linear guide is arranged oblique to the bottom surface of the carrier or housing such that a lateral movement of the spike inserter carrier exerts a downward movement for the canula holder towards the skin of the patient. The inclination angle of the linear guide with respect to the normal of the bottom surface varies between 20° and 80°, preferably between 30° and 70°, more preferably between 40°and 60°. As an alternative, the driving means is shaped as a non-linear curve, for example as an "S" shaped curve to control the insertion speed and/or force of the canula holder.

Preferably, for moving the canula holder from the needle retracted to the needle inserted position, it is required to release the biasing means and activate the driving means of the spike inserter carrier. In order to achieve this, the needle control element is rotated over a first angle such that the abutment between the arrester of the control element and the canula holder is released. This ensures that the spike inserter carrier is allowed to move from the first position to the second position, driven by the force of the biasing means, and thereby the driving means simultaneously moves the canula holder from the needle retracted to the needle inserted position. The movement of the canula holder towards the inserted position ends once the canula holder abuts a second arrester which can be positioned on the housing, a part attached to the housing, the bottom of the housing but also on the needle control element.

The needle control element preferably has a locking mechanism which locks the canula holder in the needle inserted position once the needle control element has been rotated over the first angle. The locking mechanism prevents that during injection the needle retracts or can be moved by external forces from the needle retracted to the needle inserted position. The locking mechanism is preferably designed as at least one locking arm or latching element present on the needle control element. The part attached or attachable to the locking arm can match a corresponding part, a catch, a cut-out, a flex-arm or latching element present on the canula holder. The parts are designed such that a movement towards the needle inserted position activates the locking mechanism, for example by arms that flex into a locking position. Preferably, the end of a locking arm ends in a tip that matches a recess present in the canula holder.

For retraction of the canula, the canula holder must move from the inserted back to the retracted position. Preferably the needle control element ensures needle retraction and the release of the locking mechanism described previously. The needle control element is preferably rotated further and/or translated in the same direction whereby the needle control element has a needle retraction means. The needle retraction means is preferably designed as a needle retraction arm, which abuts the canula holder once the needle control element rotates over a further angle above the first angle while releasing the locking mechanism and driving the canula holder from the needle inserted position back to the needle retracted position. Alternatively the needle retraction means is not present on the needle control element but designed as a resilient element, for example a spring means present in the linear guide of the housing which is biased or strained by the movement of the canula holder from the needle retracted to the inserted position. In the latter case, latching elements fixate the needle canula holder in the inserted position and the needle control element releases this engagement during the further rotation above, or at a greater angle than the first rotation angle.

The needle insertion is preferably activated or powered by the first guiding means or linear guide of the spike inserter carrier which is sloped with respect to the bottom of the device. Once in the inserted position, a second guiding means which is adjacent to the guiding means becomes available for the transformation means of the canula holder. The lateral movement of the spike inserted carrier is halted such that the second guiding means, which is connected to the first guiding means becomes available for the transformation means. The retraction of the needle is not powered by a further lateral movement of the spike inserter carrier since this would occupy more lateral space and additionally the spike that penetrated the septum prevents any further lateral movement of the carrier. Therefore the retraction of the canula is powered by a different means which can be a spring means or the needle control element which pushes or draws the canula holder back into the needle retracted position. The second guiding means preferably is a guide slot which guides the canula holder preferably vertically back to the starting position. The spike inserter carrier has a second guiding means, preferably adjacent to the first guiding means, preferably designed as a linear guide slot oriented parallel to the normal of the bottom surface, which linearly guides the canula holder back to the needle retracted position. The housing or a part attached to the housing can also have a linear guide slot preferably oriented parallel to the normal of the bottom surface which guides on its own or in combination with the second guiding means the canula holder during needle retraction.

The needle control element is preferably keyed to or engaged with the housing to allow for rotations and/or translations of the needle control element with respect to the housing or a housing part, for example a wall arranged perpendicular to the bottom surface of the device. Therefore, the housing or housing part has at least two guiding means, a third and fourth guiding means preferably designed as third and fourth guiding slots which match at least two corresponding keys, preferably protrusions present on the needle control element. Preferably the keys protrude from the surface of the needle control element and the third and fourth guiding means are designed as grooves in the housing. The longitudinal axes of the guiding slots are preferably oriented at an angle with respect to each other. The needle control element is preferably driven by rotating a third part having engagement means, preferably at least one cam or gear-wheel which is in engagement with the needle control element. A rotation of the third part around its central axis results in a rotation and/or translation of the needle control element as the at least two keys pivot and/or move in the at least two guiding means. The rotations and combined translations are controlled by varying the center of rotation of the needle control element due to the different interactions between the engagement means of the third part and the needle control element. The needle control element itself has a guiding element, preferably a guiding contour or guiding slot, which interacts with the at least one cam or gear wheel of the drive mechanism, preferably with the third part. Thus preferably, the guiding contour is shaped as a non-linearly curved opening penetrating the surface of the needle control element. In a preferred embodiment the two keys protrude from the surface of the needle control element and the guiding contour penetrates the same surface of the needle control element. Preferably the at least one cam or gear wheel of the drive mechanism engage with the guiding contour of the needle control element. The guiding contour is preferably formed as a closed or partially open non-circular loop or curve. The surface of the contour can be smooth but can also comprise a toothing. In the latter case, the interaction between the cams and the contour is a gearing engagement between two engaging toothings. Each of the at least one cam of the drive mechanism or third part can interact with a different part of the guiding contour of the needle control element depending on the angle of rotation of the drive mechanism or third part. The different cam-guide contour interactions enable a variation of the center of rotation, or to control the force vector acting upon the needle control element such that preferably one key of the needle control element initially rotates in one of the two guiding means followed by a combined rotation and axial translation of both keys in the guiding means. The third part is coupled to or coupleable to the drive mechanism using the coupling mechanism described previously.

In a preferred embodiment the first key only rotates in the third guiding means whereas the second key axially slides in the fourth guiding means. This first rotational step corresponds preferably to the needle insertion step. In a second step, corresponding to the needle retraction, the first key and second key both axially translate through the guiding means and due to the angle between the third and fourth guiding means, the needle control element is forced to rotate and translate further over a further or second angle.

The needle control element is preferably rotated and/or translated by the third part preferably having at least one cam which interacts, preferably with the guiding contour of the needle control element. The drive mechanism for rotating the third part comprises an electromotor, a gearing, preferably including a worm wheel arrangement to reduce the number of revolutions of the treaded rod and increase the torque that can be transmitted. Alternatively, other power packages can be used for the drive mechanism such as gas driven systems, springs, electromagnetic drive trains and the like.

For insertion and retraction of a needle of a medication delivery device having the needle insertion and retraction mechanism described above, a method is presented comprising the steps of:
a) Rotation of the needle control element over the first angle to release the abutment between the arrester and the canula holder to move the canula holder from the needle retracted to the needle inserted position, and simultaneously insert the spike into the reservoir,
b) Injection of the medicament present in the reservoir via the fluid path using the drive mechanism of the medication delivery device,
c) Rotation and/or translation of the needle control element over a further angle to move the needle from the needle inserted position back to the needle retracted position.
The method may also comprise the coupling sequences for coupling the drive mechanism to the needle insertion mechanism; preferably before step (a), the coupling mechanism needs to be closed and the first part rotates in the first rotation direction, thereby moving the second part to the third part, the third part preferably having the cams that interact with the guide contour of the needle control element. The movement of the second part couples the second part to the third part and the one-way ratchet is closed. The third part also rotates the needle control element in the first rotation direction over a first angle to release the arrester of the needle control element from the canula holder and initiate step a) of the method. Preferably for step b) of the method, the rotation of the first part of the coupling mechanism is reversed to the second rotation direction and consequently the coupling is opened and the third part will not co-rotate with the first part leaving the canula holder in the needle inserted position. The reversal of the rotation direction rotates a threaded rod which will advance the piston rod for medication delivery. Preferably after emptying the cartridge, step c) of the method is initiated, and the rotation direction of the drive mechanism is reversed to the first rotation direction again. The coupling mechanism between the first, second and third part is closed and the cams of the third part rotate and translate the needle control element such that the locking mechanism for the canula holder is released and that the needle canula holder is translated back to the needle retracted position, preferably by the needle retraction arm. In an alternative method, the drive mechanism is activated such that preferably prior to step (a), the piston rod advances without releasing the needle insertion mechanism to reduce an axial distance between the piston rod and the plunger or plug in the reservoir.

A second example for the needle insertion and retraction mechanism is described below. The needle insertion mechanism is suitable for an injection device for delivering a medication from a reservoir having a septum, the needle insertion mechanism being embodied in a housing having a bottom surface that is attachable to the skin of a patient. The mechanism has a steering drum having a rotation axis which can be parallel, perpendicular or inclined to the bottom surface. The steering drum is biased for rotation in one rotation direction by a biasing means, preferably a spring means, a magnetic or electromagnetic biasing means or by a motor. The steering drum has a guiding means, preferably a guiding slot on the walls extending parallel to the rotation axis, furthermore, the steering drum has a first and second arrester located on the walls extending parallel to the rotation axis. Preferably, the first and second arrester are radially pointing in the outward or inward direction and are preferably positioned at different angles and/or axial positions of the walls extending parallel to the rotation axis of the steering drum. The needle insertion mechanism has a canula holder for holding a needle or canula, the canula holder being linearly guided by the housing and moveable from a needle retracted to a needle inserted position in a direction perpendicular or inclined to the bottom surface of the housing. The canula holder being directly or indirectly coupled to the steering drum and/or guiding means by a transformation means, the transformation means preferably shaped as a linear protrusion present on the canula holder and matching the guiding means. The guiding means is designed such, or preferably has an inclination angle with respect to the rotation axis of the steering drum, to bias or drive the canula holder for moving from the needle retracted towards the needle inserted position.

The needle insertion and retraction mechanism has a stop means which abuts the first arrester such that the steering drum is prevented from rotating in the one rotation direction and consequently the needle canula holder remains in the needle retracted position.

Preferably the needle insertion mechanism whereby the stop means is rotated over a first stop means angle which releases the abutment between the stop means and the first arrester such that the steering drum is rotated in the one rotation direction over a first angle by the biasing means until the second arrester catches the stop means. During rotation of the steering drum over the first angle, the canula holder moves from the needle retracted to the needle inserted position and is driven by the interaction of the guide means engaging the transformation means.

The guiding means on the steering drum is preferably shaped preferably as a sinusoidal curve on the inside or outside wall that extends parallel to the longitudinal axis, whereby the transformation means follows the first part of the preferably sinusoidal shaped curve from the maximum to the minimum as the steering drum rotates over the first angle. The needle canula holder moves due to the gearing engagement from the needle retracted to the needle inserted position.

The needle insertion mechanism preferably enables a further rotation of the stop means around its rotation axis, whereby the stop means is rotated further to a second stop means angle which is greater than the first stop means angle and which releases the abutment between the stop means and the second arrester such that the steering drum is rotated over a second angle due to the biasing force of the spring means. During the further rotation of the steering drum, the transformation means of the canula holder follows the second part of the guiding means, preferably the sinusoidal curve from the minimum to the maximum and the canula holder moves back from the needle inserted to the needle retracted position. During this movement, the canula holder is linearly guided by the housing or by a part attached to the housing.

The needle insertion mechanism preferably has a gearing which is attached or attachable or connectable to the steering drum and arranged along the rotation axis of the steering drum, the gearing is preferably designed as a toothing or gear wheel, which engages a spike carrier, preferably having a matching second toothing. The spike carrier is, preferably linearly guided by the housing and moveable from a first spike carrier position to a second spike carrier position such that when the steering drum rotates over the first angle, the spike carrier is moved from the first to the second position. The spike carrier comprises a spike which penetrates the septum of the reservoir when the spike carrier moves from the first spike carrier position to the second spike carrier position. Preferably, the gearing is rotationally and axially fixed with the steering drum.

The gearing of the steering drum preferably comprises an arrester which abuts a second arrester, preferably designed on the housing or spike carrier and limits the rotation of the steering drum when the stop means is rotated over the second stop means angle and limits the rotation of the steering drum for not going beyond the second angle.

The stop means has at least one counter-arrester which can interact with the first arrester and/or second arrester of the steering drum. The counter-arrester is, preferably is a semi-circular arch or rim or half-moon shaped such that a rotation of the stop means over a first stop means angle releases the abutment between the first arrester of the steering drum and the counter-arrester, but the rotation over the first stop means angle ensures that the counter-arrester is within the path of rotation of the second arrester as the steering drum rotates and consequently can abut the second arrester. Thus the arch shaped counter arrester is rotated to release the abutment with the first arrester whereas the arch is within the line of rotation of the second arrester. The stop means with the counter arrester is, preferably attached or attachable to an axis and the axis is coupled or coupleable to a drive mechanism of the injection device. Preferably, the stop means is directly or indirectly coupled to the third part and the sequence of rotations in the first and second rotation direction, as described above for the first example, ensures that the stop means is coupled to the drive mechanism and rotated to release the needle insertion mechanism and the spike insertion mechanism. The canula holder is moved from the retracted to the needle inserted position prior to delivery of the medication and moved from the needle inserted to the needle retracted position after delivery of the medication. During the movement of the canula holder to the needle inserted position, the spike simultaneously penetrates the septum of the reservoir and remains inserted in the reservoir during retraction of the needle.

In the third example, the transformation means is a lever arm for guiding the needle canula holder and the transformation means is engaged with a gearing mechanism present on the rotational axis of the steering drum. Alternatively, the transformation means is activated by a rotation of the steering drum by abutment of a protrusion or rim present on the steering drum with a protrusion or rim present on the lever arm.

The stop means blocks rotation of the steering drum after insertion of the needle. Preferably a first stop is present on the lever arm or lever arm mechanism which abuts with a counter stop on the steering drum such that the rotation of the lever arm is stopped in the needle inserted position. Further rotation of the steering drum after releasing the abutment between the stop means and the first arrester of the steering drum drives the engagement between the first stop on the lever arm and the counter stop on the steering drum, such that the lever arm is rotated back and drives the canula holder from the needle retracted to the needle inserted position. Preferably, a gearing is attached to, or attachable to the steering drum for driving the spike inserter into the septum of the reservoir.

The drive mechanism comprises a piston rod for delivering the medication form the reservoir. In the examples presented, the piston rod is a segmented piston rod and/or bended or wrapped for a space saving configuration in the device, but also a rigid or straight or non-segmented piston rod configuration can be combined with the needle insertion and retraction mechanism presented.

An aspect of the medication delivery device is a segmented piston rod for delivering a medication from a reservoir having a plunger, the piston rod comprises multiple segments that each are joined together via a hinge located laterally of each segment or between adjacent segments. The piston rod can be bent by rotation of adjacent segments around the hinge or articulated in one direction such that subsequent hinges are opened to form a curved piston rod. Preferably, the segmented piston rod thus can be bent in one direction only. An axial force can be transmitted by the segmented piston rod to the plunger in the reservoir via the subsequent hinges of the segmented piston rod. When the hinges between subsequent segments are closed, the segments form a stacked or linear configuration for entry into the reservoir, the stacked arrangement prevents buckling of one or more segments of the piston rod. With the segmented and curved piston rod arrangement, a space saving arrangement of the piston rod in the device can be accomplished. The segmented piston rod is preferably driven by a threaded rod that rotates and engages with at least the first segment of the segmented piston rod. The first segment of the segmented piston rod is defined as being furthest away from the last segment of the segmented piston rod, the latter contacts the plunger of the reservoir. The segmented piston rod is secured against rotation with respect to the housing around its longitudinal axis and the at least first segment of the segmented piston rod is equipped with an internal thread matching an external thread of a drive sleeve or threaded rod, wherein rotation of the drive sleeve or threaded rod by the drive mechanism advances the segmented piston rod towards the plunger of the reservoir. The segmented piston rod is preferably guided in the housing or a part connected to the housing such that the segmented piston rod is rotationally secured with respect to the housing. For example, the segments of the segmented piston rod have a non-circular cross section matching a guidance or passage within the housing or a part connected to the housing. Alternatively, each of the segments of the segmented piston rod has a groove, notch or wing matching a complementary protrusion or groove in the housing to prevent rotation of the piston rod.

The piston rod comprises multiple segments that are each joined together via a hinge, preferably a strap hinge located on one side, preferably the lateral side of each segment. The piston rod can be bent in one direction thereby opening subsequent hinges between the segments, while an axial force can be transmitted to the plunger when subsequent hinges are closed and the segments abut each other on the opposite side of the hinge. The last segment of the segmented piston rod abuts the plunger of the reservoir, preferably with a flange or a connector for connecting to the plunger of the reservoir. The last segment has a guiding element which guides the last segment in, or into, the reservoir, whereby the guiding element is designed for abutting the inner lateral wall of the reservoir and guide the last segment such that the normal of the element is parallel to the longitudinal axis of the reservoir. The reservoir preferably has a tubular or cylindrical shape and preferably is made from glass or a polymeric material. The normal of the last segment is defined as perpendicular to the plane that abuts the plunger. When the last segment enters the opening of the reservoir, the opening being defined on the side opposite to the septum, then the last segment can enter under an angle and initiate a so-called piston squeezing and block delivery of medication. Therefore and for correctly connecting to the plunger in the reservoir, the guiding element ensures that the normal of the last segment is parallel, preferably equal to the longitudinal axis of the reservoir. The guiding element can be guided by the housing or a housing part prior to entering the reservoir, e.g. already ensures that the last element correctly enters the opening of the reservoir.

The guiding element protrudes from the last element towards the second to last element and is oriented parallel to the normal of the last segment. Preferably, the guiding element is shaped as a fin that is attached to, or attachable to the last element, and one side of the fin is parallel to the longitudinal axis of the reservoir when the last element is completely enclosed by the reservoir. During entry of the last element into the opening of the reservoir, the guiding element functions as a lever arm and thereby orients the flange of the last element parallel to the end surface of the plunger that is not in contact with the medication. Before entry, the fin can already be guided towards correct entry, preferably by the housing or by a housing part. The segmented piston rod for an injection device whereby the guiding element acts as a lever arm on the last segment and ensures that the normal of the last segment is parallel to the longitudinal axis of the reservoir when the last segment abuts the plunger of the reservoir. The fin of the last segment points towards the second to last segment, but is not directly engaged or coupled to the second to last segment, e.g. it does not interfere with the bending or articulation range of the hinge between the two last segments. The hinge between the last two segments ensures that the two segments can articulate around a hinge axis. The guiding element or fin pointing towards the second to last segment is preferably an integral part of the last segment, for example made as a monolithic part during injection molding. The guiding element or fin is preferably located opposite of the hinge and is preferably oriented perpendicular to the hinge axis of the last two segments. Once the segmented piston rod is in a stacked configuration after entry in the reservoir, also the segments not having a guiding element are preferably guided by the inner wall of the reservoir. The segmented piston rod thus can transfer axial loads via the hinges to the last segment abutting the plunger.

Preferably, the medication delivery device is controlled by a control unit having a processor. The control unit controls the several steps during the delivery process, e.g. the rotation direction of the drive mechanism. The device is activated by closing first the electrical circuit of the system which includes the control unit since the device is preferably not powered during shelf-life. The activation of the electrical system can be done by a remote control, a mechanical switch an RFID circuitry or a sensor such as the capacitive sensor. Alternatively the electrical circuitry is automatically switched and ready for delivery once the device is removed from its packaging or cardboard box or when the user removes the liner for the adhesive layer. The control unit gets inputs from sensors such as the capacitive sensor or switches, or temperature sensors to indicate that the device is ready for delivery, e.g. attached to the skin of the patient and can get started by the inputs from the sensors, or by a separate mechanical switch such as a button being present on the device, preferably in or on the top cover. The control unit can receive information from internal sensors such as a temperature sensor, the capacitive sensor, the electrical circuitry, pressure or the encoder of the electromotor in the drive system. The control unit preferably includes a timer or a clock. The processing unit, preferably is equipped with a sending/receiving unit to connect to other external devices such as smart phones or a remote control unit. The connection can be established, preferably, by a Bluetooth or more preferably Low Energy Bluetooth connection protocol. The processor thus can also react to inputs from external sensors or remote control devices or smart phones. The device can be operated without the external control unit as is, thereby only using switches and/or sensors present in the device. The sending and receiving unit and the remote control are complementary features. The data received by the processor are preferably stored in a storage medium, such as date, time, injection time, injection volume, temperature, time between removal from the cold-chain and start of delivery, any errors during delivery such as occlusion, the time lapsed between delivery of the medication and the needle retraction or removal of the device from the skin of the patient, number of revolutions of the drive system, rotation directions and the like. Those data can be sent from the device or storage medium of the processing unit to a cloud server or a smart phone. Thus the communication between the medication delivery device is either one-way whereby the device sends data without receiving signals or a two-way communication whereby the medical device communicates and receives commands or data from the external device or system such as a network. The communication with the external device or system follows a secured and encoded protocol to prevent interference with other external devices, e.g. not the preferred external device which preferably has been connected and allowed to communicate with the medication delivery device. In a preferred example, the device is approached by a device having a Near Field Control (NFC) chip, which can activate the main electrical circuitry and/or start the injection and/or start data transmission or communication. The data received from the device, either directly or indirectly via the cloud server can be used by the patient, a medical practitioner or a health care professional.

The medication delivery device is after production and assembly with the medication reservoir stored under cold conditions since most of the medicaments require cold storage before use to increase the longevity and guarantee the shelf-life time. The storage conditions can be in a freezer, e.g. below 0°C or in in a refrigerator, which is slightly above 0°, e.g. 5°-8°C. This effects both the viscosity of liquid medications as well as the dimensional tolerances of the diverse components that mechanically interact in the device due to the different thermal expansion coefficients of the materials used. Generally, the viscosity of most medicaments, for example solutions containing large protein molecules retrieved from recombinant DNA techniques, increases as the temperature decreases. To compensate for this, the control unit of the device can adjust the speed of delivery depending on the temperature within the device, thus the rotational speed of the drive mechanism or change the gearing ratio such that cold medication is delivered at a lower speed and/or with a higher force for advancing the piston rod. Alternatively, the medication is heated prior to the start of the injection procedure. This can be accomplished passively by waiting for a certain time after removal from the refrigerator but also actively by a heating source present in, or surrounding the medical device. The medication in the reservoir can be heated by an infrared source either present on the outside to heat the medication via the viewing window, or it is present on the inside. Heating sources used can be, but are not restricted to an IR source, a heating element surrounding the reservoir or the tubing or a heat exchanger encapsulating the tubing, microwaves, heater bags using exothermic crystallization or oxidation to generate heat. Also the battery itself can be used as a heat source to the medication and/or fluid path. The heat generated can be transferred to the reservoir or to the fluid path, e.g. in the latter case the medication is heated just prior to injection. In another example, the body heat of the patient is used to either heat the reservoir, the fluid path or an intermediate element.

The medication delivery device delivers the medication to the patient under aseptic conditions which means that the sterility of the fluid path unit and the connection to the cartridge during production and shelf-life must be guaranteed.

In a first example, the fluid path unit is produced and assembled, preferably in a clean-room environment, for example GMP EU classification Grade C. The fluid paths are packaged in a peel pouch or a blister, either individually or stacked in a tray or tub. Preferably, fluid path units are assembled in a tray that is put into a tub which is subsequently packaged in at least two-fold peel pouches, optionally, these fluid path units comprise the cartridge or reservoir. The peel pouches can be air tight for subsequent gamma sterilization or the peel pouches are made from gas-permeable membranes such as Tyvek, and subsequently sterilized using Ethylene Oxide or Gas Plasma Sterilization (for example H2O2, or NOx). The packaged tubs are shipped to a fill-finish company that brings the tubs with the fluid paths in an aseptic environment, for example GMP EU classification Grade A, by first removing the first peel pouch of the tub, sterilize the surface for example by using e-beam and bring the tub into the aseptic environment via a double-door system. The same track for entering the aseptic environment is followed for the empty and sterile cartridges that are also arranged in tubs enclosed in a double peel pouch. Also the cartridges are brought into the aseptic environment. The empty cartridges are then aseptically filled and stoppered in the aseptic environment and finally assembled with the fluid path units in an aseptic environment. The sterile fluid path units comprising the filled cartridge can now be assembled with the drive mechanism, bottom and cover sub- units outside the aseptic environment.

In a second example, the same path is followed, except for the fact that the sterile fluid path units in the tubs are shipped to the company producing the reservoirs or ampoules. That company enters the tubs into their aseptic environment after removing the first pouch and sterilizing the tub after unwrapping, and inserts the empty cartridges into the fluid path. The fluid paths in the tubs are packaged and sterilized again and shipped to the company doing the aseptic filing in an aseptic environment. After filing, the sterile fluid paths are ready for assembly outside the aseptic environment, for example in the clean room.

In the first two examples, the reservoir and the fluid path unit are both sterilized before entering the aseptic environment and are assembled in the aseptic environment which means that the space between the spike and the septum of the reservoir is also sterile. In the last example, the reservoir and the fluid path unit are both assembled in a different class environments and after assembly of the two, the space between the septum of the reservoir and the spike is sterilized in a separate step. In the third example, the fluid path is preferably assembled in a clean room environment and the reservoirs are filed in an aseptic environment. The two are assembled in a clean room environment which implies that the interspace requires an additional sterilization step, for example using ETO or Gas Plasma sterilization, the housing part is designed such that the intermediate space can be sterilized in a separate and preferably final step, for example using ETO or gas plasma sterilization.

A medical device for injection medication from a reservoir having a septum which is attachable or attached to the skin of a patient which comprises:
- A fluid path comprising a spike for insertion through the septum of the reservoir, a canula holder for holding a canula or needle for insertion into the patient and a tubing for a fluid connection between the spike and the canula holder, the fluid path being enclosed in a sterile enclosure during shelf life of the medical device,
- A fluid path housing enclosing the fluid path and having at least one passage for the needle or canula,
- A bottom surface which is either part of the fluid path or a separate part, the bottom surface having an adhesive layer for mounting to the skin of the patient,
whereby during injection of medication the canula or needle and/or the spike communicates with the outside of the sterile enclosure, characterized in that the sterile enclosure at least partially encloses the adhesive layer during shelf-life. Preferably, the adhesive layer is covered by a peel foil during shelflife of the device, and which preferably forms part of the sterile enclosure. The adhesive layer at least partially encloses or surrounds the bottom surface of the medical device. The peel foil of the medical device preferably comprises a main peel foil and a sticker, the sticker connecting to another sterile foil covering the passage to the ambient of fluid path housing.

A method for manufacturing and assembly of a medical device comprising the steps of:
- Providing a fluid path comprising a spike for insertion through the septum of the reservoir, a canula holder for holding a canula or needle for insertion into the patient and a tubing for a fluid connection between the spike and the canula holder, the fluid path being enclosed in a sterile enclosure during shelf life of the medical device and whereby the fluid path is enclosed in a fluid path housing with a bottom surface that is attachable to the skin of a patient
- Providing a drive mechanism which is connectable to a needle insertion and retraction mechanism present in the fluid path housing and which initiates the insertion of the needle into the patient
- Providing a cartridge with medication that can be inserted into the fluid path housing
- Providing a top cover for the medical device that matches the fluid path housing
Assembling the fluid path housing comprising the fluid path, the drive mechanism, the cartridge and top cover to form the medical device

A method for sterilizing a fluid path unit for an injection device comprising the steps of:
a) Providing an empty cartridge having a glass barrel and a septum connected to the distal end of the glass barrel using a crimp, the glass barrel preferably being cylindrically shaped having a reduced diameter section for receiving the septum. The septum is located at the distal end and is attached to the glass barrel by a crimp, which can be a metal or plastic crimp which fixates the septum to the glass barrel.
b) Providing a fluid path comprising a spike or a canula, preferably a metal canula, adapted for piercing the septum of the cartridge and a needle adapted for piercing the skin of a patient, the spike and needle being connected preferably by a tubing. The fluid path unit has a first cavity encapsulating the spike, the needle and preferably the tubing; furthermore, the fluid path unit has a second cavity adapted to receive the distal end of the cartridge. The first and second cavity being connected to each other and having a passage there between adapted to receive the spike of the fluid path.
c) Inserting the empty cartridge into the second cavity of the fluid path unit, the empty cartridge with the septum and crimp is preferably inserted such that the distal end of the cartridge first enters the second cavity and preferably the empty cartridge is axially aligned with a longitudinal axis of the second cavity.
d) Subsequently, the empty cartridge is fixated or attached to the fluid path unit, followed by
e) Sterilizing the first cavity of the fluid path unit. At least the first cavity is sterilized but as an alternative, both the first and the second cavity are simultaneously sterilized. The first cavity can be sterilized using gas or plasma sterilization and the first cavity is preferably flooded or flushed with the sterilization agent via the opening that is adapted for receiving the needle when the needle penetrates the skin of the patient. That opening is preferably covered with a gas permeable membrane such as Tyvek which forms a sterile barrier that is penetrable by the gaseous sterilization agent.
   The method is further characterized by that the empty cartridge having a glass barrel and a septum connected to the distal end is provided with a sterile interface between the glass barrel and the septum prior to step a) and that
f) the empty cartridge is filled with a medicament after step e)
These method steps are preferably executed in an alphabetic order, e.g. step c) follows step b)

The empty cartridge having a glass barrel and a septum connected to the distal end is preferably sterilized as a unit prior to step a), and is preferably sterilized using steam sterilization, NOx, gas plasma, ETO , X-ray, dry heat sterilization or gamma sterilization. The steam sterilization is preferably done in an autoclave using a temperature of 134°C and the dry heat sterilization at a temperature preferably above 160°C. For the plasma sterilization preferably hydrogen peroxide or ozone is used. If the septum is coated with a coating preventing the release of leacheables (for example a fluoropolymer based coating, such as a fluro-tec coating), then the autoclaving step is preferred since the barrier coating for preventing components leaching out of the septum is also a barrier for gases penetrating through or along the coating from the outside.

The empty cartridge is inserted in step c) into the fluid path unit and this insertion step is preferably done in a non-sterile environment, such as a clean room environment. Alternatively step c) is done in an aseptic environment. Step c) can be done at the same geographical location as the manufacturing location of the fluid path unit but can also be done at a different geographical location. To have a maximum flexibility in the assembly process, steps b), c), e) and f) are done at one location, preferably at more than one location.

The method for sterilizing a fluid path, whereby during sterilization step e), at least the first cavity is sterilized, the sterilization is preferably done using gas plasma, ETO or NOx sterilization. Both the first and the second cavity can be sterilized in the sterilization step e), and as an alternative, the whole fluid path unit with the assembled empty cartridge is sterilized in one step. If the fluid path unit including the empty cartridge is sterilized, also the inner wall surface of the glass barrel and the surfaces of the septum that are exposed to the sterilization gas are sterilized. With this sterilization step, the direct interface between the septum and the glass cannot be sterilized since the gas cannot penetrate the interface. If the septum is additionally coated with an anti-leaching coating, then it is eventually more difficult to sterilize the interface and the surface of the septum. As a consequence, it is preferred to have the assembled and empty cartridge sterilized prior to insertion into the fluid path unit. The steam sterilization step prior to the insertion ensures that also the direct interface between the septum and the glass barrel is sterilized. This interface will remain sterile even if the empty cartridge is removed from the autoclave and is subsequently inserted into the fluid path unit in, for example, a non-sterile clean room environment.

The empty cartridge that has been inserted into the second cavity of the fluid path unit is filled in step f) of the method and is preferably done in a sterile (aseptic) environment of a fill finish line.

As an alternative, the empty cartridge is not autoclaved prior to step a) of the method. To ensure that the interface between the septum and the glass surface is sterile, also both components can be washed with a sterilizing fluid such as alcohol, peracetic acid solution, or a formaldehyde solution or the like. The surfaces of the glass barrel, the septum and preferably also the crimp are first rinsed with the solution then assembled using the crimp and subsequently dried. The drying step can also done before the assembly or attachment of the septum to the glass barrel. The thus obtained sterile components comprising the septum, the glass barrel and the crimp need to be assembled in a sterile environment.

After filing the empty cartridge in an aseptic environment (step f), a plug or stopper is inserted into the cartridge. The plug or stopper can be advanced by the drive mechanism of the device, for example by the segmented piston rod to expel medication from the device.

The first and the second cavities are connected to each other, for example the first and second cavities are formed by a wall separating two sections of a box. There is a passage or opening between the two cavities which is sized such that the spike (or septum canula) of the fluid path can go through the passage and puncture the septum of the cartridge. There is an opening or passage between the two cavities such that normally gas and/or liquid can flow from one cavity to the other and vice versa. The passage or opening between the first and second cavity is preferably circularly shaped. During the insertion step d) of the method, a sealing is established between the first cavity and the second cavity which forms a leak proof connection. The sealing surrounds the passage and is preferably located at the side of the cartridge. Thus if the leak proof sealing is established during step c) then a gaseous sterilization agent entering the first cavity cannot enter the second cavity via the passage during subsequent step e) of the method. Vice versa, any gaseous or liquid component entering the second cavity after step c) cannot move to the first cavity due to the sealing. The sealing provides a gas tight and liquid tight - sterile barrier.

Preferably, the second cavity comprises a cartridge holder which comprises at least one circumferential protrusion that surrounds the passage between the first and second cavity and which deforms the septum of the cartridge during insertion step c), or whereby the cartridge holder comprises an O-ring that forms the sealing between the first and second cavity. During step c) the cartridge is inserted into the cartridge holder with the septum oriented towards the second cavity, once inserted, the circumferential rim penetrates and elastically deforms the septum such that the combination of the circumferential rim and the septum forms a leak proof sealing. Alternatively, the second cavity has a circumferential rim of an elastic material, which is shaped such that the material of the second cavity is elastically deformed by the distal end of the cartridge, preferably the crimp of the cartridge. The elastic material forms a sealing on the axial end of the crimp or seals the radial walls of the crimp or seals both surfaces of the crimp. As an alternative, the sealing component is present as a separate part such as an O-ring present between the cartridge and the cartridge holder and axial movement of the cartridge elastically deforms the O-ring to form a leak proof sealing between the first and the second cavity. The sealing is thus provided between the septum of the cartridge and the second cavity directly or via a separate and preferably elastic element. The separate and elastic element is either injection molded into the second cavity or it is an O-ring or an elastic element that is applied to the crimp of the cartridge prior to insertion into the second cavity.

As yet another alternative, at least one sealing is present between the outside of the glass barrel and the cartridge holder, for example a circumferential sealing such as an O-ring is sandwiched between the glass barrel and the cartridge holder. The sealing can be located at the cylindrical wall section of the glass barrel and/or at the neck portion of the cartridge.

The leak proof connection is tested after step d), preferably using a pressure difference between the first and second cavity. This ensures an in-line process control of the quality of the sealing between the first and second cavity after insertion of the cartridge. A certain pressure is applied to one of the first or second cavity and it is established whether there is no pressure drop in that cavity due to leakage. Alternatively, the leak proof connection is tested, for example, using a trace gas such as helium that is present in one of the first or second cavity on one side of the sealing, and trace gas concentration is measured on the other side of the sealing in the other one of the first or second cavity. The concentration is measured with adequate techniques such as Infrared spectroscopy, laser spectroscopy or gas chromatography techniques.

The medicament used in filling step f) for filling the empty cartridge is preferably a liquid medicament or formulation. Alternatively also solid medicaments can be filled into the empty cartridge such as microspheres. For solid medication, the solid needs to be reconstituted prior to the injection with the patch device, e.g. a solvent for the solid medicament is preferably filled into the empty cartridge after a plug has been inserted for the first medicament. Such a system is known as a two chamber cartridge. In yet another alternative, a solution of the medicament is filled into the cartridge and afterfixation of the cartridge in the fluid path unit, the system is subjected to a lyophilisation step to remove the liquid and obtain a solid medicament. In this case also the option with a two chamber mixing cartridge with a so-called by-pass is preferred, e.g., inserting a stopper after lyophlisation, filling with the reconstitution liquid and using a second stopper to close the cartridge.
Once the liquid medication or solid medication is filled into the empty cartridge, a plug is inserted after step f) of the method to close the cartridge and provide a body that can be pushed forward during the injection step and thus inject the medication.

An injection device that is preferably attachable to the skin of the patient which comprises:
- a drive unit that is adapted to advance the plug in the cartridge containing the medicament
- a needle insertion unit that is adapted for inserting the needle into the patients skin and the spike into the cartridge
- a control unit controlling the needle insertion unit and the drive unit
- a fluid path unit sterilized according to one of the methods for sterilizing the fluid path unit described above.

A method for fixating a cartridge adapted for receiving a medicament, the cartridge is fixated to a cartridge holder of an injection device, and the method comprises the steps of:
a) Providing a cartridge having a cylindrical glass barrel, or alternatively a plastic barrel, and a pierceable septum that is connected to the distal end of the glass barrel using a crimp, or another suitable connecting means known in the art.
b) Providing a cartridge holder, the cartridge holder can be an integrated part of the injection device such as a patch pump. The cartridge holder can also be part of a needle insertion and retraction unit. Alternatively, the cartridge holder is a separate part that is inserted into the injection device, preferably after the cartridge has been inserted into the cartridge holder being a separate part.
c) Inserting the cartridge comprising the septum and crimp into the cartridge holder of step b) such that the distal end of the cartridge with the septum ahead first enters the cartridge holder. The distal end is received by the second cavity that is dimensioned and shaped to receive the neck portion of the cartridge with the crimp and septum. The second cavity is part of the cartridge holder and preferably closed by an end-wall and lateral side walls. The end wall preferably has a passage or opening to the first cavity.

The method is further characterized by that the cartridge is axially (e.g. along the longitudinal axis of the cartridge) and rotationally (e.g. rotation around the longitudinal axis) fixed after the insertion step c). The fixation is such that preferably also lateral movements perpendicular to the longitudinal axis are controlled, damped and/or restricted. The cartridge is fixated by at least one elastic attachment means for attaching the cartridge to the cartridge holder.

Prior to step c) of the method, the surfaces of the cartridge, the cartridge holder or the crimp can be treated or modified to enhance the fixation by the at least one attachment means. The surfaces can be cleaned, for example with a detergent, alcohol, acetone or a suitable solvent to remove any contamination, for example silicone oil, from the surface that could decrease the attachment properties of the attachment means. The surfaces can also be roughened with sanding, blasting or an embossing step, the latter preferably being integrated with the injection molding of the cartridge holder. Alternatively, the surfaces are flamed or etched to remove any contaminants from the surface and/or increase the roughness. In yet another alternative, the surfaces used for fixation are treated with a plasma such as an oxygen or hydrogen peroxide plasma or are treated with UV light. These surface treatment methods ensure that the surfaces are clean and preferably have a good wettability for the attachment means.

In an example, the elastic attachment means is an elastic adhesive or at least one multilayered system comprising at least one adhesive surface and at least one elastic core layer, preferably being an elastic foam layer. The elastic adhesive is flexible and remains flexible after the insertion step c) of the method. This ensures that the flexible adhesive between the two surfaces ensures that dimensional tolerances are compensated for, or that movement of the cartridge, for example during an impact (when the device drops on a floor), is dampened. The elastic adhesive preferably behaves like a rubber like material. The elastic adhesive is a polymeric material and is selected, preferably from the group of epoxies, polyurethanes, polypropylene (preferably a-tactic PP), polystyrenes, ABS, PVC, acrylic based polymers such as PMMA, natural rubber based elastomers, polyisoprene and the like. The material can be used as such or as a blend with a filler such as, for example silica. The elastic adhesive can be applied in the form of a hot-melt (with a gun) or as a solid part that is subsequently melted during or after insertion step c) of the method. The use of the solid part is advantageous for the handling and accurate positioning of the elastic adhesive. If used as a solid part, the polymeric material can be molten to express its adhesive properties after the positioning. The material can be molten by heat, Infra-Red light, microwaves or ultrasonic waves. Alternatively, a curable elastic adhesive is used which is cured by heat, light (UV or IR) after the cartridge has been inserted into the second cavity. The elastic adhesive remains elastic after the fixation process and, although being made of one material, it functionally can be seen as comprising a core layer having elastic properties and two adhering layers that attach to adjacent surfaces. The conceptual layer thickness of the core layer is above the layer thickness of the adhesive surfaces, to ensure sufficient volume to compensate for tolerances or relative movements between the parts. The total layer thickness of the elastic adhesive is below 4 mm, preferably between 0.1 mm and 3 mm, more preferably between 0.5 and 2 mm.

The movement of the cartridge is restricted by the device. The cartridge is preferably enclosed in a compartment which can be the cartridge holder or rigid parts forming a cavity that behaves like a cartridge holder. The minimal movement within the compartment is given by the cartridge holder or rigid parts surrounding the cartridge, and the tolerance field of the cartridge itself. The minimum dimensional tolerance of the cartridge combined with the maximum dimensional tolerance of the surrounding rigid parts or holder define the maximum play of the cartridge within the compartment. The maximum tolerance of the cartridge combined with the minimum tolerance of the surrounding compartment or rigid parts define the minimum play for the cartridge. The dimensional tolerances can be along the longitudinal axis or perpendicular to the longitudinal axis of the cartridge or the cartridge can tilt versus the longitudinal axis of the surrounding compartment or rigid parts. The elastic attachment means connecting the cartridge to the compartment has to form a sterile barrier and additionally must sustain relative movements between the cartridge and the compartment due to the play described above. Thus the elastic properties of the attachment means together with the adhesive layers are designed such that the sterile barrier stays intact during those movements. Additionally, the elastic attachment means can restrict the movements of the cartridge in the surrounding compartment while keeping the sterile barrier.

In another example, the elastic attachment means is a multilayered system and comprises at least one adhesive layer and at least one elastic core layer, preferably being an elastic foam layer. The adhesive layer adheres to one surface and the elastic material is resilient to fill the gap between the two surfaces to compensate for dimensional tolerances. A certain press fit is required to ensure that the two opposing surfaces on the non-adhering side are pressed together to compensate for dimensional tolerances to ensure that a leak proof sterile barrier is maintained and that any shocks are properly absorbed and dampened. Differences in surface roughness and surface texture are compensated by the elastic core, to form a leak proof connection. Additionally, the use of at least one multi-layered system with at least one adhesive surface enables an accurate positioning of the adhesive system. The at least one adhesive layer is preferably covered by a liner sheet which, once removed, exposes the adhesive surface that can be fixed exactly at the desired located. This also enables thus a precise dosing of the adhesive, since the amount of adhesive is predefined in the multi-layered system.

In yet another example, the elastic attachment means is a multilayered system comprising at least three layers and preferably has one elastic core layer having a thickness substantially (e.g. at least twice, preferably at least ten times) above the thickness of the two adhesive layers. The two adhesive layers can comprise the same adhesive material or have different adhesive materials to adjust to the specific properties of the matching surfaces that needs to be adhered to. The total layer thickness of the multilayered system is below 4 mm and preferably between 0.1mm and 3 mm, more preferably between 0.5 and 2 mm. The thickness is defined here in the non-compressed state of the core layer.

In yet another example, the elastic attachment means comprises a multilayered system with at least four layers and preferably comprises at least one elastic core layer and at least two adhesive layers. One of the layers of the four layers, preferably an internal layer (defined as not being designed for attachment to the cartridge or the cartridge holder) can be used for internal bonding of the layers in the multilayered system. Alternatively, the internal layer ensures a certain color or has the purpose of providing an indication layer, to indicate whether a sterilization step has been executed. As another alternative, one of the internal layers is a reinforcement layer, for example comprising glass fibres. One of the four layers can also comprise an antibacterial agent such as silver ions or silver particles. One of the layers may also comprise an absorbing material such as polyacrylic acid which swells once it absorbs a liquid, thus providing a further sealing and prevents leaking of fluid out of the device. The layers can comprise other agents such as a sterilization agent (formaldehyde or alcohol) to disinfect the surrounding area of the site where the cartridge is fixated to the cartridge holder. The core of the multilayered system is, as discussed above preferably a foamed material. The foamed material can also be soaked with other agents such as the antibacterial agents discussed above or as disclosed in US20120087966. The advantage of soaking the foam with a liquid or gel like material before the insertion step is that any intended compression of the foam during insertion expels the component soaked in the foam to the surrounding area. The total layer thickness of the multilayered system with at least 4 layers is below 5 mm and preferably between 0.1 mm and 3 mm, more preferably between 0.5 and 2 mm. The thickness is defined here in the non-compressed state of the core layers.

In an example, a method for fixating the cartridge to the cartridge holder is presented whereby the at least one multilayered system is a double sided adhesive tape comprising two opposing adhesive layers and two opposing non-adhesive surfaces preferably arranged perpendicular to the adhesive layers and having the at least one elastic core layer arranged between the two opposing adhesive layers. An examples of such a double sided adhesive tape is a Scotch VHB™ tape or Scotch 1772 polyolefin foam. The elastic core with the two adhesive layers ensures that the fixation system has elastic compressible or tensile properties, thus when the cartridge moves with respect to the cartridge holder then those relative movements are compensated for, either by compressing, tensioning or shearing the elastic core layer. The elastic core layer can be a foamed material, for example a polyolefin (PE, PP), polyurethane or acrylic foam. Alternatively, the core material is an elastic non-foamed material. The advantage of a resilient elastic material, both foamed and non-foamed is that irregularities in the surfaces are compensated for which is beneficial for keeping a sterile barrier. The adhesive layers can be made for example from an epoxy based, isocyanate, acrylic based resin or synthetic rubber based resin. The adhesive layer adheres tightly to the surface to prevent the passage of any germs or viruses, additionally the adhesive material itself has sufficient barrier properties to form a sterile barrier perpendicular to the plane of adherence. Instead of a core layer made from a foamed material also rubber like cores (non-foamed) can be used as long as they maintain their elastic properties during use. Examples are a natural rubber core, butadiene rubber or a polydimethylsiloxane (PDMS) core.

The multilayered system provides for a sterile barrier between the two lateral sides of the multilayered system that do not adhere to a surface. Those two lateral sides are preferably perpendicular to the adhering surfaces but can also be at another angle to those surfaces. The two adhering surfaces are preferably parallel to another. To ensure a sterile barrier, preferably the core layer is adapted such that it has barrier properties between the two lateral sides. This can be defined such that if initially both lateral wall surfaces are sterile, that if one of the two is exposed to a non-sterile environment, there is no passage of germs, viruses or parts of viruses through the core layer and thus the one lateral side that is not exposed to the environment will remain sterile. The foam material can be a closed foam having no connectivity between the holes in the foam or is an open foam with connections between the cavities. The terms holes, pores and cavities define the same entity within the foam. The size of the cavities (pores) and connectors between the cavities prevents passage of germs. The size of the connectors is preferably below 20 micrometers, more preferably below 10 micrometers and even more preferably below 2 micrometers.

A method for fixating the cartridge to the cartridge holder whereby the at least one piece of double-sided adhesive tape is positioned between the crimp of the cartridge and a wall surface of the second cavity. The at least one piece can be positioned between the lateral wall surfaces of the crimp and the wall surface of the second cavity of the cartridge holder. Alternatively, two pieces of double sided adhesive tape are positioned between the cartridge and the holder to ensure proper fixation. One piece of tape can be present at the crimp and the other piece for example at the glass surface of the cartridge. Preferably, the at least one piece of double sided tape is flat and has a closed shape with a central opening. Preferably, the at least one piece of double-sided adhesive tape is ring-shaped and is attached to and surrounds the lateral wall surface of the crimp and/or covers the circular shaped distal end surface of the crimp.

A method for fixating the cartridge to the cartridge holder whereby the at least one piece of double-sided adhesive tape is attached to the cartridge prior to step c) or the at least one piece of double sided-adhesive tape is attached to the end-wall of the second cavity prior to step c). The double sided adhesive tape comprises a core layer and two adhesive layers. Preferably the adhesive layers are covered by a liner sheet that prevents undesired sticking to surfaces. Preferably, one of the liner sheets is removed to expose one of the two adhesive layers to the ambient, apply the double sided adhesive tape either to the cartridge or the end-wall of the cartridge holder, remove the second liner sheet to expose the second adhesive layer and insert the cartridge into the cartridge holder. This ensures that the adhesive tape can be accurately applied at the desired location.

A method for fixating the cartridge to the cartridge holder, whereby after insertion step c) the cartridge is fixated to the cartridge holder and a sterile barrier is established between the second cavity of the cartridge holder and a first cavity, separated from the second cavity by the end-wall of the cartridge holder. The cartridge is inserted and the double sided adhesive tape with the adhesive layers is compressed and sandwiched between the crimp of the cartridge and the end-wall separating the first and second cavity.

A method for fixating the cartridge to the cartridge holder, whereby the first and second cavities are connected to each other by the end-wall, the end-wall having a passage between the first and second cavity which is closed or covered after step c) by the distal end of the cartridge and the at least one piece of double-sided adhesive tape. The double sided adhesive tape is a sterile barrier. Before the insertion step c) there is a passage between the two cavities, after the insertion step c) the lateral surfaces of the double sided adhesive tape form, together with the crimp, a sterile barrier and are part of a sterile enclose enclosing the first cavity.

The first cavity encloses a fluid path unit of the injection device which comprises at least a needle adapted for penetrating the skin of a patient, a spike adapted for penetrating the septum of the cartridge, preferably through the passage of the end-wall, and a tubing providing a fluid connection between the spike and the needle. Alternatively also a steel canula or hollow needle can be used instead of a spike for penetrating the septum. Furthermore, the cartridge holder comprises the second cavity enclosing the cartridge after the insertion step c).

For the fixation method an empty cartridge can be used or a full cartridge containing a liquid medicament and which is closed by a plug can be used. After the insertion step c), the cartridge is preferentially fixated at the distal end. The proximal end can be supported or fixated by a proper attachment means, which itself can also have a resilient nature therewith forming a shock absorbing system for the cartridge together with the distal fixation, preferably using a double-sided adhesive tape.

### Legends to the Figures

Figure 1: Patch device, lateral view,
Figure 2: Patch device, top view,
Figure 3: Parts of subassembly for drive mechanism and control unit,
Figure 4: Parts of subassembly for cover,
Figure 5: Parts of subassembly for fluid path unit,
Figure 6: Parts of subassembly of bottom part housing,
Figure 7: Adhesive layer and capacitive sensor,
Figure 8: Top view of assembled device without top cover, before medication delivery,
Figure 9: Top view of assembled device without top cover, after medication delivery,
Figure 10: Coupling mechanism according to a first embodiment of the device whereby the second part directly engages the housing,
Figure 11: Coupling mechanism according to a first embodiment,
Figure 12: Coupling mechanism according to a second embodiment, the second part is indirectly coupled to the housing via a third part; the second part surrounds the first part,
Figure 13: Coupling mechanism according to a third embodiment, the second part is indirectly coupled to the housing via a third part, the first part at least partially surrounds the second part,
Figure 14: Fluid path unit, top view,
Figure 15: Fluid path unit, lateral view showing the first and second guiding means,
Figure 16: Fluid path unit, top view showing the spike inserter carrier in the non-inserted position,
Figure 17: Fluid path unit, lateral view showing the tubing, canula holder and the third and fourth guiding means on the housing for guiding the needle control element,
Figure 18: Detail of the needle insertion mechanism, needle retracted position,
Figure 19: Detail of the needle insertion mechanism, needle control element prevents movement of the canula holder and the spike inserter carrier,
Figure 20: Detail of the needle insertion mechanism, needle control element prevents movement of the canula holder and the spike inserter carrier, guide contour and cams of the third part are shown,
Figure 21: Detail of the needle insertion mechanism, needle control element rotated over the first angle to release the canula holder and spike inserter carrier,
Figure 22: Detail of the needle insertion mechanism, showing the pivoting of the first key in the third guiding means and the axial movement of the second key in the fourth guiding means,
Figure 23: Detail of needle insertion mechanism showing the position of the needle canula holder after being driven by the first guiding means to the needle inserted position,
Figure 24: Detail of needle insertion mechanism showing the canula holder in the needle inserted position and the locking mechanism,
Figure 25: Detail of the needle insertion mechanism showing the movement of the canula holder through the guiding means of the housing,
Figure 26: Detail of needle insertion mechanism, needle retraction arm of the needle control element guides the canula holder back to the needle retracted position,
Figure 27: Detail of needle insertion mechanism showing the guidance of the canula holder through the second guiding means of the spike inserter carrier,
Figure 28: Detail of needle insertion mechanism, cams of third part interacting with the guiding contour of the needle control element after rotation over the second angle for needle retraction,
Figure 29 a: Detail of the interaction between the two cams of the third part interacting with the guide contour of the needle control element as the third part is rotated to rotate the needle control element over the first angle for needle insertion and over the second angle for needle retraction: Start position,
Figure 29b: Needle control element rotated to release the canula holder,
Figure 29c: Canula holder moved to the needle inserted position,
Figure 29d: Needle control element rotated over further angle, needle retraction arm abuts the canula holder,
Figure 29e: Needle control element rotates and translates to move the canula holder towards the needle retracted position,
Figure 29f: Canula holder moves towards needle retracted position,
Figure 29g: Canula holder back in the needle retracted position,
Figure 30: Detail of the needle insertion mechanism according to a second embodiment, needle retracted position.
Stop means in abutment with the first arrester,
Figure 31: Detail of the needle insertion mechanism according to the second embodiment, needle retracted position showing the gearing on the steering drum for driving the spike carrier,
Figure 32: Detail of the needle insertion mechanism according to the second embodiment, needle retracted position showing the rotation of the stop means over the first stop means angle to release the first arrester,
Figure 33: Detail of the needle insertion mechanism according to the second embodiment, needle inserted position after the steering drum rotated over a first angle, spike carrier in inserted position,
Figure 34: Detail of the needle insertion mechanism according to the second embodiment, needle inserted position,
Figure 35: Detail of the needle insertion mechanism according to the second embodiment, needle inserted position showing the gearing for driving the spike carrier into the inserted position,
Figure 36: Detail of the needle insertion mechanism according to the second embodiment, stop means rotated over a further angle to release the engagement with the second arrester,
Figure 37: Detail of the needle insertion mechanism according to the second embodiment, steering drum has rotated over a further angle to retract the canula holder,
Figure 38: Detail of the needle insertion mechanism according to the second embodiment, showing the stops between the gearing and the spike carrier,
Figure 39: Detail of the needle insertion mechanism according to the third embodiment, needle retracted position,
Figure 40: Detail of the needle insertion mechanism according to the third embodiment, needle retracted position,
Figure 40b: Detail of the needle insertion mechanism according to the third embodiment, needle retracted position,
Figure 40c: Detail of the needle insertion mechanism according to the third embodiment, needle inserted position,
Figure 40d: Detail of the needle insertion mechanism according to the third embodiment, needle inserted position,
Figure 41: Drive mechanism of the injection device with a segmented piston rod according to a first embodiment,
before expelling medication,

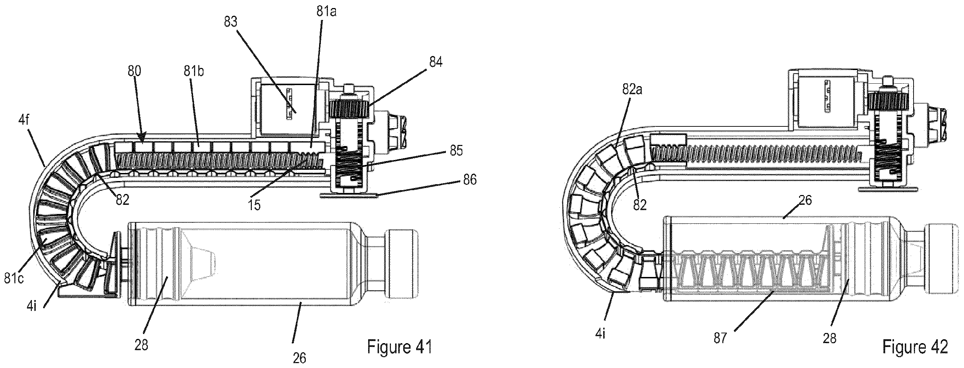

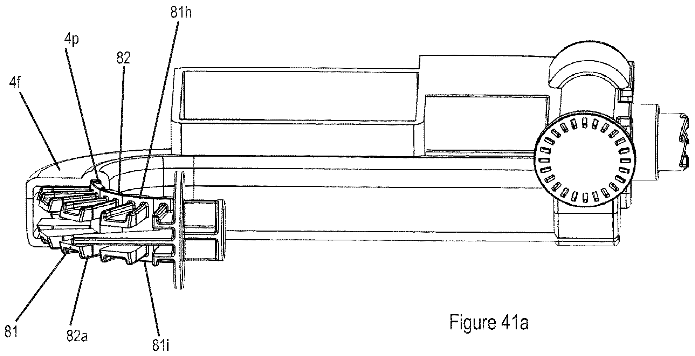

Figure 41 a: Drive mechanism of the injection device with a segmented piston rod according to a first embodiment,
before expelling medication, details of the guidance for the piston rod,
Figure 42: Drive mechanism of the injection device with a segmented piston rod according to the first embodiment,
piston rod has advanced, reservoir is empty,

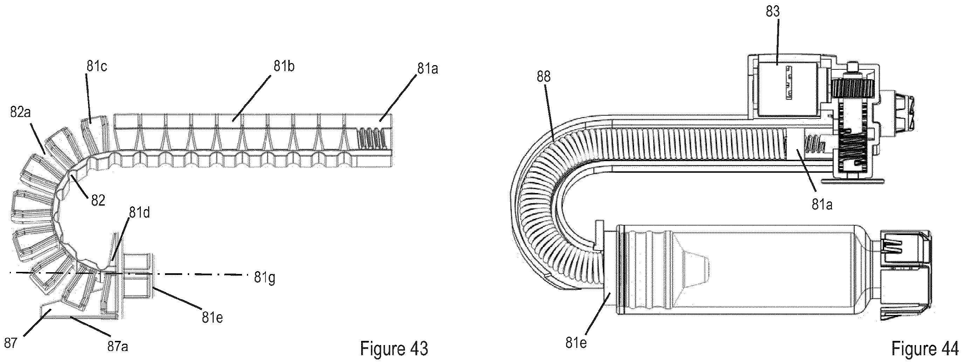

Figure 43: Segmented piston rod according to the first embodiment,
Figure 44: Drive mechanism of the injection device according to a second embodiment with a spring-type piston rod,

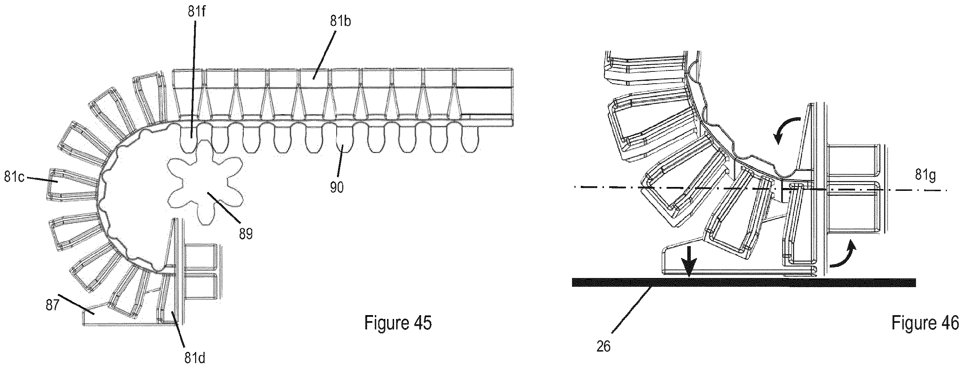

Figure 45: Drive mechanism of the injection device according to a third embodiment, drive mechanism operates a gear wheel for advancing the piston rod,
Figure 46: Schematic drawing showing the force vectors that intend to tilt the last segment and how this is counteracted by the guiding element,

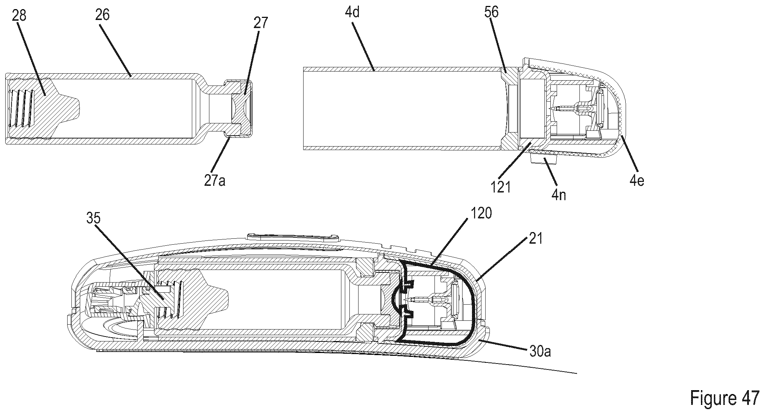

Figure 47: Assembly of the cartridge in the cartridge holder connector at the cartridge holder and sterile barrier at the front of the cartridge,

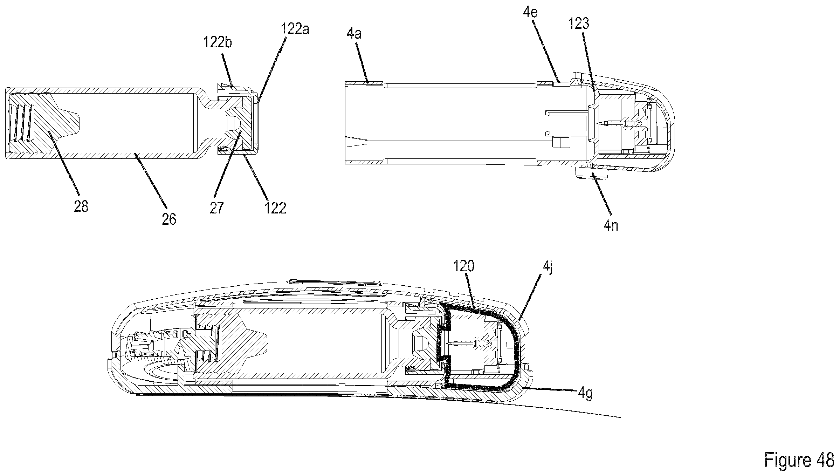

Figure 48: Assembly of the cartridge in the cartridge holder, connector and crimp integrated in one part with the sterile barrier at the front of the cartridge,

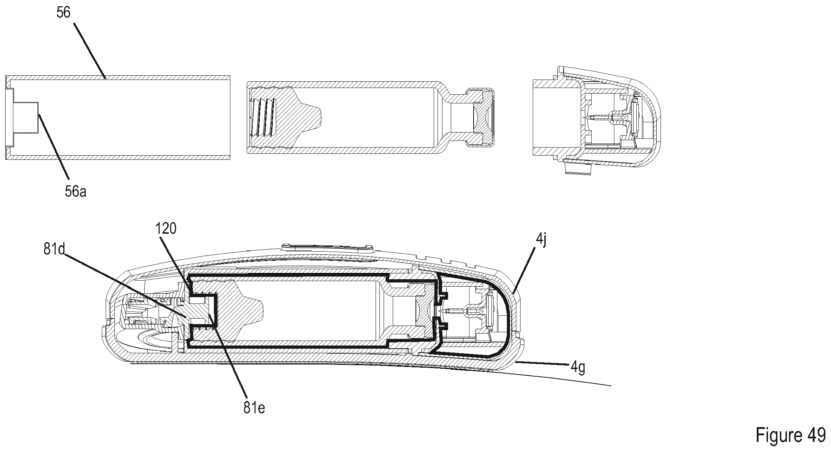

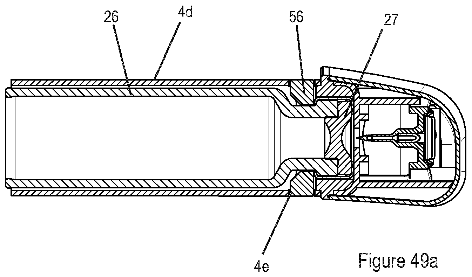

Figure 49: Assembly of the cartridge to the needle insertion sub-unit. The cartridge fixator is tubular shaped and has a sterile barrier foil at one end,
Figure 49a: Assembly of an empty cartridge to the needle insertion sub-unit,

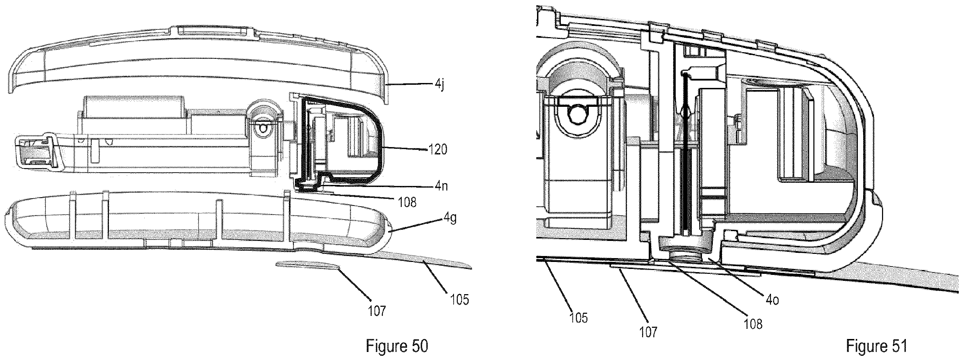

Figure 50: Assembly of the needle insertion unit with the bottom and top cover part: Sterile closure of the needle passage using a separate sticker to release the sterile barrier,
Figure 51: Assembly of the needle insertion unit with the bottom and top cover part: Sterile closure of the needle passage using a separate sticker to release the sterile barrier,

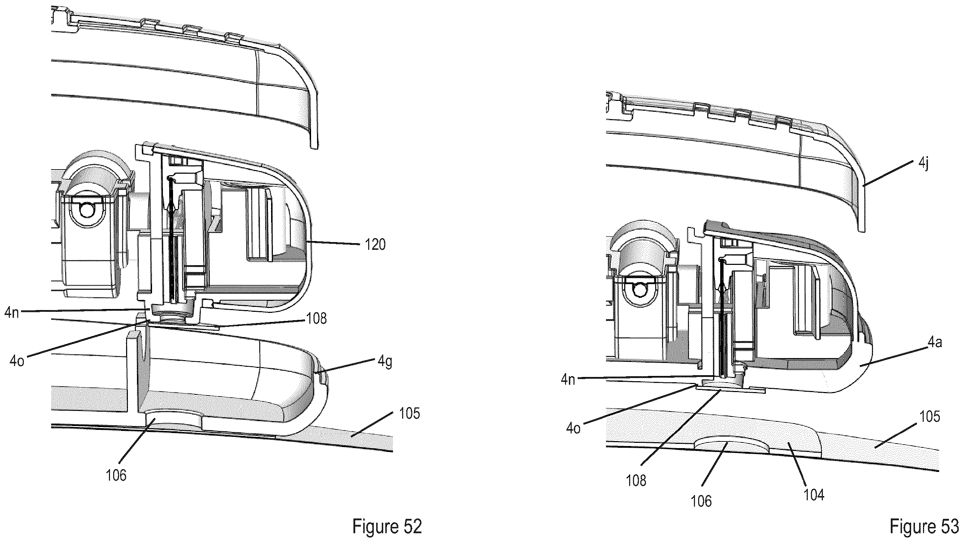

Figure 52: Assembly of the needle insertion unit with the bottom and top cover part: Sterile closure of the needle passage using a sterile barrier that is released together with the peel foil of the adhesive layer,
Figure 53: Assembly of the needle insertion unit with the top cover part: The bottom surface of the device is integrated with the needle insertion sub-unit; sterile barrier is released together with the peel foil of the adhesive layer,

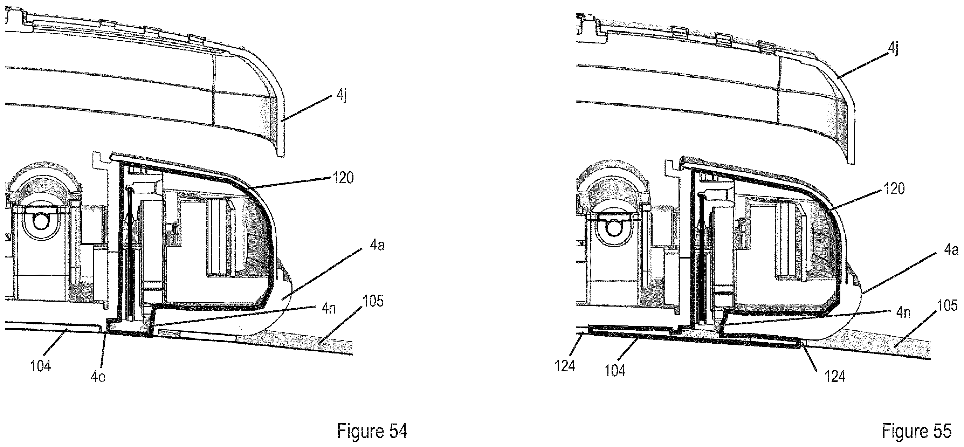

Figure 54: Assembly of the needle insertion unit with the top cover part: The bottom surface of the device is integrated with the needle insertion sub-unit; Peel foil of the adhesive layer forms the sterile barrier,
Figure 55: Assembly of the needle insertion unit with the top cover part: The bottom surface of the device is integrated with the needle insertion sub-unit; Peel foil of the adhesive layer forms the sterile barrier.

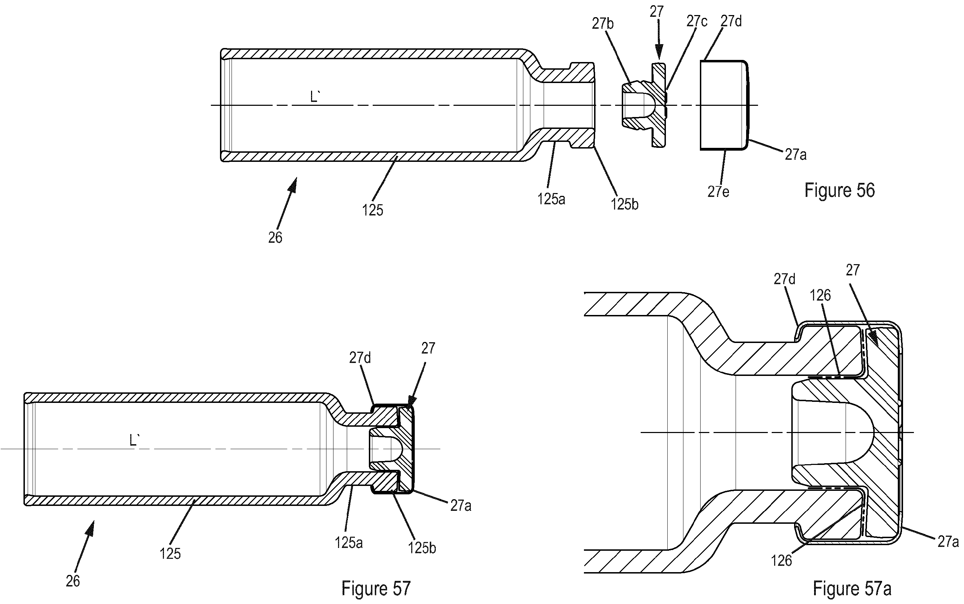

Figure 56: Assembly of the empty cartridge with the septum and crimp.
Figure 57: Assembled empty cartridge; sterilization of the empty cartridge creating a sterile interface between the septum and the glass barrel of the cartridge.
Figure 57a: Detail showing the sterile interface.

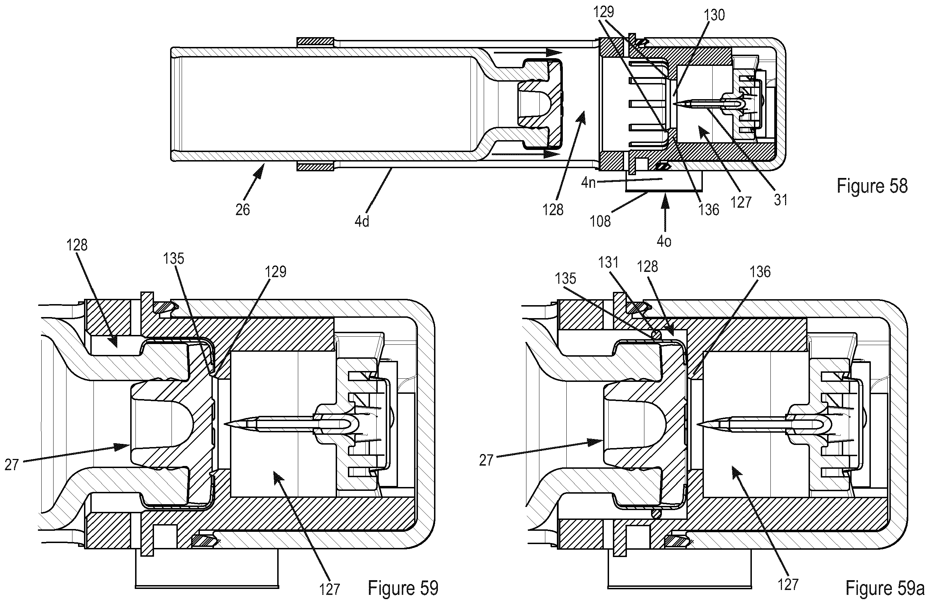

Figure 58: Insertion of the empty cartridge into the second cavity of the fluid path unit.

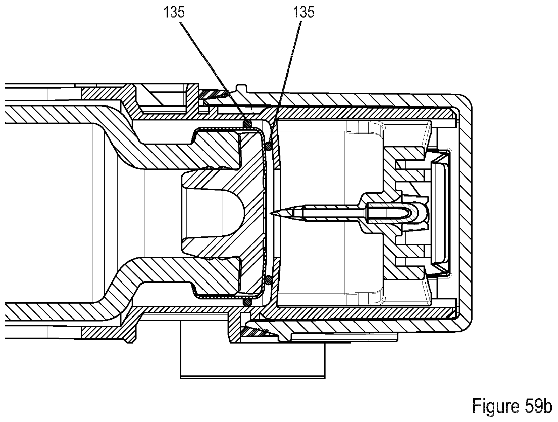

Figure 59: Formation of the sealing between the second cavity and the first cavity during cartridge insertion using a circumferential rim surrounding the passage.
Figure 59a: An alternative sealing solution for the sealing arrangement between the first and second cavity.
Figure 59b: Another alternative sealing solution for the sealing arrangement between the first and second cavity.

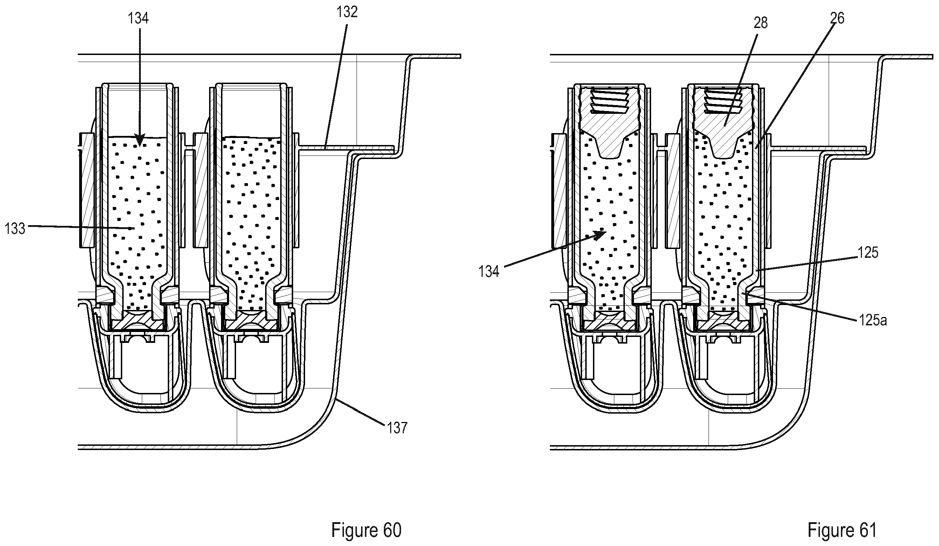

Figure 60: Filling of the empty cartridge with a liquid medication, the fluid path unit is positioned in a tray of a tub.
Figure 61: Closing the filled cartridge with a stopper or plug.

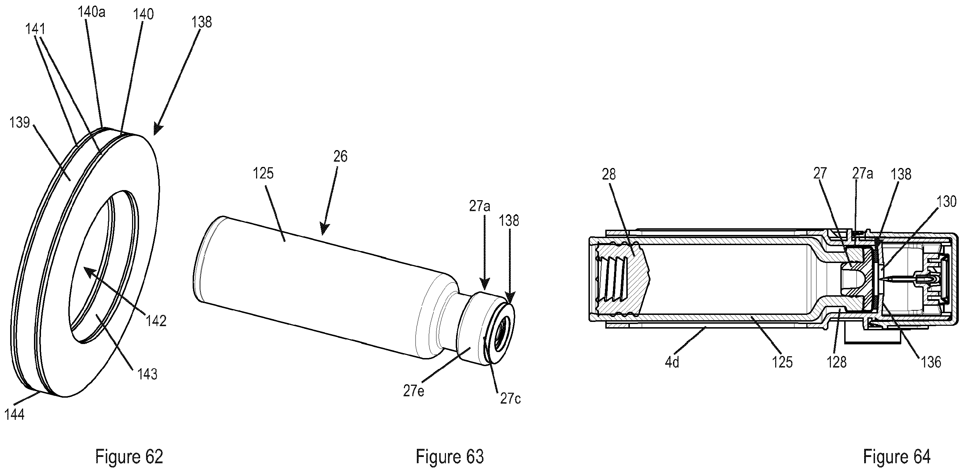

Figure 62: Ring-shaped multi-layered adhesive system for cartridge fixation.
Figure 63: Multi-layered adhesive system applied to the crimp of the cartridge.
Figure 64: Fixation of the cartridge using a ring-shaped multi-layered adhesive system.

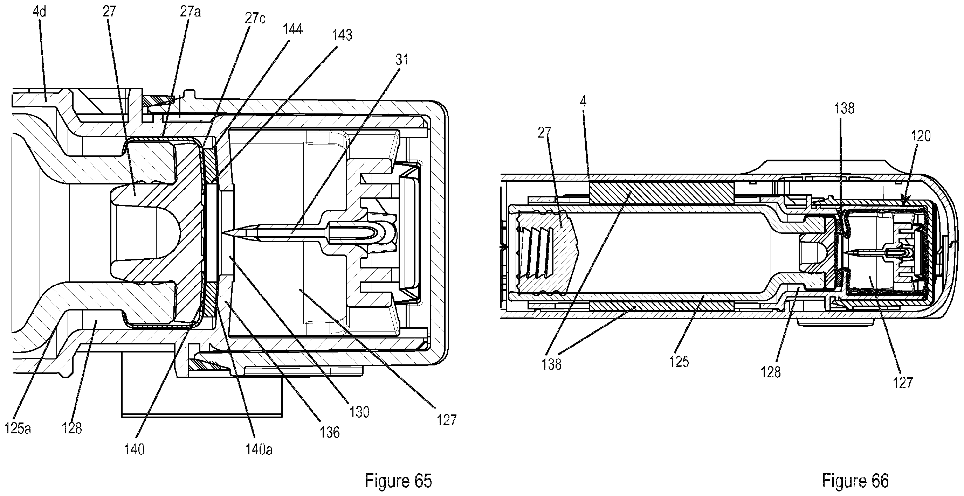

Figure 65: Detail of the cartridge fixation.
Figure 66: Fixation of the cartridge both on the crimp and on the glass surface of the cartridge

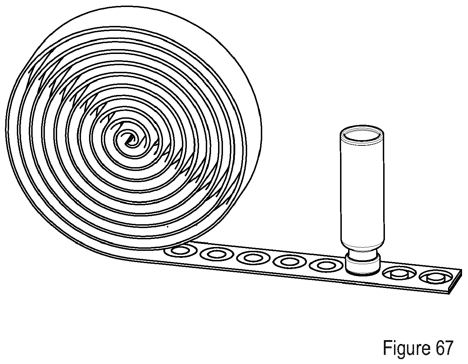

Figure 67: Fixation method using a role of double sided adhesive tape with pre-punctured rings

### Detailed description

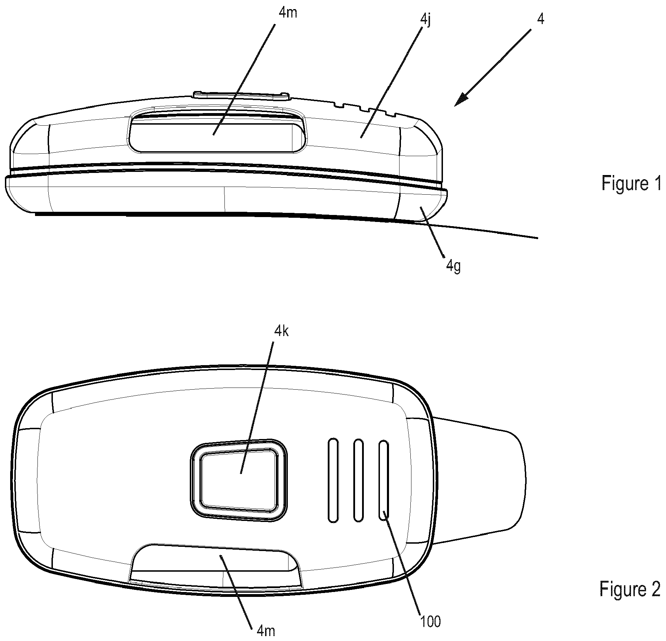

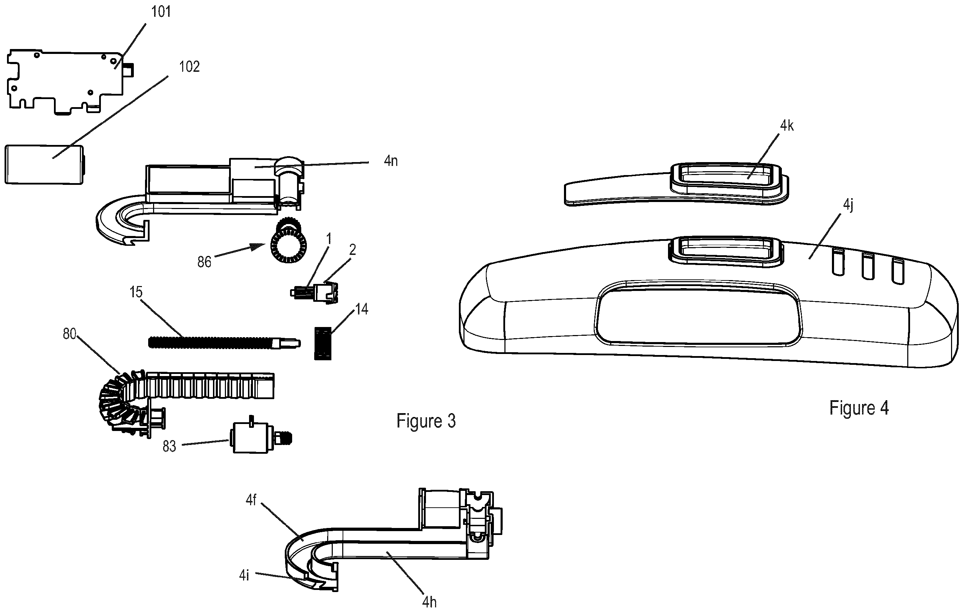

A lateral view and a top view of a patch device comprising the coupling mechanism, the segmented piston rod and fluid path sub-assembly is presented in Figures 1 and 2, respectively. The housing (4) comprises a housing cover (4j) with a window (4m) for viewing a cartridge and, preferably, a bottom housing part (4g). The housing furthermore preferably comprises a button (4k) for starting the injection or starting the device and/or an optical indicator (100), preferably a LED light. The subassemblies of the device are presented in Figures 3 to 6 showing the separate parts in an exploded view.

Figure 3 shows the drive and control unit comprising a drive carrier (4h) which holds and guides a segmented piston rod (80). The drive carrier (4h) is closed by a drive cover (4n) and the carrier and cover hold the electromotor (83), a threaded rod (15) and a gearing arrangement including a worm wheel for driving the threaded rod (15). The gearing arrangement comprises an encoder (86) which counts the number of revolutions of the motor and/or threaded rod (15). The gearing arrangement is also connected to a first part (1) which forms together with a second part (2) a coupling mechanism that will be described below. A battery (102) and a control unit (101) are part of the subassembly as well.

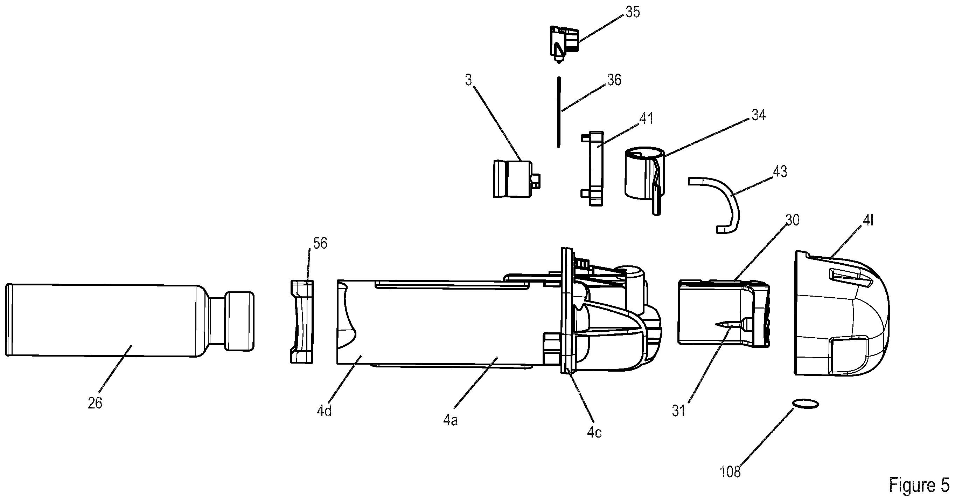

In Figure 4, the cover unit for the patch device is shown comprising a housing cover (4j) and, preferably, a button (4k), the button can also be located on one of the lateral planes of the housing (4). The fluid path unit in Figure 5 presents a housing (4a) with a cartridge holder (4d) and a vertical wall (4c) which separates the cartridge holder from the needle insertion mechanism, the wall (4c) and a cover (41) for the insertion mechanism are part of a sterile barrier. The needle insertion mechanism comprises a canula holder (35) for holding a canula (36), a spring means (34), a spike inserter carrier (30), a needle control element (41) and a third part. The functioning of the several parts will be described below.

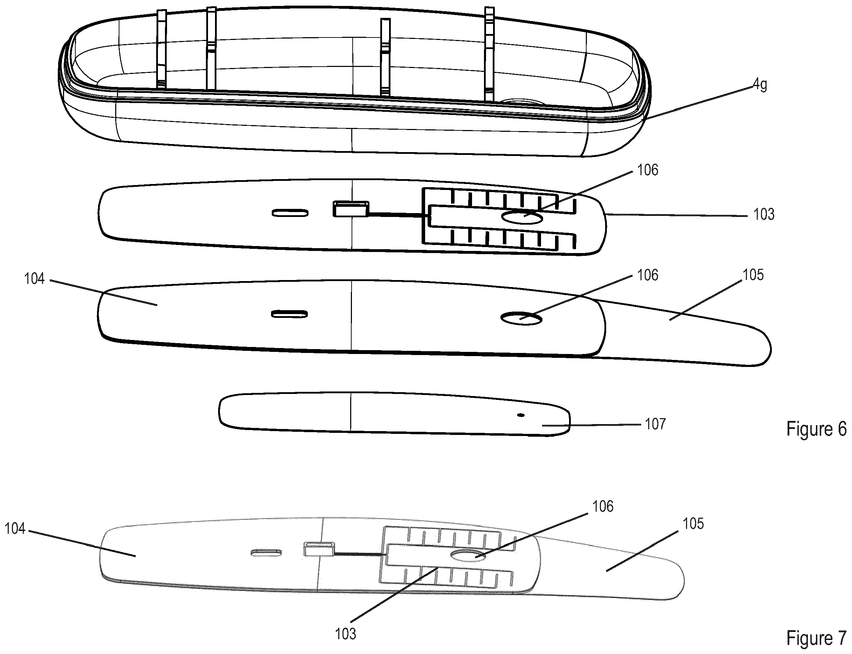

Figure 6 shows the subassembly of the bottom section of the device with a bottom housing part (4g), a capacitive sensor (103) an adhesive layer (104) which can be covered by an additional sticker for connecting the sterile barriers of the fluid path unit. In some embodiments, there is no separate bottom housing part, but is integrated with the housing (4a) of the fluid path sub-assembly. A detail of the capacitive sensor (103) is presented in Figure 7, with conductive elements that are embedded or printed onto, or attached to an adhesive layer (104). The sensor and/or adhesive layer have a passage (106) required for insertion of the canula. The peel-off foil (105) for the adhesive layer (104) prevents unintended adhesion or contamination of the adhesive layer. The peel-off foil (105) has a lid which extends beyond the contour of the adhesive layer which the user can grip. Removing the peel-off from the adhesive layer releases the adhesive layer but the removal can also be combined with other functionalities such as switching or activating the electrical circuitry or removing a sterile barrier of the fluid path sub-unit or opening a passage for the canula. For example, the peel-off comprises as separate lid that isolates the battery contact, removing the peel-off directly closes the electrical circuitry. In another example, the peel-off comprises a pin that is attached to the peel-off foil and the pin penetrates though a passage the bottom housing part. Removing the peel-off activates a micro-switch that is part of the electrical circuitry.

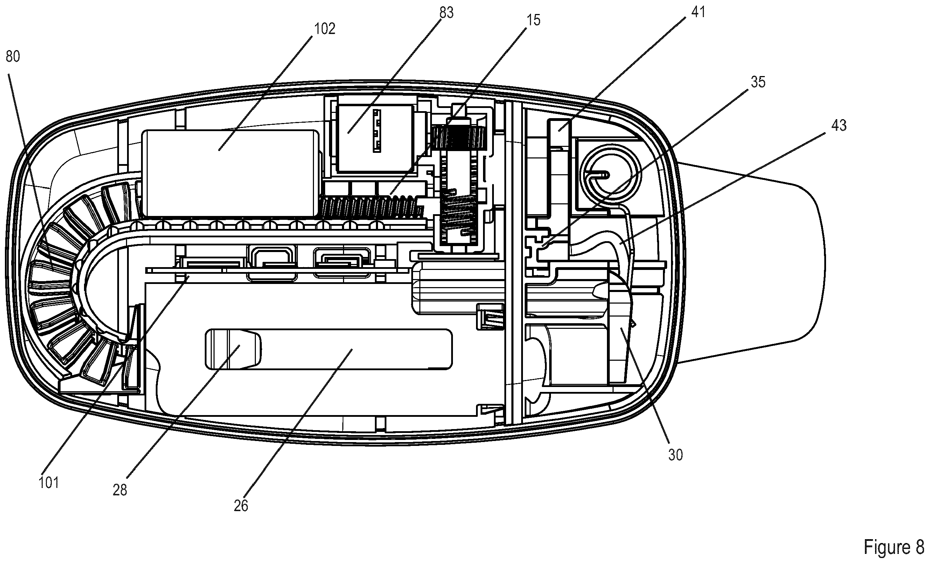

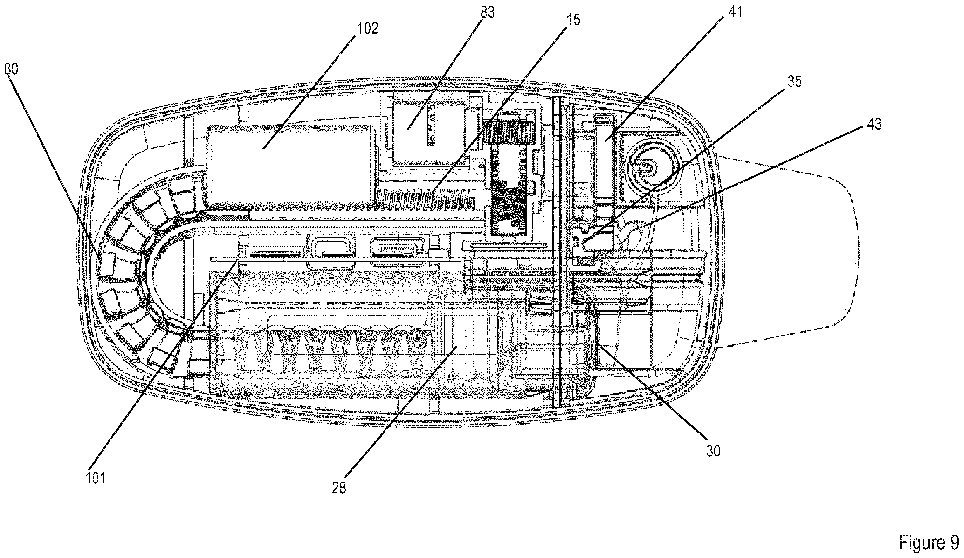

A top view of the assembled device without the housing cover (4j) is shown in Figure 8 for a device with a reservoir that is full and the piston rod being in the starting position prior to delivery. Once the medication has been expelled, the piston rod has been advanced by the drive mechanism as shown in Figure 9.

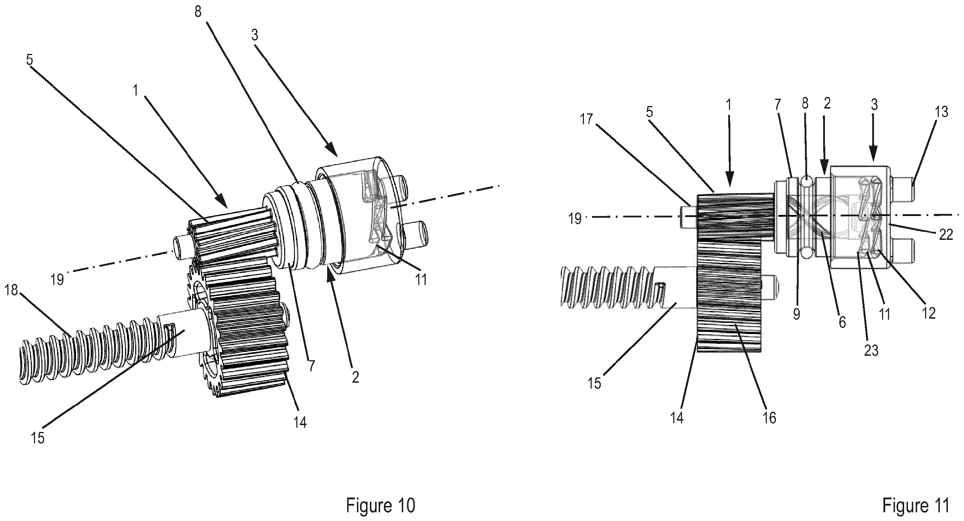

In Figure 10, a first embodiment of the coupling mechanism is presented with a first part or ratchet shaft (1) having a toothing (5) and at least one external thread or gearing (6). A circumferential flange (7) is located between the toothing (5) and threading (6). Adjacent to the toothing (5) the first part (1) has a bearing member (17) which ensures that the first part (1) can rotate, preferably with respect to a housing (4), around its longitudinal axis (19). The toothing (5) of the first part (1) matches a toothing (16) of a gear wheel (14). Preferably, the matching toothings are slightly oblique with respect to the longitudinal axis and form a helical gearing to ensure a smooth drive mechanism and/or ensure that an axial force along the rotation axis is exerted onto the first part to enable an axial shift of the first part, or to secure its position in a bearing or to use the axial shift for coupling, or decoupling to the second part. The gear wheel (14) is directly coupled, e.g. axially and rotationally fixed coupled to a threaded rod (15) having an external threading (18). The threaded rod drives the piston rod which ensures that medication is expelled from the reservoir. The threaded coupling between the gear wheel (14) and the first part (1) is permanent, thus a rotation of the gear wheel will always result in a rotation of the first part (1), but also in a rotation of the threaded rod (15).

A second part, or coupling member (2) concentrically surrounds the threading or gearing (6) of the first part (1). The second part (2) has at least one thread segment or protrusion (10) which engages the threading (6) of the first part (1). The thread segment or protrusion (10) is located on the inside of the second part and points towards the longitudinal axis (19). In the first embodiment, the second part (2) is surrounded by an O-Ring or friction element (8) which preferably provides an axial and/or radial friction force or torque between the second element (2) and the housing (4) or a part attached to the housing. The O-ring is axially secured with respect to the second part in a circumferential notch (9). The dimensional tolerances and/or materials are designed such that the friction between the gearing or threading (6) of the first part (1) which is in engagement with a pin or thread segment (10) of the second part, has a first frictional resistance below the frictional resistance, or second frictional resistance generated by the O-ring or friction element (8) and the housing (4).

A rotation of the gear wheel (14), preferably by a drive mechanism comprising the motor and a worm wheel, results in a rotation of the first part. Due to the threaded engagement between the first (1) and second (2) part in combination with the higher friction on the outside of the second part, which axially and rotationally temporarily holds or fixates the second part (2) with respect to the housing, this ensures that the second part (2) axially shifts away from the flange (7) of the first part (1) without rotation or at least rotates less, e.g. at a lower angular velocity than the first part. Reversing the rotation direction, for example by reversing the rotation direction of the electromotor, results in a rotation of the first part (1) in the opposite direction. The opposite rotation direction together with the fact that the frictional resistance on the outside of the second part is above the resistance on the inside, e.g., the second part is held on the outside yet still allowing for axial and/or rotational movements, results in a movement of the second part (2) towards the flange (7). The axial movement of the second part (2) with respect to the first part (1) is restricted by the threaded engagement or axial abutment between the two parts. For example, once the thread segment (10) of the second part reaches an end of the threading (6) on the first part, a radial abutment between the end surfaces of the at least one thread segment (10) and the threading (6) forces the second part (2) to co-rotate with the first part (1) at the same angular velocity, thus surpassing the external friction on the outside of the second part (2). Alternatively, there is an axial abutment of the second part (2) with the flange (7) of the first part (1).

The second part is concentrically surrounded by a third part (3) and the first, second and third part preferably rotate around the same longitudinal axis (19). The second part (2) is in the present embodiment positioned between the first part (1) and the third part (3). The third part has an end surface (22) with at least one cam or protrusion (13) pointing outwards and a toothing (12), preferably an asymmetric toothing, pointing towards the second part (Figure 11).

The second part has an end surface (23) with an toothing (11), preferably an asymmetric toothing circumferentially arranged that matches or complements the toothing (12), preferably an asymmetric toothing present on the end surface (22) of the third part (3). Once the toothings of the second and third part (11,12) are in a form fit after closing the coupling by the axial shift of the second part (2) towards the third part (3), the first part, the second part and the third part co-rotate in one rotation direction at the same angular velocity. Reversing the rotation direction opens, or decouples the coupling (11,12) between the second part and the third part, e.g., the toothings (11,12) move out of engagement, the third part (3) will stop co-rotating with the first (1) and second (2) part. The first rotation direction for closing the coupling is preferably accompanied with a needle insertion and/or needle retraction, the second rotation direction which is opposite to the first rotation direction is linked to or causes delivery of medication.

The coupling of the second part (2) to the third part (3) in a rotationally locked configuration ensures that the cams (13) which are on the end surface (22) of the third part, also rotate a needle control element (41) over a defined angle. Preferably, the angle is defined by the number of revolutions of the electromotor, which via a worm wheel gearing results in rotation in a first rotation direction of the third part (3) and via the cams (13) to a rotation of the needle control element (41). The needle control element has a guiding element or a guiding contour which can interact with the at least one cam (13) of the third part such that a controlled rotation and/or translation occurs once the third part (3) has been rotated over a first angle. Further rotation /and or translation of the needle control element (41) over a further angle being greater than the first angle will activate a needle retraction mechanism.

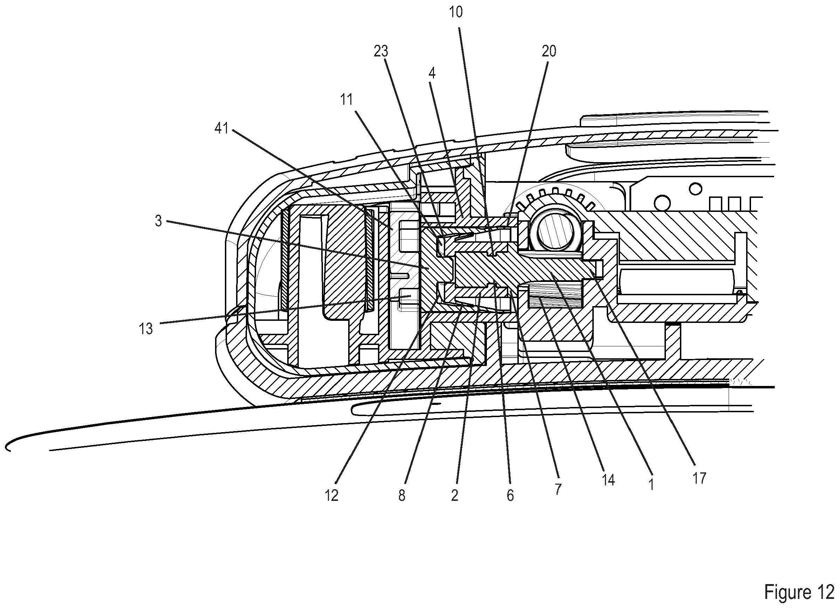

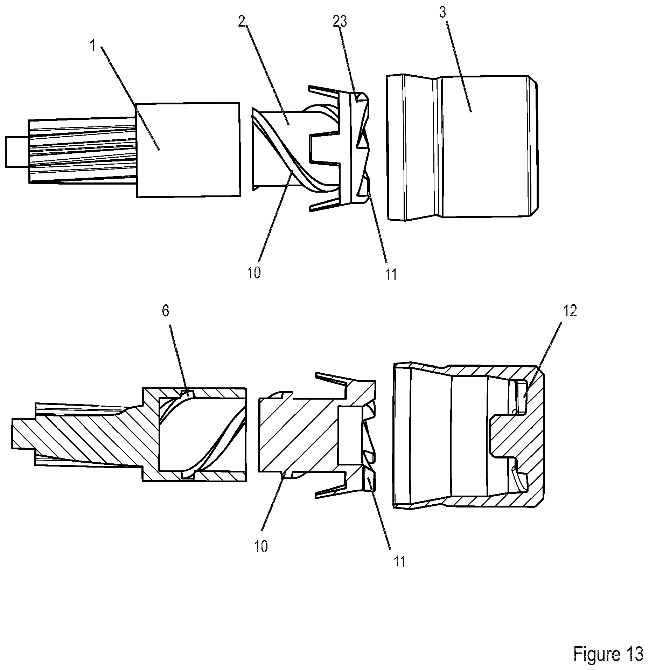

A second embodiment of the coupling mechanism is shown in Figure 12. The coupling mechanism functions comparable to the first embodiment, however the second part (2) is directly coupled to the third part (3) via at least one friction element (8), preferably shaped as at least one elastic wing or wing segment that is biased against the inner wall of the third part (3) which runs parallel to the longitudinal axis (19). The third element (20) is press fitted into the housing (4) resulting in a second friction fit engagement. The rotation of the first part and the accompanying axial shifts and coupling-decoupling sequences of the second and third part are described above. A third embodiment of the coupling mechanism is presented in Figure 13. The third part (3) concentrically surrounds the first (1) and second part (2). However, the first part surrounds at least partially the second part (2) and the threading or gearing which is present on the outside of the first part (1) in the first embodiment has shifted to the inside surface. In all embodiments the location of the gearing or threading and the engaging thread segment or pin can also be reversed, e.g. the threading or gearing can also be located on the second part and the engaging pin or thread segment is available on the first part.

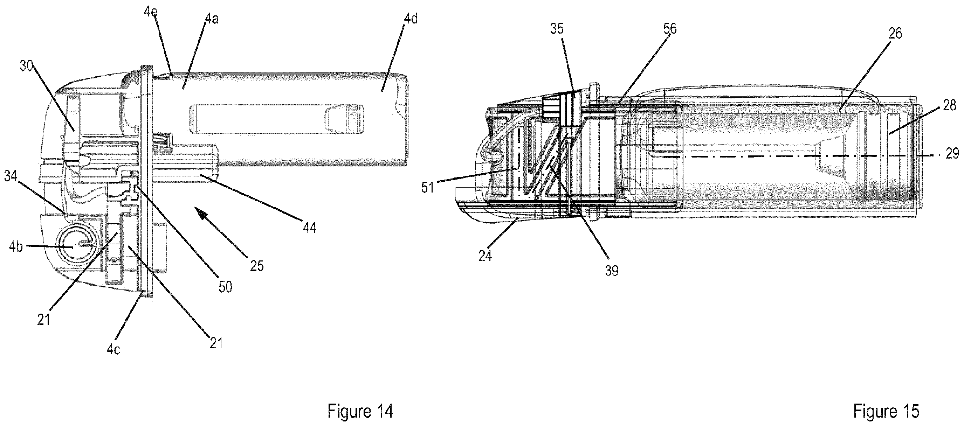

In Figure 14 a fluid path assembly (25) is presented showing the housing (4a) having the cartridge holder (4d), preferably shaped as a cylinder having an open end for receiving the cartridge or reservoir (26). The cartridge holder has a viewing window that can be aligned with the viewing window of the housing. The cartridge holder (4d) is separated from the needle insertion and retraction compartment by a vertical wall (4c) which is optionally surrounded by an elastomeric material to provide a fluid tight and/or sterile barrier between the parts on one side of the vertical wall (4c) and the inside of the insertion mechanism, once the insertion mechanism is encapsulated by a cap or needle housing cover (4l). The housing (4a) comprises a spring holder (4b) for holding a spring, preferably a leaf spring. The housing (4a) has a guide slot (44) for guiding a spike inserter carrier (30), the spike inserter carrier being biased by the end (34a) of the leaf spring (34).

Figure 15, presents a lateral view of the fluid path assembly showing the cartridge or reservoir (26) having a longitudinal axis (29) that is oriented parallel to the bottom surface (24) of the fluid path assembly (25) or the injection device. The reservoir (26) has a plunger (28) on one end and a septum (27) on the opposite end which is crimped onto the reservoir. The reservoir or cartridge is fixated around the tapered neck of the cartridge using a cartridge fixator (56) which is a flexible or semi-flexible element for holding the neck of the cartridge, for example directly onto the shoulder of the cartridge. Alternatively, the cartridge fixator engages the metal crimp of the cartridge. In another alternative, the cartridge is glued or welded to the cartridge holder. In yet another alternative, the edge of the opening of the cartridge which is opposite to the septum, is in a snap-fit connection to the housing. The cartridge fixator (56) is connected to the housing (4a) by a snap-fit connection using holes (4e) in the cartridge holder (4d) and at least one matching protrusion on the cartridge fixator (56). Preferably, the cartridge (26) is inserted in the cartridge holder opening with the septum (27) pointing towards the needle insertion unit, as presented in Figure 15. Alternatively, the insertion is reversed and the plunger (28) points towards the insertion unit. Furthermore a canula holder (35) is shown in a needle retracted position (37) whereby the canula holder is furthest away from the bottom surface (24). In side view, the first guiding means (39) and the second guiding means (51) are indicated, which both are shaped as linear guiding slots on a lateral wall surface of the spike inserter carrier (30).

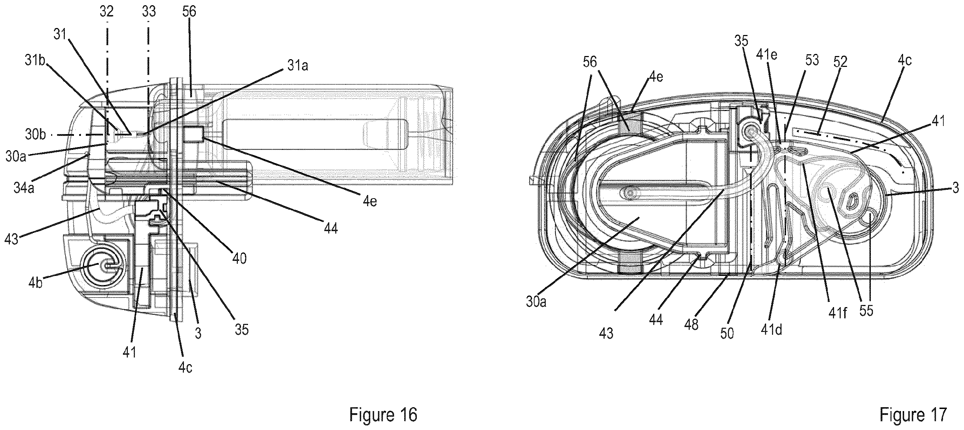

A top view (Figure 16) of the fluid path unit (25) presents the spike inserter carrier (30) as an "L" shaped part with one leg being guided in the guide slot (44) of the housing and the other leg carrying a spike (31). The spike being attached to a base surface (30a) of the spike inserter carrier (30) and in the current example, the spike (31) and spike inserter carrier (30) are injection molded as one unit. The spike (31) has a longitudinal axis (30b) which is aligned with the septum (27) or longitudinal axis (29) of the cartridge (26). The spike inserter carrier can shift from a first position (32) to a second position (33) and is guided through the guide slot (44) of the housing, the second position is where the tip (31a) of the spike penetrates the septum (not shown here). The movement from the first (32) to the second position (33) is biased by the spring (34) and a release of the spring energy moves the spike inserter parallel to the bottom surface (24). The tip of the spring (34a) abuts the base surface (30a) of the spike inserter carrier for transferring the spring energy. As a result, the spike penetrates the septum of the reservoir, preferably the longitudinal axes of the spike and the reservoir are aligned with each other for a central puncture of the septum. A tubing (43) connects the spike (31) to the canula holder (35) to create a fluid path between a canula (36) and the contents of the reservoir (26) once the spike (31) has been inserted.

Another lateral view (Figure 17) of the fluid path unit (25) shows an example of the cartridge fixation (56) being formed as an ellipsoidal shaped ring, preferably made from an elastic material, which is connected to the openings (56) of the cartridge holder housing (4d) via connectors that are attached to the ellipse part parallel to the long axis of the ellipse. The ellipse elements parallel to the long axis fit around the neck of the cartridge (26) and fit behind a metal crimp holding the septum. The cartridge fixation ensures that the cartridge (26) remains axially and radially fixated in the cartridge holder. Also the axial forces which are acting upon the cartridge due to the penetration of the septum by the spike are counteracted by the cartridge fixation ring (56). Other alternatives include a crimp designed to fit into the cartridge holder (4d) and which can have a locking element, preferably a flexular element or snappers that directly fit into matching counter-elements to fixate the cartridge in the cartridge holder. Such a crimp can be made from a metal having, for example, flexural or spike elements pointing outwards that plastically deform the inside wall of the cartridge holder and/or the element itself during axial and/or rotational movement during cartridge insertion. Alternatively, the crimp is made from a plastic material with snappers that fit into holes present in the wall of the cartridge holder (4d). Or the plastic or metal crimp has an outside threading matching a threading on the inside of the cartridge holder such that the cartridge can be screwed into the holder. The lateral view shows the tubing (43) connecting the end of the spike with the canula holder (35), the tubing is partially keyed to the base surface (30a) of the spike inserter carrier. The lateral view shows guiding means which are part of the vertical wall (4c) which provide a motion-link for several parts that are engaged with the side wall (4c). The guiding means are highlighted in Figure 17 with dot-dash lines and are shaped as at least one groove, edge or rim that is formed during the manufacturing process of the fluid path housing assembly (25), preferably during injection molding. A linear guide (50) is engaged with the canula holder to linearly guide the canula holder from the needle retracted to the needle inserted position. Preferably, the linear guide (50) is part of the housing. The linear guide may be oriented perpendicular to the bottom surface or is inclined to the bottom surface, thus enabling also a non-perpendicular insertion of the canula into the patient's skin. The wall section (4c) of the housing comprises further a third guiding means (52) and fourth guiding means (53) which are shaped as at least one groove, rim or edge to guide the needle control element (41). The needle control element has at least two keys (41d, 41e) which each engage the third and fourth guiding means of the housing (52, 53), respectively. The needle control element (41) is driven by the third part (3) having at least one cam, in this example two cams (55). Depending on the interaction of the cams (55) with a guiding contour or motion-link on the needle control element, the needle control element (41) rotates and/or translates due to the engagement between the keys of the needle control element and the third and fourth guiding means (52, 53) of the wall of the housing (4c).

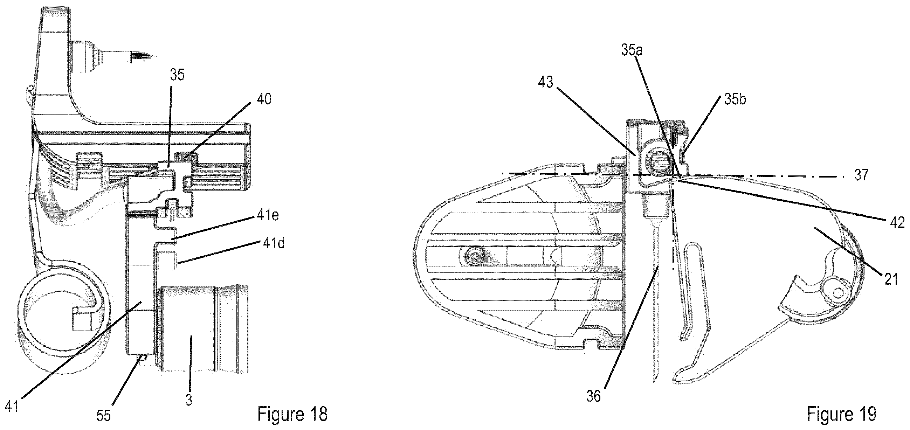

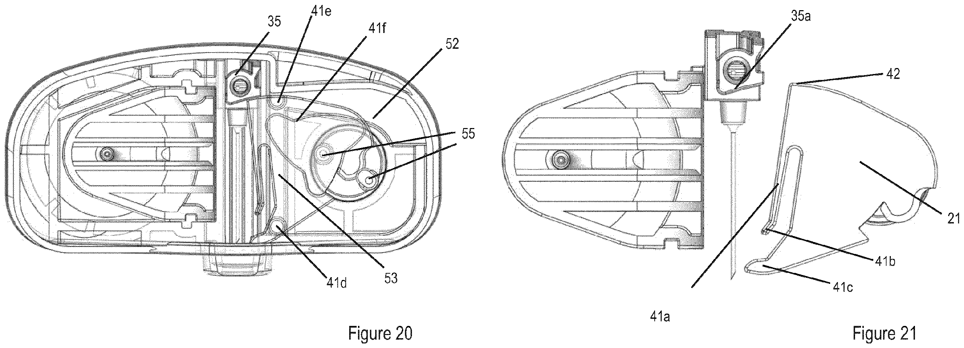

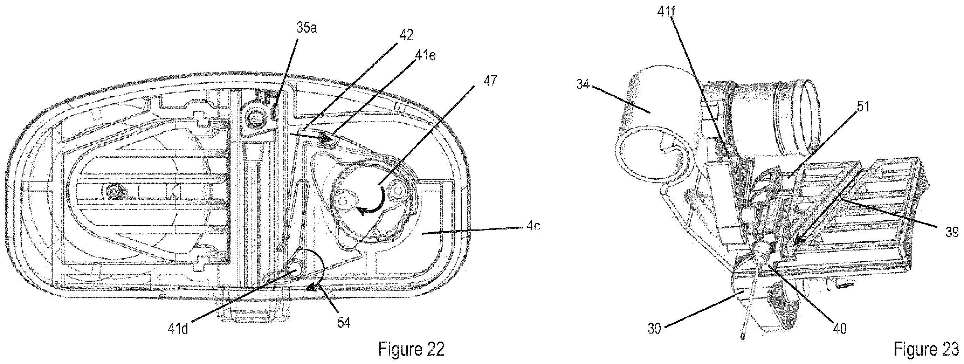

Figure 18 shows a detail of the needle insertion mechanism in the needle retracted state prior to insertion of the spike into the reservoir. The third part (3) is shown with the two cams (55) whereby one cam interacts with the guiding contour of the needle control element (41). The two keys (41d, 41e) of the needle control element are visualized in Figure 18 without the matching third and fourth guiding means of the wall surface (4c). In Figure 19, the arrester (42) abuts an abutment surface (35a) of the canula holder (35) such that the canula holder (35) remains in the needle retracted position (37). The same position of the elements is presented in Figure 20, but the position of the guiding contour (41f) and the position of the two cams (55) of the third part are visualized in a see-through view. The functioning of the needle insertion mechanism and the coupling of the drive mechanism to the needle control element is described as follows. The third part (3) is coupled to the first part (1) which is rotated in the first rotation direction and the coupling ensures that the two cams (55) of the third part rotate in the first rotation direction such that the needle control element (41) is rotated around the first key (41 d) of the needle control element and rotates in the fourth guiding means (53) without axial movement. The second key (41e) of the needle control element (41) axially translates in the lateral direction within the third guiding means (52) without rotation. As a result, the needle control element is rotated around the first key (41d) from the starting position over an angle, preferably the first angle which ensures that the abutment between the arrester (42) and the contact surface (35a) of the canula holder is released (Figure 21). Details of movement of the second key (41e) within the third guiding means (52) and the pivoting (54) of the needle control element (41) around the first key (41d) of the needle control element are shown in the see-through view of Figure 22. In the shown example, the needle control element (41) is rotated over a first angle between 5° and 40°, preferably between 8° and 30°, more preferably between 10° and 20°, most preferably to an angle of 15°.

The release of the abutment between the needle canula holder (35) and the needle control element (41) releases the spring forces of the spring (34) and advances the spike inserter carrier (30). The advancement of the carrier drives the canula holder (35) via the transformation means (40) and the first engagement means (39) from the needle retracted position to the needle inserted position (38), as schematically presented in Figures 23 to 25. In this example, the transformation means (40) is shaped as a protrusion with a cross section having at least one face parallel to the angulated guiding means, for example a triangular shape. Other shapes of the cross section such as circular or elliptical can also be envisaged.

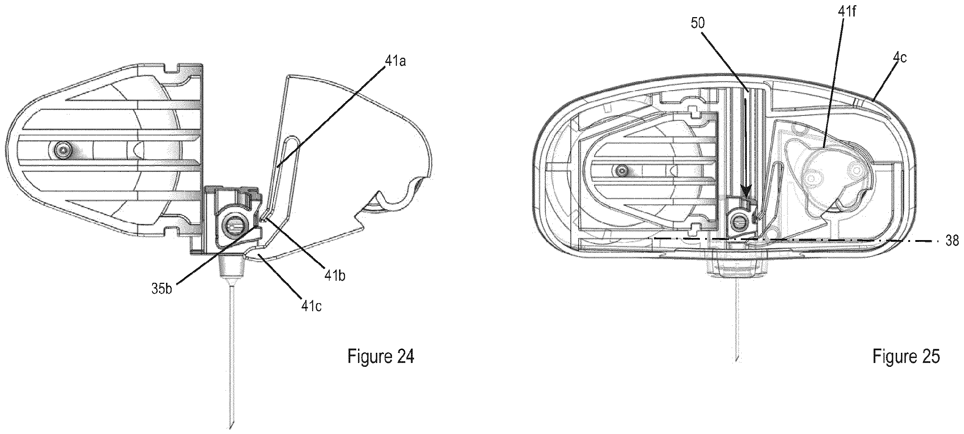

As the canula holder (35) moves into the needle inserted position (38), the locking arm (41 a) of the needle control element flexes into the catch (35b) of the canula holder (35) such that the end (41b) of the locking arm prevents a reversed motion of the canula holder (35) towards the needle retracted position. In Figure 25, a transparent view is shown including the wall (4c) of the housing and including the linear guide (50) which guides the canula holder vertically as the canula holder is driven to the needle inserted position.

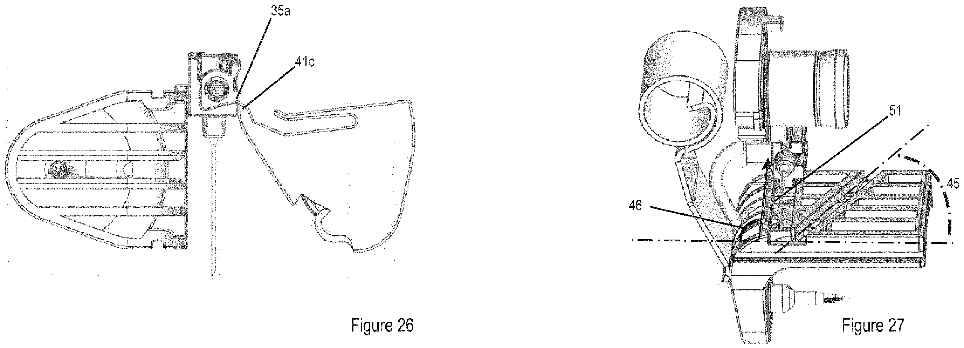

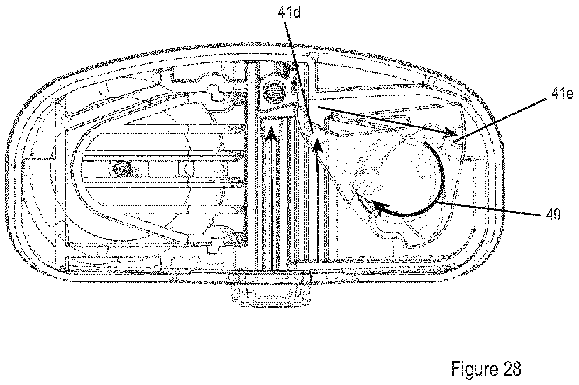

Once the canula holder (35) is in the inserted position, the rotation direction of the first part (1) is reversed to the second rotation direction, the coupling is opened and the needle control element (41) is not rotated. The rotation of the drive mechanism in the second rotation direction advances the piston rod for medication delivery. After medication delivery, the rotation direction of the drive mechanism and, therewith the first element (1), is reversed once again to the first rotation direction and the coupling between the second and third part (11,12) is closed. The third part (3) is rotated and the cams (55) engage with the guiding contour (41f) or motion-link of the needle control element (41), and the needle control element (41) is rotated further, starting the release of the insertion mechanism. The rotation of the third part (3) drives the needle control element such that the first key (41d) axially shifts upwards in the fourth guiding means (53) whereas the second key (41e) axially shifts sideways in the third guiding means (52). The resulting movement is a combined rotation and translation of the needle control element (41) which ensures that the needle retraction arm (41c) of the needle control element (41) abuts the abutment surface (35a) of the canula holder (35) and pushes the canula holder (35) back to the needle retracted position (37) (Figure 26). The canula holder is guided in the linear guide (50) of the housing but is also guided in the spike inserter carrier (30) through the second guiding means (51) as is shown in Figure 27 and in a semitransparent view in Figure 28, where the movement of the keys (41d, 41e) in the guiding means (52,53) of the housing is illustrated.

The needle control element (41) is fixated after this second rotation by latching or locking means present in the third or fourth guiding means of the housing which interact and lock one or both of the keys (41d, 41e) of the needle control element. For example, a flexural arm present in the third guiding means (52) flexes as the second key (41e) moves axially in the third guiding means and irreversibly locks the second key. In another alternative, a catch is present in the fourth guiding means and the first key (41d) moves into the catch as the first key moves upwards in the fourth guiding means (53).

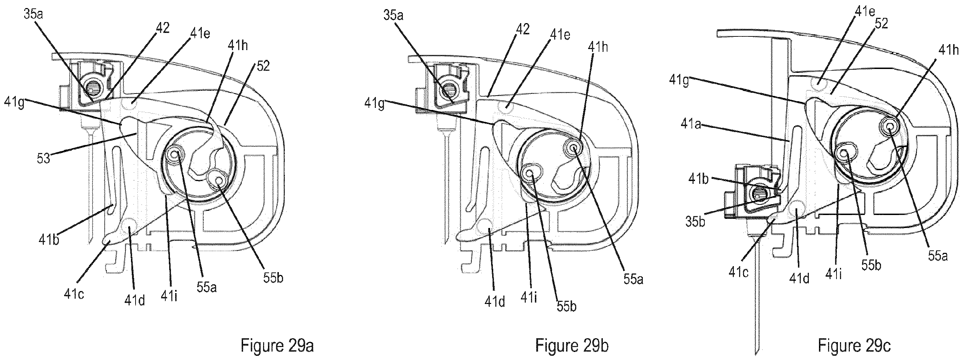

The interaction between the two cams of the third part and the guiding contour of the needle control element which form together with the keys of the needle control element a motion-link system is described in more detail in Figures 29a to 29g.

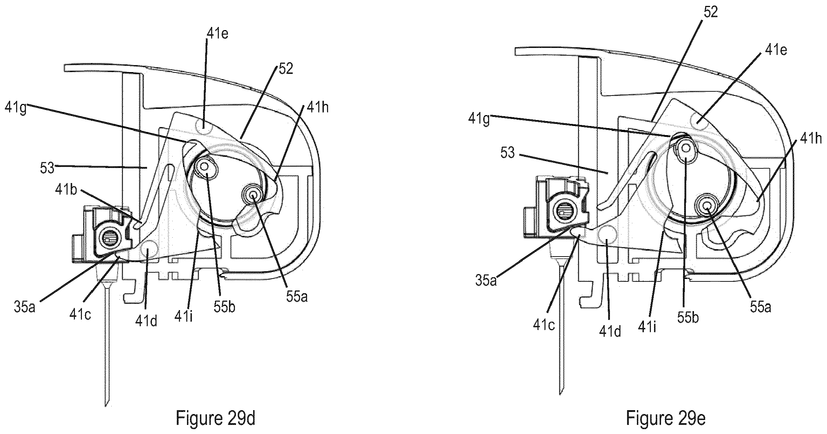

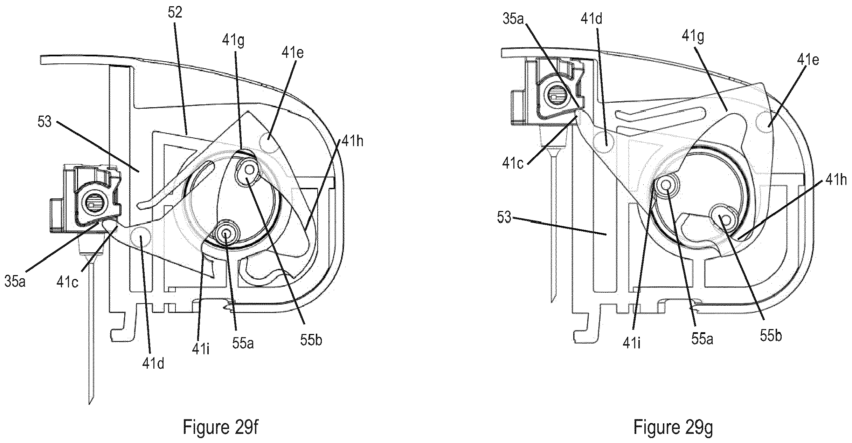

In this example the two cams (55) of the third part are not identical, cam (55a) is a circular protrusion whereas the cam (55b) is rounded but more elongated. The two cams protrude from the third part and each can a have a different length for interacting with different parts of the guiding contour (41f) of the needle control element (41). The guiding contour is shaped as a recess in the needle control element and comprises three pockets (41 g, 41 h and 41 i) that can interact with the two cams of the third part as the third part is rotated. The three pockets are in this example positioned as a triangle with respect to each other but other configurations can be envisaged. The needle control element (41) is guided in the housing by the two keys (41 d, 41 e) of the needle control element that interact with the two guiding means (52,53) of the housing, respectively. In Figure 29a, which represents the starting position before the needle is inserted, the two cams (55a and 55b) do not interact with the guiding contour of the needle control element and the arrester (42) holds the canula holder in the needle retracted position. For needle insertion, the third part is rotated over an angle such that first cam (55a) engages the second pocket (41 h) of the needle control element which starts to rotate around the first key (41d), and the second key (41e) axially shifts in the third guiding means (52), see Figure 29b. Once the needle control element has been rotated over an angle, the abutment between the arrester (42) and the canula holder is released such that the needle can move from the needle retracted to the needle inserted position (Figure 29c). The rotation direction of the drive mechanism is now reversed to open the coupling such that the third part does not rotated while the threaded rod is rotated to expel medication from the device. After emptying the reservoir, the rotation direction is reversed and the third part is coupled such that it can rotate over a further angle. The further rotation of the third part ensures that the first cam (55a) of the third part (3) moves in the second pocket (41 h) such that the needle control element is rotated over a further angle and pivots around the first key (41d), Figure 29d. The needle retraction arm (41 c) abuts the abutment surface (35a) of the canula holder (35) and axially starts moving the canula holder towards the needle retracted position. Further rotation of the third part (3) releases the first cam (55a) of the third part from the second pocket (41 h) of the needle control element whereas the second cam (55b) contacts the first pocket (41 g), Figure 29e. The needle control element rotates and translates as both the first key and second key (41d, 41e) of the needle control element axially shift in the corresponding fourth and third guiding means (53, 52). Further rotation of the third part (Figure 29f) ensures that the second cam (55b) remains in contact with the first pocket (41 g) whereas the first cam (55a) abuts the third pocket (41 i). Further rotation of the third part ensures that the second cam (55b) abuts the second pocket whereas the first cam (55a) contacts the third pocket (41 i) such that the needle control element is rotated and translated so far as to bring the canula holder back into the needle retracted position (Figure 29g)

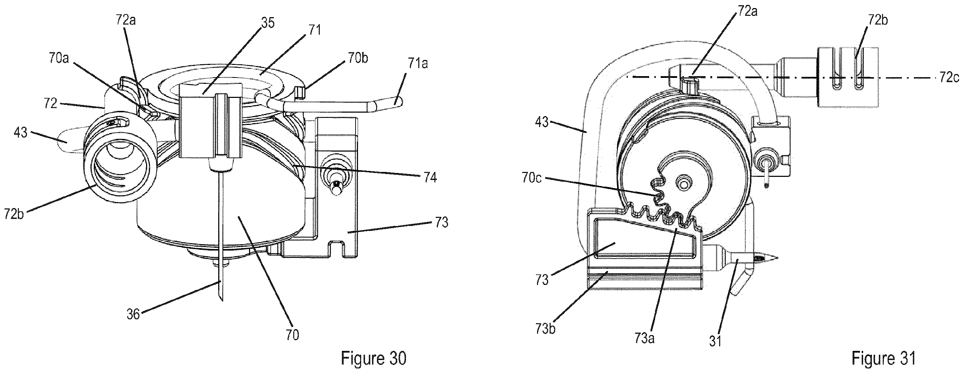

In Figure 30, a needle insertion mechanism according to a second embodiment is shown with the canula holder (35) in the needle retracted position. A steering drum (70) is positioned with its longitudinal axis preferably perpendicular to the bottom surface of the device and can rotate with respect to the housing. The steering drum (70) has two arresters, a first steering drum arrester (70a) and a second steering drum arrester (70b) that are attached or attachable to the outside surface of the steering drum (70). A biasing means (71), preferably a spring means or a torsional spring is functionally positioned between the housing and the steering drum (70). Preferably, the spring is pre-stressed and the end of the spring (71 a) preferably abuts the housing or housing part whereas the other end of the spring (not shown) is attached to the steering drum (70). The steering drum has a guiding means (74), preferably shaped as a recession on the outside surface, preferably with a sinusoidal shape which catches a transformation means or pin present on the canula holder (35). The canula holder (35) is linearly guided with respect to the housing and can be guided perpendicular to the bottom surface, or as an alternative, under an inclination angle such that the canula (36) is inserted under an angle. The steering drum (70) is biased by the spring (71) to rotate in one rotation direction and rotation of the steering drum (70) is prevented by a stop means (72) with a counter arrester (72a) which abuts the first arrester (70a) of the steering drum (70). The stop means (72) has a longitudinal rotation axis (72c) and a coupling member (72b). The coupling member (72b) is preferably equipped with a ratcheting element or flexural element which can, for example, interact with the third part (3) of the coupling mechanism, such that the drive mechanism can be coupled to the stop means (72).

A view from the bottom of the device of a detail of the needle insertion mechanism according to the second embodiment is shown in Figure 31. The steering drum (70) has a gearing (70c) which is attached or attachable to the bottom surface and/or longitudinal axis of the steering drum (70). The gearing is preferably composed as a toothing which matches a corresponding toothing (73a) on a spike carrier (73) which carries the spike (31) that can be inserted in a reservoir, preferably having a septum. The spike carrier (73) has a guiding means or guiding slot (73b) which is preferably engaged with the housing and guides the spike carrier (73) as the carrier is driven towards the reservoir.

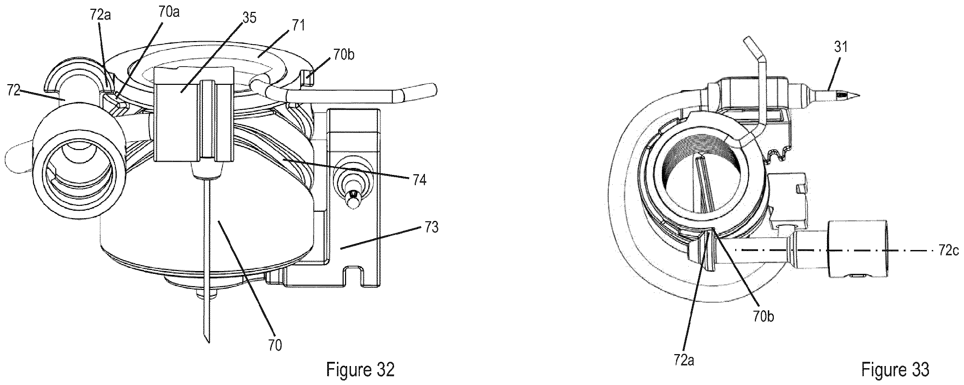

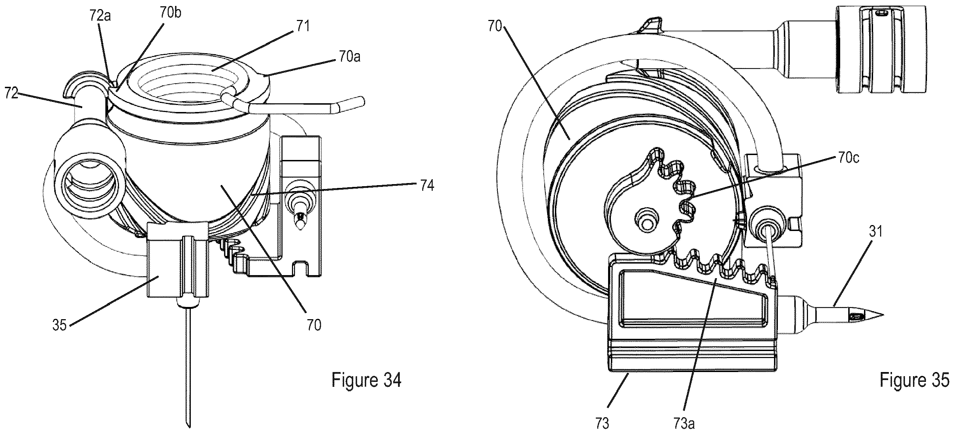

In Figure 32, the stop means (72) is rotated around the rotational axis (72c), for example by the third part (3), such that the counter arrester (72a) is moved out of abutment with the first arrester (70a) of the steering drum (70), such that the spring (71) rotates the steering drum over a first angle until the stop means (72) catches the second arrester (70b), see also Figure 33. The counter arrester is preferably shaped as a semi-circular arch or rim and rotation of the stop means over the first angle releases the contact between the counter arrester and the first arrester (70a), but brings the semi-circular arch or rim within the line or rotation of the second arrester (70b). As the steering drum rotates, the guiding means (74) on the steering drum (70) drives the canula holder (35) to the needle inserted position (Figures 33 and 34). The guiding means (74) preferably has a sinus shape and during the rotation of the steering drum (70), the transformation means of the canula holder (35) is driven by the guiding means (74) as the guiding means rotates and the transformation means goes from the maximum of the sinus shaped curve to the minimum. The canula holder (35) is linearly guided by the housing as it moves to the needle inserted position. During rotation of the steering drum (70), the gearing (70c) drives the spike carrier (73) towards the reservoir, as presented in Figure 35. The spike carrier is guided by the housing, preferably in a direction perpendicular to the movement of the canula holder.

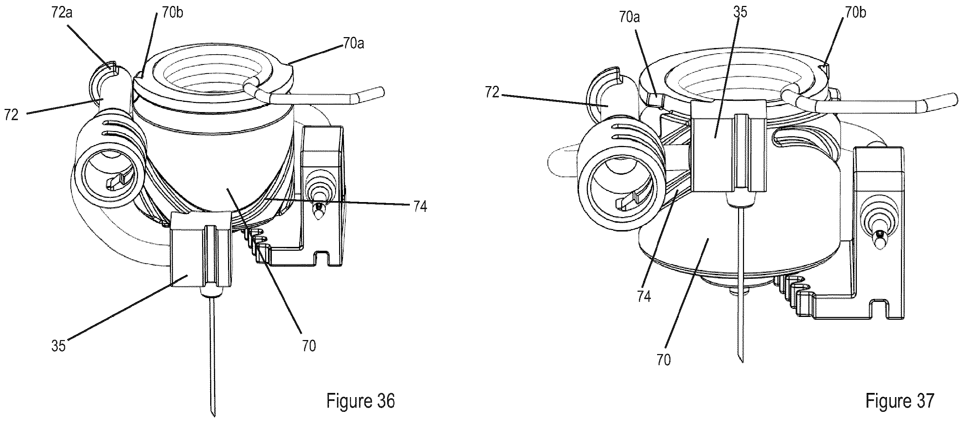

The release of the second arrester (70b) with the counter arrester (72a) of the stop means (72) is shown in Figure 36. The stop means (72) is rotated further in the same direction as the first rotation for releasing the first arrester, the stop means (72) is rotated over an angle that is greater in magnitude as the first rotation angle. The zero angle position is hereby defined by the starting position of the stop means (72) when the counter arrester (72a) abuts the first arrester (70a) of the steering drum (70). The release of the second arrester (70b) ensures that the spring or biasing means (71) can rotate the steering drum (71) further in the same rotation direction as the first rotation. The further rotation of the steering drum (70) ensures that the guiding means (74) drives the canula holder (35) back to the needle retracted position as the guiding means goes from the minimum to the maximum of the sinus curve (Figure 37). The canula holder is guided by the housing during the axial shift. The spike inserter is not moved during the further rotation after releasing the second arrester since there are no engaging teeth between the gearing and the inserter (Figure 35).

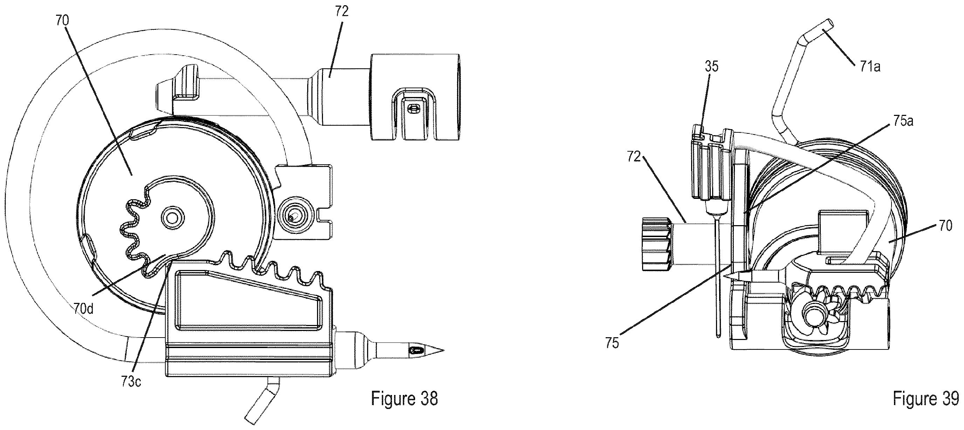

The rotation of the steering drum (70) after releasing the second arrester (70b) is halted when a gearing stop (70d), which is part of the gearing (70c), abuts a stop on the housing, or preferably a stop (73c) on the spike carrier, Figure 38.

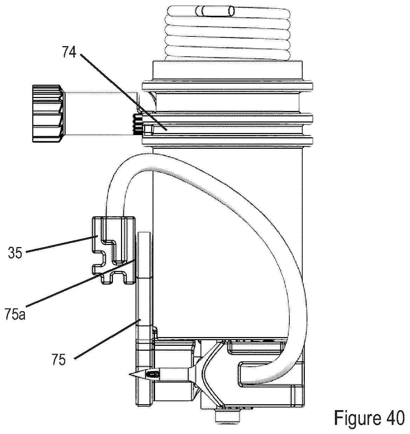

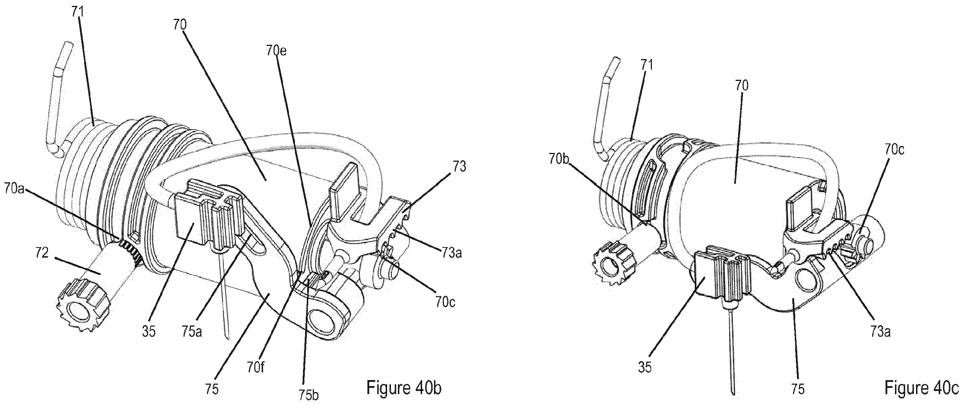

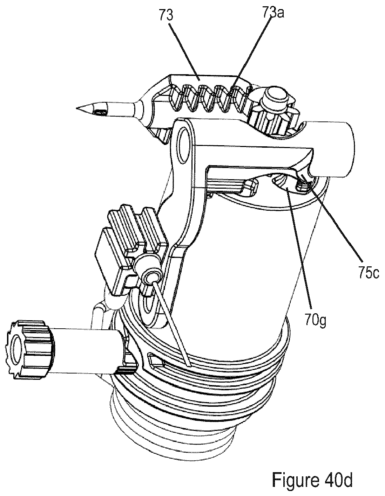

A needle insertion mechanism according to a third embodiment is presented in Figures 39 and 40. The rotational axis of the steering drum (70) is preferably oriented parallel to the bottom surface of the device. The stop means (72) has a toothing on one end which can be coupled to another part, preferably the third part (3). The steering drum (70) is biased by a spring and rotation of the stop means (72) releases the steering drum (70) to rotate over an angle until the stop means catches or abuts the second arrester. A gearing is connected to the longitudinal axis which inserts a spike carrier via the gearing mechanism as the steering drum rotates over the first angle. The gearing has a toothing and the toothing advances the spike as the steering drum rotates. Rotation of the steering drum furthermore rotates a lever arm (75), to drive the canula holder towards the patients skin via a guide opening (75a) in the lever arm (75) that is adapted to be connected or connectable to the canula holder (35). Further rotation of the stop means ensures that the steering drum is also rotated further and the lever arm moves the canula holder back towards the needle retracted position. The torsional spring (71) biases the steering drum (70) which is held in its position by the stop means (72) abutting the first arrester (70a) of the steering drum (70), see Figure 40b. Gearing (70c) is attached to the end of the steering drum, the gearing having a toothing which matches corresponding toothing (73a) of the spike carrier (73). The spike carrier (73) has a wing which ensures that the carrier can be guided by the housing for a linear movement. The steering drum (70) has a circumferential rim (70e) located at the end of the steering drum (70). The lever arm (75) has the guide slot (75a) for guiding the canula holder (35) from the needle retracted to the needle inserted position by rotation of the lever arm. Rotation of the stop means (72), releases the abutment with the first arrester (70a) and the steering drum (70) starts rotating until the stop means (72) abuts the second arrester (70b, Figure 40c). Rotation of the steering drum ensures that the end (70f) of the rim (70e) abuts a steering element (75b) which is part of the lever arm (75) and ensures that the lever arm starts rotating. Rotation of the lever arm drives the canula holder (35) from the needle retracted to the needle inserted position (Figure 40c). During the rotation of the steering drum (70) the gearing (70c) ensures that the spike (73) is moved and inserted in the reservoir. The rotation of the lever arm 75 is halted by a second steering element or second stop (75c, Figure 40d), which is part of the lever arm mechanism and the second steering element (75c) is moved in a recess (70g) of the steering drum. As the steering drum is rotated, an abutment between the second steering element (75c) and the wall surface of the recess (70g) stops the rotation of the lever arm. Once the stop means (72) is rotated over an additional angle, the abutment with the second arrester (70b) is released and the steering drum (70) rotates further, whereby the abutment between the second steering element (75c) and the recess (70g) of the steering drum ensures that the lever arm rotates back and consequently the canula holder moves back to the needle retracted position.

A first embodiment of the drive mechanism of the medication delivery device is shown in Figure 41. A segmented piston rod (80) is embodied in a guidance for the piston rod (4f) which is a housing part, which guides the elements from the drive mechanism along a 180° U-turn or curve into the opening of the reservoir (26). This arrangement ensures that the longitudinal axes of the motor for the drive mechanism and the longitudinal axis of the reservoir are oriented parallel to each other to reduce the length of the device. The segmented piston rod (80) is composed of multiple segments (81) which are connected to each other laterally via a hinge (82), preferably via a strap hinge. When subsequent hinges are articulating, the piston rod can curve or bend and when all hinges are closed all segments abut each other on the side opposed to the hinges (82a) to form a stacked configuration. In the latter case, the piston rod behaves like a rod, quasi like a non-segmented piston rod for efficient load transfer. The segmented piston rod preferably has a drive segment (81a), which is typically the first segment. The drive segment (81a) preferably has an internal thread segment that matches the outside threading of the threaded rod (15). The threaded rod (15) is rotated by an electromotor (83) which drives the threaded rod via a gearing (84) in combination with a worm wheel (85). The number of rotations is measured by an encoder (86) which is either part of the electromotor or as a separate optical system using, for example, a LED light source, an external disc shaped chopper in combination with an optical sensor or magnetically controlled sensor (not shown). Rotation of the threaded rod (15) advances the drive segment (81a) of the piston rod as the piston rod is prevented from rotation by the housing part (4f). Subsequent segments are advanced via the hinges.

Advancement of the drive segment (81a) advances transfer segments (81b) which are adjacent to the drive segment and which are U-shaped to enclose the threaded rod (15). The transfer segments (81b) are connected to delivery segments (81c) which are intended for making the U-turn and for partially entering the reservoir. The last segment (81 d) of the piston rod (81) has a flange (81 e) or connector for connecting to the plunger (28) of the reservoir.

The guidance (4f) of the segmented piston rod (82) in the housing is illustrated in Figure 41a. The guidance (4f) guides the segmented piston rod such that the piston rod is bent in one direction and guided towards the entrance of the reservoir. For guiding the piston rod, each segment has two wings (81 h, 81 i) which extend from each segment (81) and fit into a guide slot (4p) of the guidance (4f) for the piston rod. The guide slot (4p) in combination with the wings (81 h, 81 i) ensure that the piston rod cannot rotate and that the piston rod is bent during advancement of the segments.

Advancement of the piston rod advances the plunger in the reservoir until the reservoir has been emptied as presented in Figure 42. The number of revolutions for emptying the reservoir are typically recorded by the sensor system having an encoder and the data are transmitted to the processor of the device. The processing unit can use the data for the control of the coupling mechanism described above. The last segment (81d) of the segmented piston rod has a guiding means (87), preferably shaped as a fin which points into the direction of the next-to-last segment. The guiding means can pass through notches of the segments adjacent to the last segment (81d), preferably through a notch or groove suitable for receiving the guiding means when the segmented piston rod transfers from the curved to the linear configuration. The guiding means (87) does not interfere or engage with the segments adjacent to the last segment, e.g. the groove or notch does not interfere with the fin shaped guiding means. The guiding means is preferably also guided by the housing part (4f) prior to entering the reservoir, for example by a cut-out (4i) of the housing or housing part (4f). The guiding means has an edge (87a) which is directed parallel to the normal (81g) of the last segment (81e). When entering the cartridge, the edge (87a) interferes with the wall of the reservoir (26) and/or cut-out (4i) of the housing, and thereby guiding the last segment (81d) such that the flange (81e) is prevented from off-axis entrance into the reservoir, thereby enabling an efficient loading of, or coupling to the plunger and preventing blockage or buckling of the segmented piston rod (80) in the reservoir. The correct guidance of the last element (81d) is transmitted to the adjacent elements due to the hinged connection which is stiff enough to withstand any warpage between the elements. The guiding means (87) or fin partially functions as a lever arm and thereby arranging a parallel orientation of the normal of the last segment (81 g) and the longitudinal axis of the reservoir. The guiding means or fin also prevents tilting of the last segment once connected to the plunger, the tilting forces are compensated for by the interaction between the fin and the inner wall of the reservoir (Figure 46). The fin is positioned opposite to the hinge axis of the strap hinge and the plane of the fin is oriented perpendicular to the hinge axis with the edge (87a) facing the inner wall of the reservoir.

A second embodiment of the drive mechanism is presented in Figure 44, using a spring or coil (88) instead of the segmented piston rod. The spring (88) preferably is a close wound-up helical coil and the windings of the spring can bend versus adjacent windings such that the spring can form the U-turn before entering the reservoir. A flange (81 e) is attached or attachable to one end of the spring for abutting the plunger of the reservoir. On the other end of the spring, a nut element (81a) is attached to the spring having an internal threading matching an external thread of the threaded rod. Rotation of the threaded rod advances the spring-type piston rod, alike the first embodiment.

A third embodiment of the drive mechanism is shown in Figure 45, the transfer segments (81b) are equipped with a teething forming a toothed rack that matches a gear wheel (89) and rotation of the gear wheel advances the segmented piston rod.

In Figure 46, a schematic representation is shown of the forces acting upon the last segment when medication is expelled. The last segment intends to tilt and the guiding element counteract the tilting moment to prevent incorrect abutment of the plunger.

The assembly of the subunits to form an assembled device having a sterile environment for the needle insertion unit, and which can connect to a sterile cartridge will be discussed in the following. In Figure 47, a filled cartridge (26) is assembled with the cartridge holder (4d) of the subunit comprising the needle insertion and retraction mechanism. The subunit is preferably sterilized, for example using ETO and brought into an aseptic environment, for example in a fill-finish unit. The cartridge (26) is filled with the medication at the fill-finish line in an aseptic environment and closed with a plunger (28). The cartridge is closed with a septum (27) which is connected to the neck of the cartridge using a crimp (27a). The full cartridge is inserted, preferably in the aseptic environment into the opening of the cartridge holder (4a). The cartridge is fixated in the holder with the cartridge fixator (56), which fixates, for example around the neck of the cartridge, the cartridge in the holder to reduce or eliminate axial movements of the cartridge (26) in the holder (4a). In this example, the cartridge is fixated using an ellipsoidal shaped ring that can flex around the crimp of the cartridge. The long axis of the ellips fits between the neck of the cartridge and the crimp. The cartridge fixator (56) also ensures that the end of the crimp (27a) and/or septum (27) remains in a preferably fluid- and air-tight contact with a sealing element (121) which is part of the cartridge holder. The sealing element preferably is made from an elastic material and after insertion there is a sealing between the end of the cartridge, e.g. the crimp and/or septum, and the sealing element (121) which ensures that there is a sterile barrier between the cartridge and the cartridge holder. Since the parts of the needle insertion and retraction unit which are within the needle housing cover (4l) were already sterile, the sterility remains guaranteed while the assembly of the cartridge holder and cartridge is outside the sterile environment. Additionally, the passage (4n) for the needle needs to be sealed by a sterile barrier and the connection to the coupling mechanism requires a sterile sealing as described above. The sterile barrier element forms a sterile enclosure (120) which encloses the fluid path unit and which is indicated as a thick line in Figure 47. The device can now be assembled with the bottom housing part (4g) and the housing cover (4j), which can be done outside the sterile environment.

An alternative for the sterile barrier between the cartridge (26) and the housing of the fluid path is shown in Figure 48. In this example, there are several functionalities combined in the sealing element (122). The sealing element (122) has a sealing surface (122a) which, once in abutment with a counter surface of the fluid path unit, ensures a sterile barrier. The crimp combines the features of providing a stable and sterile connection between the glass barrel of the cartridge (26) and the septum (27), a mechanical connection to the cartridge holder using connector (122b) which can snap fit into passage (4e) of the holder, and finally a sterile barrier between the cartridge and the cartridge holder using sealing surface (122a) and counter surface (123) of the cartridge holder. The sterile barrier or sterile enclosure is indicated with (120). The assembly of the device with the bottom surface (4g) and housing cover (4j) is described above.

Another alternative for the cartridge fixation and sterile barrier configuration is presented in Figure 49. The cartridge fixator (56) is tubular shaped and at least partially encloses the cartridge (26). The cartridge fixator is attachable to the needle insertion unit via a snap fit connection using flexural element or a screw type of connection. The connection between the cartridge fixator (56) and the needle insertion unit is such that is forms a tight and sterile barrier. The tubular shaped fixator (56) is open on one end to receive the cartridge end with the plunger. A sterile barrier (56a) is located on the opposite end of the fixator (56), preferably shaped to receive connector (81e) of the last segment (81d) of the segmented piston rod (80). After aseptic assembly of the cartridge (26) with the fixator (56), the whole cartridge is enclosed in a sterile environment or sterile enclosure (120) during shelf life. Once the bolus is injected from the device, the advancement of the piston rod punctures the sterile barrier (56a) for advancing the plunger (28). The sterile barrier (56a) is preferably made from a material that combines a sterility barrier but enables the connector (81e) of the last segment to penetrate the barrier, e.g. it is made from a paper (cellulose) type of material or Tyvek. The connector (81e) can be equipped with a puncturing device such as a sharp tip or fin which is an integral part of the last segment.

In Figure 49a, an example is presented for an assembly of an empty cartridge (26) that is inserted into the cartridge holder (4d) with the needle insertion and retraction mechanism. Both the cartridge and the needle insertion subunit comprising the cartridge holder (4d) are brought into an aseptic environment, assembled and packaged again, preferably in a sterile tub. The packaged subunits comprising the empty cartridge are subsequently brought to a fill-finish line where the subunits are removed from the packaging the subunits are filled and stoppered with a plunger in an aseptic environment.

The subunit comprising the needle insertion and retraction mechanism is assembled such that the medication in the cartridge remains in a sterile environment from the assembly throughout shelf-life and subcutaneous delivery. The subunit has at least three passages to the ambient, the passage for the coupling device, for example the third part (3), the connection to the reservoir (26) as described above and the passage (4n) for the needle. The latter is described in the following paragraphs.

In Figures 50 and 51, the passage (4n) for the needle is closed by a peel foil (108) which is attached to the end surface (4o) of the passage. The peel foil (108) can be made from a gas permeable material like Tyvek to enable gas-plasma or ethylene oxide sterilization of the compartment inside the housing cover (4j) comprising the needle insertion unit. Alternatively, the foil is made from a barrier film such as PET or PE/PA multilayer foil if other sterilization techniques such as gamma or e-beam are used. After assembly of the cartridge, a sterile enclosure (120) surrounds the needle insertion mechanism as is schematically indicated with a thick line. The needle insertion subunit preferably already comprises the peel foil (108) which is attached to the end surface (4o) using welding, ultrasonic welding or glueing techniques. The subunit is assembled with the top cover (4j) and bottom surface (4g) and an additional sticker or adhesive foil (107) is attached or attachable to the peel foil of the adhesive layer (105). The sticker (107) makes the connection between the peel foil (105) of the adhesive layer and the peel foil (108) of the insertion unit, e.g. it adheres both to both foils. The assembly of the stacked peel foils is presented in Figure 51, the user removes the peel foil (105) of the adhesive layer (104) and, via the sticker (107), releases the bonding between the end surface of the passage (4o) and the sterile peel foil (108).

Another example for the closure of the passage (4n) is presented in Figure 52. The needle insertion sub-unit is closed by the peel foil (108) forming a sterile barrier alike the previous example. However the bottom surface (4g) comprises a peel foil (105) for the adhesive layer (104) which closes the passage (106) of the bottom surface. In the assembled device, the peel foil (105) of the bottom surface directly contacts and adheres to the sterile peel foil (108) of the needle insertion sub-assembly. The adhesive contact can be, preferably, due to a separate adhesive layer either present on the surface of one of the two peel foils (105, 108).

In the examples of Figures 50 to 52, the bottom surface (4g) is a separate housing part. In Figures 53 to 55, examples are presented where the bottom surface is not a separate housing part but is integrated with the needle insertion and retraction sub-assembly.

In Figure 53, the passage (4n) of the needle insertion compartment is closed with the peel foil (108) as described above, the bottom surface of the device is integrated with the needle insertion sub-assembly and the adhesive layer (104) and top cover (4j) are attached to the sub-assembly. In this example, there is a direct contact envisaged between the foils (105) and (108) alike the example of Figure 52, but as an alternative also a sticker (107) can be used to contact the peel foil (105) of the adhesive layer to the sterile foil (10), alike the example described in Figure 50.

In yet another example, no separate foil (108) closes the passage (4n) of the needle insertion compartment, see Figure 54. The sterile barrier from the inside of the compartment to the ambient is governed by the peel foil (105) for the adhesive layer (104). This implies that the adhesive layer including the peel foil (10) are already attached to the bottom surface of the needle insertion sub assembly when the cartridge (26) is inserted or that the compartment itself is sterilized in a final sterilization step. The peel foil (105) is attached or attachable to the end surface (4o) of the passage (4n) for the needle as shown in Figure 54 or the peel foil is not connected to the end surface (4n) as presented in Figure 55. In the latter case, sterile barriers are required at the rim of the adhesive layer or which are integrated within the surface of the adhesive layer, which is indicated with (124) in Figure 55. The sterile enclosure of the example presented in Figure 55 encloses the fluid path which is in the housing of the needle insertion subassembly, encloses the passage for the needle (4n) and the adhesive layer (104). The sterile enclosure is schematically indicated as the thickened line (120) in Figure 55.

The adhesive layer (104) ensures that the device can be attached to the skin of the patient after removing the peel foil (105). For removing the device after the injection, the adhesive layer may have a lid, or a part attached to the adhesive layer that can easily be held or grabbed by the patient to remove the device from the skin, the lid preferably having an area with non-skin adhering properties.

A method for sterilization of a fluid path unit (25) is explained in Figures 56 to 61. The reservoir or cartridge (26) comprises a glass barrel (125) having a circular cross section and a longitudinal axis (L'). At the distal end of the cartridge (26), a neck section (125a) is formed ending in flange (125b) having a circular opening for receiving the septum (27). The septum (27) comprises a circular protruding ring (27b) which fits into the opening of the flange section of the cartridge. The septum (27) can be attached to the glass barrel using a crimp (27a) which can be made from a plastically deformable material such as a metal or a suitable polymer (Figure 56). The septum is made from a polymeric and elastomeric material and for handling purposes often coated with a coating such as a fluoropolymer polymer (e.g. fluro-tec) coating. Additionally such a coating prevents that volatile components of the rubber compound can enter the medication. Using the autoclaving (hot steam sterilization) method ensures that all components, especially at the interface (126) between the glass barrel and the septum become sterile.

In an example of the sterilization method, the septum (27a) is attached to the flange (125b) by first inserting the septum (27) into the opening of the flange (125b) and then plastically deforming the proximal rim (27d) of the crimp (27a) to achieve a irreversible connection between the septum (27a) and the glass barrel (125), Figure 57. The distal end surface of the cartridge thus has a central circular area with the elastic material of the septum (27) that can be penetrated by the spike (31) which is surrounded by a rim of the crimp (27a) that is preferably non-piercable by the spike (31). The empty cartridge (26) with the attached septum (27) is preferably sterilized in an autoclave using hot steam such that the whole cartridge becomes sterile, including the sterile interface (126) between the inner surface of the flange section (125b) and the outer surface of the septum (27) that is in direct contact with the glass surface. When removing the empty cartridge from the autoclave, the direct interface (126) is prevented from contact or exchange with the non-sterile ambient since a leak proof and sterile barrier is formed during the crimping. The remainder of the empty cartridge can become non-sterile once exposed to the ambient. In an alternative, the septum (27), the crimp (27a) and the glass barrel (125) are rinsed or washed with an alcohol or peracetic acid solution prior to the assembly. This ensures that the interface (126) between the septum and the glass surface is sterile without the need for autoclaving the assembly.

The method for sterilizing the fluid path unit (25) further comprises the insertion of the empty cartridge (26) into a second cavity (128) of the fluid path unit. The second cavity (128) may comprise the cartridge holder (4d). The cartridge is inserted into the second cavity (128) with the septum side ahead and the longitudinal axes (L') of the cartridge and the second cavity are preferably aligned first. At the end of the second cavity (128) there is an end wall (136) comprising a passage or opening (130). The end wall (136) separates the second cavity (128) from a first cavity (127). The first cavity (127) preferably encloses the fluid path with the spike (31), the tubing (43) and the needle (36). The second cavity preferably has the passage (4n) for the needle (36) with the sterile foil (108) attached to the end surface (4o) of the passage for the needle (4n). The second cavity (128) is attached or attacheable to the first cavity (127) or the first and second cavities form one enclosed volume that is separated by the end wall (136). Before inserting the cartridge, there is direct communication between the first (127) and the second cavity (128) via the opening (130) and, provided that the sterile foil (108) is gas permeable, also a direct communication for exchanging gaseous substances from the outside of the sterile foil to the second cavity. A circumferential rim (129) is present on the outside of the end wall (136) pointing towards the cartridge and which surrounds the passage (130). The circumferential rim (129) is preferably circular shaped having a diameter that fits into the circular area of the septum present at the distal end of the cartridge that is not covered by the crimp. Thus the inner diameter of the crimp at the distal end is above the outer diameter of the circumferential rim (129). The rim (129) preferably has a triangular shaped cross section (Figure (58)) such that when the cartridge (26) is moved forward in the second cavity (128) that the circumferential rim (129) can elastically deform the distal end surface of the septum to form a fluid tight sealing (Figure 59). As an alternative, the end wall (136) may comprise an elastomeric component (131) that is injection molded onto the end wall (136) or inserted as a separate part. The elastomeric component (131) is elastically deformed when the cartridge (26) is inserted into the second cavity (128), the elastomeric component closes the axial and/or radial end surface of the crimp and thereby provides the sealing (135) between first cavity and the second cavity (Figure 59a). The elastomeric component can also be provided as a separate sealing element such as an O-ring present between the end wall (136) and the distal end surface of the crimp (Figure 59b) or the sealing is present between radial surface of the crimp and the second cavity (Figure 59a).

The sealing between the first and second cavity provides a sterile barrier during shelflife and use. The first cavity (128) comprising the fluid path and distal end surface of the septum remains in a sterile condition during shelflife of the product. The remainder of the device including the second cavity (128) is exposed to the non-sterile ambient during shelflife and thus the sealing between the first and second quality ensures that the first cavity remains sterile. The quality of the sealing (135) which is established during insertion of the cartridge (26) is preferably tested during assembly, e.g. during in-process control (IPC). The sealing (135) can be tested using a pressure difference between the first (127) and second (128) cavity, for example the second cavity containing the inserted empty cartridge is closed off, pressurized (or vacuumized) and checked whether the pressure (or vacuum) level remains constant. If the pressure level in the second cavity (128) drops, then this is caused by an imperfect sealing between the first and second cavity. Alternatively, a trace gas method is used, for example, the first cavity (127) is flooded via the passage for the needle (4n) with a gas such as Helium and the Helium concentration in the second cavity is measured with an adequate analytical method such as gas chromatography, Infrared or Raman spectroscopy or the like.

After insertion, the cartridge (26) is axially and/or rotationally fixed to the fluid path unit (25) against the biasing force of the elastic element (131) or against the biasing force of the rim (129) deforming the septum (127) thus providing the sealing (135) between the first and second cavity. A fixation element such as a U -profile can fit around the neck (125a) of the cartridge and can be inserted perpendicular to the longitudinal axis of the cartridge such that the legs of the U-profile catch counter elements present in the cartridge holder or fluid path unit. Optionally, the legs of the U-profile or the fixation element itself are additionally secured by locally melting, welding or glueing the profile or element to the fluid path unit.

The assembly of the cartridge (26) into the fluid path unit (25) can be done in a clean room environment, e.g. a non-sterile environment. During this assembly the interface (126) between the glass barrel and the septum will remain sterile. The assembly of the fluid path unit with the cartridge (26) can be done at a different location than the assembly of the cartridge with the septum. The sterilization of the cartridge prior to the assembly into the fluid path unit can even be done at a third location, for example a service provider for autoclave sterilization.

The assembled fluid paths are preferably placed in a tub (137), oriented vertically with the open end of the empty cartridge facing the top of the tub. The fluid paths units are positioned in trays (132) that fit into the tub (137) such that the open ends of the cartridges are all at fixed positions in the tub such that the fill finish line can be adapted to the specific tub configuration for the fluid path units. After positioning into the tubs, the tubs are closed by a liner and preferably a two- or three-fold peel pouch. The peel pouches and/or the liner are preferably adapted for gas sterilization techniques. The assembly of the fluid paths into the tubs and closure of the tubs is preferably done in a clean room environment.

In the next step of the sterilization method, the first cavity (127) of the fluid path unit comprising the needle (36) and spike (31) is sterilized, preferably using gas sterilization such as ethylene oxide or a hydrogen peroxide plasma. The first cavity is flooded with the sterilization agent preferably via the passage (4n) for the needle (36), which itself is closed by a gas permeable peel foil (108). In an alternative, the peel foil for the first cavity is not gas permeable and the first cavity has separate openings covered by a gas permeable medium such as a foil (for example Tyvek) or a porous sintered body which is part of the wall of the first cavity. The first cavity (127) can have a sterilization indicator, which is attached to the outside of the cavity or it is an integral part of the porous body and such indicator changes color during the sterilization step. The first cavity (127) can be sterilized separately, e.g. the ETO gas is specifically directed to the first cavity only and afterwards flushed to remove the toxic components from the first cavity. For example a connector to the passage (4n) is used to specifically flood and decontaminate the first cavity (127) only.

The first cavity is closed by the sealing (135) from the second cavity and thus the ETO gas cannot go to other compartments of the fluid path unit. Alternatively, both the first and the second cavities are sterilized, for example by putting the whole tubs with the assembled fluid path units, including the empty cartridges, into an ETO sterilizer. If required, first one or more peel pouches are removed from the tub (137) prior to sterilization, and then the whole batch of fluid path units is sterilized. During sterilization both the first and the second cavity on both side of the sealing (135) are sterilized. The glass barrel (125) of the cartridge is sterilized both on the inside and on the outside. By sterilizing the first cavity, at least the spike, the needle, the tubing and the distal end surface (27c) of the septum are sterilized.

The space between the outside of the glass cartridge (26) and the inside of the second cavity (128) or cartridge holder is sterilized as well. With this sterilization step it is not possible to sterilize the direct interface (126) between the septum (127) and the glass barrel (125) which was therefore previously sterilized using an autoclave. Optionally, the tubs (137) containing the fluid paths are repackaged in a peel pouch again which facilitates the transport of the tubs to another location if required.

A detail of the tray (132) of the tub with the vertically positioned fluid path unit (25) is shown in Figure 60. The empty cartridge (26) is being filled with a liquid medicament (134). The filling is preferably done in an aseptic environment, which can be at a different geographical location then the locations for the assembly of the cartridge into the fluid path unit and the sterilization of the first and/or second cavity. Finally a stopper or plug (28) is inserted to close the filled cartridge (Figure 61). Thus the contents of the cartridge is closed in a sterile environment and the sealing (135) between the second cavity and the first cavity ensures that the first cavity will remain sterile once the the assembled and medicament filled fluid path unit is in a non-sterile environment. With this sterilization method, the sterility of the enclosure (120) can be ensured.

As a final step, the fluid path units (25), which are preferably still present in the tubs (137), are assembled with a drive unit. This assembly is preferably done in a non-sterile environment such as a clean room or in the ambient, this assembly can be done at the manufacturer of the fluid path units, at the location of the company providing the sterilization process, at the location of the company providing the fill finish services or directly at the pharmaceutical company that will deliver the assembled delivery devices to the customers. This sterilization and assembly method thus guarantees a maximum flexibility and leaves many options open who and where the assembly steps are done.

In the embodiments presented above several cartridge fixation systems are presented. In Figure 47, a cartridge fixator (56) shaped as an ellipsoidal ring is presented. The axis of the ellipse can be deformed such that it fits around the neck of the cartridge. In Figure 48, the crimp (122) is equipped with a snap fit connector (122b) which ensures that the cartridge can be fixed with a counter connector of the cartridge holder. In Figure 49, a tubular shaped connector (56) is presented which encloses the proximal end of the cartridge and includes a sterile barrier (56a) for the proximal end of the cartridge that will be penetrated once the piston rod advances. Finally in Figures 59a and 59b, an elastomeric component or O-ring (131) is shown which in a press-fit configuration fixates the cartridge. A further embodiment for a cartridge fixation system using an adhesive and elastic attachment means is presented below.

In Figure 62 a multi-layered system, or elastic attachment means (138) is shown, for example a double sided adhesive tape, comprising at least one core-layer (139) and at least two adhesive layers (140, 140a) that are attached to the at least one core layer (139). The core layer (139) is preferably made from a foam material, for example a polyurethane foam or a polyolefin foam. The foam material is such that is recovers its original shape after release of the pressure once it has been compressed, e.g. is a resilient material. The at least two adhesive layers (140, 140a) are preferably based on an acrylic material, epoxy based, silicone adhesive material or synthetic rubber based adhesives. The adhesive layer is covered by a liner sheet (141). In this example, the multi-layered system is ring-shaped having a passage (142) which in the method for fixation of the cartridge is preferably aligned with the passage (130) present in the end-wall (136) between the first cavity (127) and the second cavity (128), see also Figure 64. In this embodiment a ring-shaped structure is presented but other shapes that are preferably closed can also be envisaged such a rectangular shaped multi-layered systems. The ring-shaped structure has two concentrically arranged wall surfaces or lateral surfaces. The inner wall surface of the ring (143) and the outer wall surface of the ring (144) are non-adhering, whereas the adhesive surfaces (140, 140a) are adapted for attachment once the liner sheets (141) have been removed.

In Figure 63, the multi-layered adhesive system (138) has been attached to the distal end surface (27c) of the crimp (27a). First one of the two liner sheets (141) has been removed, the adhesive surface has been correctly positioned onto the distal end surface (27c) of the crimp and the ring-shaped multi-layered system (138) has been pressed onto the crimp (27a). In a next step, the second liner sheet can be removed to expose the second adhesive layer (140, 140a) that is still available. In this example, the ring-shaped adhesive system is first attached to the cartridge (26), however the adhesive system can also be first applied to another surface, preferably the end wall (136) between the first (127) and second (128) cavity (Figure 65). The adhesive system can also be applied to the outside surface (125) of the glass barrel, preferably after the outside surface has been cleaned to remove any silicone oil residues. In another alternative, double-sided adhesive tape is attached to the lateral wall surface (27e) of the crimp. For example a strip of the double sided adhesive tape is wrapped around the lateral wall surface (27e) of the crimp and then the second liner sheet (141) is removed before inserting the cartridge into the cartridge holder.

In Figures 64 and 65 a cross-section is shown of a cartridge (26) that has been inserted into the cartridge holder (4d). The distal end of the cartridge (26) is received in the second cavity (128) of the cartridge holder (4d) and the multilayered system (138), for example the ring-shaped double-sided adhesive tape of Figure 63 connects the distal end surface (27c) to the end-wall (136) that separates the first cavity (127) from the second cavity (128). The adhesive layers (140, 140a) contact the crimp (27a) and the distal end wall (136) and in between the two adhesive layers is the core layer (139). The latter is elastic and can be compressed or extended while the adhesive layers remain in contact with the wall surfaces. The inner and outer lateral wall surfaces (143, 144) of the multilayered system (138) separate the passage or opening (130) from the second cavity (128) and form a sterile barrier. Once the cartridge has been fixated, both lateral wall surfaces (143,144) are preferably in a non-sterile environment. If the first cavity (127) is sterilized with a gas sterilization method then the contents and surface walls of the first cavity are sterilized. This implies that distal end surface of the septum (27) and the inner wall surface (143) of the multilayered adhesive system (138) is sterilized as well. The outer wall surface (144) may become sterile as well but once exposed to the ambient, the outer wall surface (144), potentially becomes non-sterile, while, due to the sterile barrier formed by the multilayered adhesive system, the inner wall surface (143) and first cavity (127) remain sterile.

Any axial, radial or rotational movements of the cartridge are dampened by the elastic core layer (139) of the multi-layered system, while keeping contact between the cartridge and the cartridge holder thus ensuring that once a sterile environment has been established within the first cavity (127) that this will remain sterile during shelf-life. The inner wall surface (143) and the end surface of the septum are part of the sterile enclosure (120) of the first cavity (127) as described above.

The cartridge (26) can be fixated to the cartridge holder (4d) by using several double sided adhesive tapes as presented in Figure 66. The outside surface of the glass barrel (125) is attached in this example to the housing (4) using two additional double sided adhesive tapes (138) to compensate for lateral movements of the cartridge (26) versus the housing (4).

The double sided adhesive tape may be provided on a role and the ring-shaped elastic attachment means may be already predetermined punching the desired shape during manufacturing of the role. The punching punches through the elastic layer and adhesive layer, but preferable keeps the liner sheet intact. Alternatively, not all layers are punched but still prepare a predetermined breaking point/ area in the layers. The role of tape can be unrolled to express one of the two adhesive layers and the cartridge can easily be pressed onto the adhesive layer (Figure 67). When removing the cartridge, the ring-shaped adhesive tape is released and attaches to the distal end of the cartridge.

| | | | |
|---|---|---|---|
| **Part annotation** | | (8) | O-ring, friction element |
| (1) | Ratchet shaft, first part | (9) | Notch |
| (2) | Coupling member, second part | (10) | Pin, protrusion, thread segment |
| (3) | Cam shaft, third part | (11) | Asymmetric toothing, second part |
| (4) | Housing | (12) | Asymmetric toothing, third part |
| (4a) | Housing fluid path unit | (13) | Cam, coupling member third part |
| (4b) | Spring holder | (14) | Gearwheel |
| (4c) | Vertical wall housing | (15) | Threaded rod |
| (4d) | Cartridge holder | (16) | Toothing gearwheel |
| (4e) | Passage for cartridge fixator | (17) | Bearing member |
| (4f) | Guidance for piston rod | (18) | Thread on threaded rod |
| (4g) | Bottom housing part | (19) | Rotational axis |
| (4h) | Drive carrier part | (20) | Sealing element, second friction fit |
| (4i) | Cut-out housing part | (21) | Needle control element |
| (4j) | Housing cover | (22) | End surface third part |
| (4k) | Button | (23) | End surface second part |
| (4l) | Needle housing cover | (24) | Bottom surface |
| (4m) | Window | (25) | Fluid path unit |
| (4n) | Passage for needle | (26) | Reservoir |
| (4o) | end surface passage | (27) | Septum |
| (4p) | Guide slot | (27a) | Crimp |
| (5) | Toothing | (27b) | Circular protruding ring |
| (6) | Gearing, threading, guide slot | (27c) | Distal end surface |
| (7) | Flange | (27d) | Proximal rim of crimp |
| (27e) | Lateral wall surface crimp | (49) | Second angle of rotation needle control element |
| (28) | Plunger, plug, stopper | | |
| (29) | Longitudinal axis reservoir | (50) | Linear guide housing |
| (30) | Spike inserter carrier | (51) | Second guiding means spike inserter carrier |
| (30a) | Base surface spike inserter carrier | (52) | Third guiding means on housing |
| (30b) | Longitudinal axis | (53) | Fourth guiding means on housing |
| (31) | Spike | (54) | Rotation around first key |
| (31a) | Tip | (55) | Cams of third part |
| (31b) | Base spike | (55a) | First cam |
| (32) | First position spike inserter carrier | (55b) | Second cam |
| (33) | Second position spike inserter carrier | (56) | Cartridge fixator |
| (34) | Biasing means, spring | (56a) | Sterile barrier cartridge fixator |
| (34a) | End of spring, lever arm | (70) | Steering drum |
| (35) | Canula holder | (70a) | First arrester steering drum |
| (35a) | Abutment surface | (70b) | Second arrester steering drum |
| (35b) | Catch for locking mechanism | (70c) | Gearing |
| (36) | Canula, needle | (70d) | Gearing stop |
| (37) | Needle retracted position | (70e) | Rim on steering drum |
| (38) | Needle inserted position | (70f) | End of rim |
| (39) | First guiding means of spike inserter carrier | (70g) | Recess |
| (40) | Transformation means on canula holder | (71) | Biasing means, torsional spring |
| (41) | Needle control element | (71a) | End of spring |
| (41a) | Locking arm | (72) | Stop means |
| (41 b) | End locking arm | (72a) | Counter arrester |
| (41c) | Needle retraction arm | (72b) | Coupling member |
| (41d) | First key needle control element | (72c) | Rotation axis |
| (41e) | Second key needle control element | (73) | Spike carrier |
| (41f) | Guiding contour needle control element | (73a) | Toothing |
| (42) | Arrester | (73b) | Guiding slot |
| (43) | Tubing | (73c) | Stop |
| (44) | Guide slot on housing for spike inserter carrier | (74) | Guiding means steering drum |
| | | (75) | Transformation means, lever arm |
| (45) | Inclination angle first guiding means | (75a) | Guide slot lever arm |
| (46) | Inclination angle second guiding means | (75b) | Steering element |
| (47) | First angle of rotation needle control element | (75c) | Second steering element, second stop |
| | | (80) | Segmented piston rod |
| (48) | Second arrester | (81) | Segment |
| (81a) | Drive segment, first segment | (122) | Crimp with sealing element |
| (81b) | Transfer segment | (122a) | Sealing surface |
| (81c) | Delivery segment | (122b) | Connector |
| (81d) | Last segment | (123) | Counter surface |
| (81e) | Flange, connector to plunger | (124) | Sterile barrier at rim adhesive surface |
| (81f) | Teeth | (125) | Glas barrel |
| (81g) | Normal last segment | (125a) | Neck |
| (81h) | Wing | (125b) | Flange |
| (81 | i) Wing | (126) | Sterile interface |
| (82) | Hinge | (127) | First cavity |
| (82a) | Opposite side hinge | (128) | Second cavity |
| (83) | Motor | (129) | Circumferential rim |
| (84) | Gearing | (130) | Passage |
| (85) | Worm wheel | (131) | Elastomeric component, O-ring. |
| (86) | Encoder | (132) | Tray, tub support |
| (87) | Guiding element, fin | (133) | Liquid solution |
| (87a) | Edge guiding element | (134) | Medicament |
| (88) | Spring type piston rod | (135) | Sealing |
| (89) | Gear wheel | (136) | End wall between first and second cavity |
| (90) | Toothing | (137) | Tub |
| (100) | Optical indicator, LED | (138) | Elastic attachment means, Multi-layered adhesive system, double-sided adhesive tape |
| (101) | Control unit | | |
| (102) | Battery | (139) | Core layer |
| (103) | Capacitive sensor | (140), (140a) | Adhesive layers |
| (104) | Adhesive layer | (141) | Liner sheet |
| (105) | Peel foil adhesive layer | (142) | Passage |
| (106) | Passage | (143) | Inner lateral wall surface |
| (107) | Sticker- connector | (144) | Outer lateral wall surface |
| (108) | Peel foil, sterile barrier | (11,12) | One way coupling, ratchet |
| (120) | Sterile barrier, Sterile enclosure | | |
| (121) | Sealing element | | |

## Claims

1. A method for fixating a cartridge (26) adapted for receiving a medicament (134) to a cartridge holder (4d) of an injection device, comprising the steps of:
a) Providing a cartridge (26) having cylindrical glass barrel (125) and a septum (27) connected to the distal end of the glass barrel (125) using a crimp (27a),
b) Providing a cartridge holder (4d),
c) Inserting the cartridge (26) into the cartridge holder (4d) such that the distal end of the cartridge (26) first enters the cartridge holder (4d) and is received by a second cavity (128) which is part of the cartridge holder (4d),
**Characterized by** that the cartridge (26) is axially and rotationally fixed (4d) after the insertion step c) by at least one elastic attachment means (138) for attaching the cartridge (26) to the cartridge holder (4d).

2. The method for fixating the cartridge to the cartridge holder according to claim 1, whereby the at least one elastic attachment means (138) is an elastic adhesive or at least one multilayered system comprising at least one adhesive surface (140) and at least one elastic core layer (139), preferably being an elastic foam layer.

3. The method for fixating the cartridge to the cartridge holder according to claim 2, whereby the at least one multilayered system is a double sided adhesive tape (138) comprising two opposing adhesive layers (140, 140a) and two opposing non-adhesive lateral wall surfaces (143, 144) preferably arranged perpendicular to the adhesive layers (140, 140a) and having the at least one elastic core layer (139) arranged between the two opposing adhesive layers (140, 140a).

4. The method for fixating the cartridge to the cartridge holder according to claim 3, whereby at least one layer of the double sided adhesive tape (138), preferably the core layer (139) is adapted such that it has sterile barrier properties between the two lateral lateral wall surfaces (143, 144).

5. The method for fixating the cartridge to the cartridge holder according to claim 4, whereby the at least one piece of double-sided adhesive tape (138) is positioned between the crimp (27a) of the cartridge (26) and a wall surface of the second cavity (128).

6. The method for fixating the cartridge to the cartridge holder according to claim 5, whereby the at least one piece of double-sided adhesive tape (138) is ring-shaped and surrounds the lateral wall surface (27e) of the crimp (27a) and/or covers the circular shaped end surface (27c) of the crimp (27a).

7. The method for fixating the cartridge to the cartridge holder according to claim 6, whereby the at least one piece of double-sided adhesive tape (138) is attached to the cartridge (26) prior to step c) or the at least one piece of double sided-adhesive tape (138) is attached to the end-wall (136) of the second cavity (128) prior to step c).

8. The method for fixating the cartridge to the cartridge holder according to claim 7, whereby after insertion step c) the cartridge (26) is fixated to the cartridge holder (4d) and a sterile barrier (120) is established between the second cavity (128) of the cartridge holder (26) and a first cavity (127) of the injection device.

9. The method for fixating the cartridge to the cartridge holder according to claim 8, whereby the first (127) and second (128) cavity are connected to each other by the end-wall (136), the end-wall having a passage (130) between the first and second cavity which is closed or covered after step c) by the distal end of the cartridge (26) and the at least one piece of double-sided adhesive tape (138).

10. The method for fixating the cartridge to the cartridge holder according to claim 9, whereby the first cavity (127) encloses a fluid path unit (25) of the injection device which comprises at least a needle (36) adapted for penetrating the skin of a patient, a spike (31) adapted for penetrating the septum (27) of the cartridge and a tubing (43) providing a fluid connection between the spike (31) and the needle (36) and that the cartridge holder (4d) comprises the second cavity (128) enclosing the cartridge (26).

11. The method for fixating the cartridge to the cartridge holder according to anyone of the previous claims **characterized by** that an empty cartridge is used or a full cartridge containing a liquid medicament and which is closed by a plug (28) is used.

12. A medication delivery device comprising a cartridge holder (4d) and a cartridge (26) whereby the cartridge (26) is fixated to the cartridge holder (4d) according to anyone of the previous claims.
